(19) **Europäisches Patentamt / European Patent Office / Office européen des brevets**

(11) **EP 1 951 198 B1**

(12) **EUROPEAN PATENT SPECIFICATION**

(45) Date of publication and mention
of the grant of the patent:
**16.06.2010 Bulletin 2010/24**

(51) Int Cl.:
***A61K 9/00*** (2006.01)

(21) Application number: **06826368.0**

(22) Date of filing: **20.10.2006**

(86) International application number:
**PCT/US2006/041081**

(87) International publication number:
**WO 2007/047948 (26.04.2007 Gazette 2007/17)**

(54) **INTRANASAL ADMINISTRATION OF RAPID ACTING INSULIN**

INTRANASALE VERABREICHUNG VON SCHNELL WIRKENDEM INSULIN

ADMINISTRATION INTRANASALE D'INSULINE A ACTION RAPIDE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **20.10.2005 US 728877 P**
**03.03.2006 US 778724 P**
**10.07.2006 US 806904 P**
**04.08.2006 US 821525 P**
**15.09.2006 US 825876 P**

(43) Date of publication of application:
**06.08.2008 Bulletin 2008/32**

(73) Proprietor: **MDRNA, Inc.**
**Bothell, WA 98021-7266 (US)**

(72) Inventors:
• **QUAY, Steven, C.**
**Woodinville, WA 98072 (US)**
• **COHEN, Annemarie, Stoudt**
**Kirkland, WA 98034 (US)**
• **COSTANTINO, Henry, R.**
**Woodinville, WA 98072 (US)**

• **QUAY, Shu-Chih Chen**
**Seattle, WA 98112 (US)**
• **SILENO, Anthony, P.**
**Brookhaven Hamlet, NY 11719 (US)**
• **KLEPPE, Mary, S.**
**Oakdale, CT 06370 (US)**

(74) Representative: **MacLean, Martin Robert et al**
**Mathys & Squire LLP**
**120 Holborn**
**London**
**EC1N 2SQ (GB)**

(56) References cited:
**EP-A1- 1 031 347          WO-A-94/22461**
**US-A1- 2003 211 972**

• **ZHANG Y ET AL: "Nasal absorption enhancement
of insulin by sodium deoxycholate in
combination with cyclodextrins" ZHONGUA
YAOLI XUEBAO - ACTA PHARMACOLOGICA
SINICA, SHANGHAI, CN, vol. 22, no. 11, November
2001 (2001-11), pages 1051-1056, XP002982895
ISSN: 0253-9756**

**Description**

BACKGROUND

Insulin is an important glucose-regulating polypeptide hormone. It is a naturally-occurring hormone secreted by the pancreas. Insulin is required by the cells of the body to remove and use glucose from the blood. Glucose allows the cells to produce the energy needed to carry out cellular functions. In addition to being the primary effector in carbohydrate homeostasis, insulin has effects on fat metabolism. It can change the liver's ability to release fat stores. Insulin has various pharmacodynamic effects throughout the body.

Researchers first gave an active extract of the pancreas containing insulin to a young diabetic patient in 1922, and the FDA first approved insulin in 1939. Currently, insulin that is used for treatment is derived from beef and pork pancreas as well as recombinant (human) technology. The first recombinant human insulin was approved by the FDA in 1982.

Insulin is used medically in some forms of diabetes mellitus. Patients with diabetes mellitus have an inability to take up and use glucose from the blood, and, as a result, the glucose level in the blood rises. In type 1 diabetes, the pancreas cannot produce enough insulin. ' Therefore, insulin therapy is needed. In type 2 diabetes, patients produce insulin, but cells throughout the body do not respond normally to the insulin. Nevertheless, insulin may also be used in type 2 diabetes to overcome cellular resistance to insulin. By increasing the uptake of glucose by cells and reducing the concentration of glucose in the blood, insulin prevents or reduces the long-term complications of diabetes, including damage to the blood vessels, eyes, kidneys, and nerves. Insulin is usually administered by injection under the skin (subcutaneously). The subcutaneous tissue of the abdomen is preferred because absorption of the insulin is more consistent from this location than subcutaneous tissues in other locations.

When insulin was first discovered and made available for people with diabetes there was only one kind of short-acting insulin. This required several injections a day. As time went on, new insulins were developed that lasted longer, requiring fewer injections, but requiring strict attention to timing of meals. Now, there are different types of insulin available. This gives more flexibility in the number and timing of administration, making it easier to maintain target blood glucose levels, based on a patient's lifestyle. Insulin is available in various forms, for example, rapid, medium- and long-acting. Insulin is typically delivered by subcutaneous injections. Other options, such as pump delivery, and more recently pulmonary delivery are available.

Several insulin analogs that are prepared with recombinant DNA technology are available for clinical use. Among these agents is insulin aspart (NovoLog™; Novo Nordisk Pharmaceuticals), which is homologous with regular human insulin except for a single substitution of aspartic acid for proline at position B28. This single substitution reduces the molecule's tendency to form hexamers. Therefore, insulin aspart is absorbed more rapidly after subcutaneous injection and has both a faster onset of action and a shorter duration of action than short-acting insulin.

Insulin mixtures are also used, especially for people with type 2 diabetes. Insulin mixtures allow treatment with different types of insulins in one combined administration.

Injectable insulin comes in three different forms-vials, prefilled syringes, and cartridges. The cartridges are used in a pen-like device that simplifies injection. Human recombinant insulin, insulin lispro, insulin aspart, and insulin glargine are the commonly-used insulins. Beef and pork insulin are infrequently used. Regular human insulin (Novolin R, Humulin R) is available in vials, cartridges, and prefilled syringes.

NPH human insulin (Novolin N, Humulin N) is available in vials, cartridges and prefilled syringes. A mixture of 70% NPH human insulin and 30% regular human insulin (Novolin 70/30, Humulin 70/30) is available in vials, cartridges and pre-filled syringes.

A mixture of 50% NPH human insulin and 50% regular human insulin (Humulin 50/50) is available in vials. Lente human insulin (Novolin L, Humulin L) is available in vials. Ultralente human insulin (Humulin U) is available in vials. Insulin lispro (Humalog) is available in vials and cartridges. Insulin aspart (Novolog) is available in vials and cartridges. Insulin glargine (Lantus) is available in vials and cartridges.

Monomeric forms of insulin include insulin homologs and are known to be rapid acting, e.g., insulin glulisine (LysB3, GluB29), HMR-1153 (LysB3, IleB28), HMR-1423 (GlyA21, HisB31, HisB32), insulin aspart (AspB28) or (AspB10), lispro (LysB28, ProB29). In every instance above, the nomenclature of the analogs is based on a description of the amino acid substitution at specific positions on the A or B chain of insulin, numbered from the N-terminus of the chain, in which the remainder of the sequence is that of natural human insulin.

A dry powder formulation of a rapid acting insulin has been described for lung delivery that comprises an insulin having the amino acid sequence of native human insulin (U.S. Patent , No. 6,737,045). There is a need to develop further pharmaceutical formulations comprising rapid acting insulins, i.e., those which are able to provide peak serum levels within 60 minutes and glucose troughs within 90 minutes.

There are several choices available for people who inject insulin. Insulin can be injected manually, or can be infused into the body with the help of a small electronic infusion device called an insulin pump. Syringes are probably the most common and cost-effective choice, and are useful for patients who take two types of insulin mixed together. An alternative to syringes is an insulin pen, which comes prefilled with insulin and may either be disposable or reusable (with disposable insulin cartridges). The device resembles a large pen, with a fine needle under the cap and a plunger at the other end. A dial allows the user to regulate the dose. Insulin pens are also available in the most frequently-prescribed mixtures of insulin types, such as 70/30 (NPH and regular insulin). Some people prefer pens to syringes because they are easy to carry and use.

Another device known as an insulin jet injector works by using a high-pressure blast of air to send a fine spray of insulin through the skin. This may be a good option for those patients that are needle-shy. However, jet injectors require a significant financial investment and aren't always covered by insurance.

An insulin pump may be a more effective way to control type 1 diabetes for some people because it more closely mimics the insulin production of a pancreas. An insulin pump is a compact electronic device with an attached infusion set (or tube) that administers a small, steady flow of insulin to a patient throughout the day, known as a "basal rate." Before eating, a pump user programs the pump to deliver a "bolus" of fast-acting insulin to cover the corresponding rise in blood glucose levels from the meal. Pump flow can also be manually adjusted by a user throughout the day as needed.

Glucose-regulating peptides are a class of peptides that have been shown to have therapeutic potential in the treatment of insulin dependent diabetes mellitus (IDDM), gestational diabetes or non insulin-dependent diabetes mellitus (NIDDM), the treatment of obesity and the treatment of dyslipidemia. See U.S. Patent No. 6,506,724; U.S. Patent Application Publication No. 20030036504A1; European Patent No. EP1083924B1; International Patent Application Publication No. WO 98/30231A1; and International Patent Application No. WO 00/73331A2. In addition to insulin and insulin analogs, glucose-regulating peptides include glucagons-like peptide, GLP, e.g., GLP-1, the exendins, especially exendin-4, also known as exenatide, and amylin peptides and amylin analogs such as pramlintide. To date these peptides have been administered to humans by injection.

ZHANG Y ET AL. ZHONGUA YAOLI XUEBAO - ACTA PHARMACOLOGICA SINICA, SHANGHAI, CN, vol. 22, no. 11, November 2001 (2001-11), pages 1051-1056, EP 1 031 347, US 2003/211972, WO 94/22461 disclose compositions for intranasal administration, which comprise monomeric insulin, a solubilizing agent (beta cyclodextrin, in WO 94/22461 a methylatged p-cyclodextrin) and a surface active agent.

ZHANG Y ET AL. describes the use of sodium deoxycholate as a powerful detergent. In EP1 031 347 didecanoyl phosphatidylcholine is used.

US 2003/211972 refers to bile salts and phospholipids, whilst WO 94/22461 focusses on didecanoyl-L-a-phosphatidylcholine.

Thus, there is a need to develop pharmaceutical formulations for administration of glucose-regulating peptides, especially rapid acting insulins, other than by injection.

DESCRIPTION OF THE DRAWINGS

FIGURE 1: PK Results for Rabbit Study 1 Comparing PDF alone to PDF with Tween Formulations;

FIGURE 2: PK Results for Rabbit Study 2 Comparing IN Administration of PDF with Tween Formulations to SQ Administration of NovoLog;

FIGURE 3: PD Results for Rabbit Study 1, % Glucose (Log Linear Scale) Comparing IN Control, IN PDF, IN PDF with Tween, IV and SC Formulations;

FIGURE 4: PD Results Comparing Study 2 and Study 1 Data, % Glucose from Initial (Linear Graph);

FIGURE 5: PD data for groups dosed in Preclinical Study 3, % Glucose from Initial (Linear Graph);

FIGURE 6: PK data for groups dosed in Preclinical Study 3 Comparing IN PDF with Tween (with and without DDPC), SC PDF, and SC Control Formulations;

FIGURE 7: PD data for groups dosed in Preclinical Study 4 Comparing IN PDF with Tween Containing 0.2% Gelatin or PG (with and without DDPC);

FIGURE 8: PK data for groups dosed in Preclinical Study 4 Comparing IN PDF with Tween Containing 0.2% Gelatin or PG (with and without DDPC), TDMhypotonic, TDMIsotonic, Oral PDF (with and without PG and/or DDPC), and SC Control;

FIGURE 9: % Glucose from Initial PD data for all groups dosed with viscosity enhancer formulations (Gelatin, HPMC, MC, Carbomer, and CMC); and

FIGURE 10: PK data for all groups dosed with viscosity enhancer formulations (Gelatin, HPMC, MC, Carbomer, and CMC).

DESCRIPTION OF THE INVENTION

In order to provide better understanding of the present invention, the following definitions are provided:

Insulin and Insulin Analogs

The current invention focuses primarily on intranasal administration of rapid acting insulins which are able to provide peak serum levels within 60 minutes and glucose troughs within 90 minutes. According to the present invention, glucose-regulating peptides also include the free bases, acid addition salts or metal salts, such as potassium or sodium salts of the peptides, and peptides that have been modified by such processes as amidation, glycosylation, acylation, sulfation, phosphorylation, acetylation, cyclization and other well known covalent modification methods.

As used herein, the term "human insulin" includes recombinant human insulin.

Pharmaceutically-acceptable salts include inorganic acid salts, organic amine salts, organic acid salts, alkaline earth metal salts and mixtures thereof. Suitable examples of pharmaceutically-acceptable salts include, but are not limited to, halide, glucosamine, alkyl glucosamine, sulfate, hydrochloride, carbonate, hydrobromide, N, N'-dibenzylethylene-diamine, triethanolamine, diethanolamine, trimethylamine, triethylamine, pyridine, picoline, dicyclohexylamine, phosphate, sulfate, sulfonate, benzoate, acetate, salicylate, lactate, tartate, citrate, mesylate, gluconate, tosylate, maleate, fumarate, stearate and mixtures thereof.

Thus, according to the present invention, the above-described peptides, and mixtures thereof, are incorporated into pharmaceutical formulations suitable for transmucosal delivery, especially intranasal delivery.

Peptide Analogs and Mimetics

Included within the definition of biologically active peptides for use within the invention are natural or synthetic, therapeutically or prophylactically active, peptides (comprised of two or more covalently linked amino acids), proteins, peptide or protein fragments, peptide or protein analogs, and chemically modified derivatives or salts of active peptides or proteins. A wide variety of useful analogs and mimetics of glucose-regulating peptides are contemplated for use within the invention and can be produced and tested for biological activity according to known methods. Often, the peptides or proteins of glucose-regulating peptide or other biologically active peptides or proteins for use within the invention are muteins that are readily obtainable by partial substitution, addition, or deletion of amino acids within a naturally occurring or native (e.g., wild-type, naturally occurring mutant, or allelic variant) peptide or protein sequence. Additionally, biologically active fragments of native peptides or proteins are included. Such mutant derivatives and fragments substantially retain the desired biological activity of the native peptide or proteins. In the case of peptides or proteins having carbohydrate chains, biologically active variants marked by alterations in these carbohydrate species are also included within the invention.

As used herein, the term "conservative amino acid substitution" refers to the general interchangeability of amino acid residues having similar side chains. For example, a commonly interchangeable group of amino acids having aliphatic side chains is alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Examples of conservative substitutions include the substitution of a non-polar (hydrophobic) residue such as isoleucine, valine, leucine or methionine for another. Likewise, the present invention contemplates the substitution of a polar (hydrophilic) residue such as between arginine and lysine, between glutamine and asparagine, and between threonine and serine. Additionally, the substitution of a basic residue such as lysine, arginine or histidine for another or the substitution of an acidic residue such as aspartic acid or glutamic acid for another is also contemplated. Exemplary conservative amino acids substitution groups are: valine-leucine-isoleucine, phenylalanine-tyrosine, lysine-arginine, alanine-vaime, and asparagine-glutalnine. By aligning a peptide or protein analog optimally with a corresponding native peptide or protein, and by using appropriate assays, e.g., adhesion protein or receptor binding assays, to determine a selected biological activity, one can readily identify operable peptide and protein analogs for use within the methods and compositions of the invention. Operable peptide and protein analogs are typically specifically immunoreactive with antibodies raised to the corresponding native peptide or protein.

Mucosal Delivery Enhancing Agents

"Mucosal delivery enhancing agents" are defined as chemicals and other excipients that, when added to a formulation comprising water, salts and/or common buffers, and glucose-regulating peptide (the control formulation) produce a formulation that produces an effective increase in transport of glucose-regulating peptide across a mucosa as measured by the maximum blood, serum, or cerebral spinal fluid concentration ($C_{max}$) or by the area under the curve, AUC, in a plot of concentration versus time. A mucosa includes the nasal, oral, intestional, buccal, bronchopulmonary, vaginal, and rectal mucosal surfaces and in fact includes all mucus-secreting membranes lining all body cavities or passages that communicate with the exterior. Mucosal delivery enhancing agents are sometimes called "carriers."

"Endotoxin-free formulation" means a formulation which contains a glucose-regulating peptide and one or more mucosal delivery enhancing agents that is substantially free of endotoxins and/or related pyrogenic substances. Endotoxins include toxins that are confined inside a microorganism and are released only when the microorganisms are broken down or die. Pyrogenic substances include fever-inducing, thermostable substances (glycoproteins) from the outer membrane of bacteria and other microorganisms. Both of these substances can cause fever, hypotension and shock if administered to humans. Producing formulations that are endotoxin-free can require special equipment, expert artisians, and can be significantly more expensive than making formulations that are not endotoxin-free. Because intravenous administration of GLP or amylin simultaneously with infusion of endotoxin in rodents has been shown to prevent the hypotension and even death associated with the administration of endotoxin alone (U.S. Patent No. 4,839,343), producing endotoxin-free formulations of these and other glucose-regulating peptide therapeutic agents would not be expected to be necessary for non-parental (non-injected) administration.

Non-Infused Administration

"Non-infused administration" means any method of delivery that does not involve an injection directly into an artery or vein, a method which forces or drives (typically a fluid) into something and especially to introduce into a body part by means of a needle, syringe or other invasive method. Non-infused administration includes subcutaneous injection, intramuscular injection, intraparitoneal injection and the non-injection methods of delivery to a mucosa.

Methods and Compositions of Delivery

Improved methods and compositions for mucosal administration of glucose-regulating peptide to mammalian subjects optimize glucose-regulating peptide dosing schedules. The present invention provides mucosal delivery of glucose-regulating peptide formulated with one or more mucosal delivery-enhancing agents wherein glucose-regulating peptide dosage release is substantially normalized and/or sustained for an effective delivery period of glucose-regulating peptide release ranges from approximately 0.1 to 2.0 hours; 0.4 to 1.5 hours; 0.7 to 1.5 hours; or 0.8 to 1.0 hours; following mucosal administration. The sustained release of glucose-regulating peptide achieved may be facilitated by repeated administration of exogenous glucose-regulating peptide utilizing methods and compositions of the present invention. Improved compositions and methods for mucosal administration of glucose-regulating peptide to mammalian subjects optimize glucose-regulating peptide dosing schedules. The present invention provides improved mucosal (e.g., nasal) delivery of a formulation comprising glucose-regulating peptide in combination with one or more mucosal delivery-enhancing agents and an optional sustained release-enhancing agent or agents. Mucosal delivery-enhancing agents of the present invention yield an effective increase in delivery, e.g., an increase in the maximal plasma concentration ($C_{max}$) to enhance the therapeutic activity of mucosally-administered glucose-regulating peptide. A second factor affecting therapeutic activity of glucose-regulating peptide in the blood plasma and CNS is residence time (RT). Sustained release-enhancing agents, in combination with intranasal delivery-enhancing agents, increase $C_{max}$ and increase residence time (RT) of glucose-regulating peptide. Polymeric delivery vehicles and other agents and methods of the present invention that yield sustained release-enhancing formulations, for example, polyethylene glycol (PEG), are disclosed herein.

The present invention provides an improved glucose-regulating peptide delivery method and dosage form for treatment of symptoms related to diseases and conditions including diabetes, hyperglycemia, dyslipidemia, inducing satiety in an individual, promoting weight loss in an individual, obesity, colon cancer, prostate cancer, or other cancer in a mammalian subject.

Within the mucosal delivery formulations and methods of this invention, the glucose-regulating peptide is frequently combined or coordinately administered with a suitable carrier or vehicle for mucosal delivery. As used herein, the term "carrier" means pharmaceutically acceptable solid or liquid filler, diluent or encapsulating material. A water-containing liquid carrier can contain pharmaceutically acceptable additives such as acidifying agents, alkalizing agents, antimicrobial preservatives, antioxidants, buffering agents, chelating agents, complexing agents, solubilizing agents, humectants, solvents, suspending and/or viscosity-increasing agents, tonicity agents, wetting agents or other biocompatible materials. A tabulation of ingredients listed by the above categories, can be found in the US. Pharmacopeia National Formulary, 1857-1859, 1990. Some examples of the materials which can serve as pharmaceutically acceptable carriers are sugars, such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols, such as propylene glycol; polyols such as glycerin, sorbitol, mannitol and polyethylene glycol; esters such as ethyl oleate and ethyl laurate; agar; buffering agents such as magnesium hydroxide and aluminum hydroxide; alginic acid; pyrogen free water; isotonic saline; Ringer's solution, ethyl alcohol and phosphate buffer solutions, as well as other non toxic compatible substances used in pharmaceutical formulations, and mixtures thereof.

Thus, some examples of humectants include propylene glycol, glycerine, glyceryl triacetate, a polyol, a polymeric polyol, lactic acid, urea, and mixtures thereof.

Some examples of buffer and buffer salt are based on glutamate, acetate, glycine, histidine, arginine, lysine, methionine, lactate, formate, glycolate, and mixtures thereof.

Wetting agents, emulsifiers and lubricants such as sodium lauryl sulfate and magnesium stearate, as well as coloring agents, release agents, coating agents, sweetening, flavoring and perfuming agents, preservatives and antioxidants can also be present in the compositions, according to the desires of the formulator. Examples of pharmaceutically acceptable antioxidants include water soluble antioxidants such as ascorbic acid, cysteine hydrochloride, sodium bisulfite, sodium-metabisulfite, sodium sulfite and the like; oil-soluble antioxidants such as ascorbyl palmitate, butylated hydroxyanisole (BHA), butylated hydroxytoluene (BHT), lecithin, propyl gallate, alpha-tocopherol and the like; and metal-chelating agents such as citric acid, ethylenediamine tetraacetic acid (EDTA), sorbitol, tartaric acid, phosphoric acid, and mixtures thereof. The amount of active ingredient that can be combined with the carrier materials to produce a single dosage form will vary depending upon the particular mode of administration.

Within the mucosal delivery compositions and methods of the invention, various delivery-enhancing agents are employed which enhance delivery of glucose-regulating peptide into or across a mucosal surface. In this regard, delivery of glucose-regulating peptide across the mucosal epithelium can occur "transcellularly" or "paracellularly." The extent to which these pathways contribute to the overall flux and bioavailability of the glucose-regulating peptide, depends upon the environment of the mucosa, the physico-chemical properties the active agent, and on the properties of the mucosal epithelium. Paracellular transport involves only passive diffusion, whereas transcellular transport can occur by passive, facilitated or active processes. Generally, hydrophilic, passively transported, polar solutes diffuse through the paracellular route, while more lipophilic solutes use the transcellular route. Absorption and bioavailability (e.g., as reflected by a permeability coefficient or physiological assay), for diverse, passively and actively absorbed solutes, can be readily evaluated, in terms of both paracellular and transcellular delivery components, for any selected glucose-regulating peptide within the invention. For passively absorbed drugs, the relative contribution of paracellular and transcellular pathways to drug transport depends upon the pKa, partition coefficient, molecular radius and charge of the drug, the pH of the luminal environment in which the drug is delivered, and the area of the absorbing surface. The paracellular route represents a relatively small fraction of accessible surface area of the nasal mucosal epithelium. In general terms, it has been reported that cell membranes occupy a mucosal surface area that is a thousand times greater than the area occupied by the paracellular spaces. Thus, the smaller accessible area, and the size- and charge-based discrimination against macromolecular permeation would suggest that the paracellular route would be a generally less favorable route than transcellular delivery for drug transport. Surprisingly, the methods and compositions of the invention provide for significantly enhanced transport of biotherapeutics into and across mucosal epithelia via the paracellular route. Therefore, the methods and compositions of the invention successfully target both paracellular and transcellular routes, alternatively or within a single method or composition.

As used herein, "mucosal delivery-enhancing agents" include agents which enhance the release or solubility (e.g., from a formulation delivery vehicle), diffusion rate, penetration capacity and timing, uptake, residence time, stability, effective half-life, peak or sustained concentration levels, clearance and other desired mucosal delivery characteristics (e.g., as measured at the site of delivery, or at a selected target site of activity such as the bloodstream or central nervous system) of glucose-regulating peptide or other biologically active compound(s). Enhancement of mucosal delivery can thus occur by any of a variety of mechanisms, for example by increasing the diffusion, transport, persistence or stability of glucose-regulating peptide, increasing membrane fluidity, modulating the availability or action of calcium and other ions that regulate intracellular or paracellular permeation, solubilizing mucosal membrane components (e.g., lipids), changing non-protein and protein sulfhydryl levels in mucosal tissues, increasing water flux across the mucosal surface, modulating epithelial junctional physiology, reducing the viscosity of mucus overlying the mucosal epithelium, reducing mucociliary clearance rates, and other mechanisms.

As used herein, a "mucosally effective amount of glucose-regulating peptide" contemplates effective mucosal delivery of glucose-regulating peptide to a target site for drug activity in the subject that may involve a variety of delivery or transfer routes. For example, a given active agent may find its way through clearances between cells of the mucosa and reach an adjacent vascular wall, while by another route the agent may, either passively or actively, be taken up into mucosal cells to act within the cells or be discharged or transported out of the cells to reach a secondary target site, such as the systemic circulation. The methods and compositions of the invention may promote the translocation of active agents along one or more such alternate routes, or may act directly on the mucosal tissue or proximal vascular tissue to promote absorption or penetration of the active agent(s). The promotion of absorption or penetration in this context is not limited to these mechanism.

As used herein "peak concentration ($C_{max}$) of glucose-regulating peptide in a blood plasma", "area under concentration vs. time curve (AUC) of glucose-regulating peptide in a blood plasma", "time to maximal plasma concentration ($t_{max}$) of glucose-regulating peptide in a blood plasma" are pharmacokinetic parameters known to one skilled in the art. Laursen et al., Eur. J Endocrinology 135:309-315, 1996. The "concentrations vs. time curve" measures the concentration of glucose-regulating peptide in a blood serum of a subject vs. time after administration of a dosage of glucose-regulating peptide to the subject either by intranasal, intramuscular, subcutaneous, or other parenteral route of administration. "$C_{max}$" is the maximum concentration of glucose-regulating peptide in the blood serum of a subject following a single dosage of glucose-regulating peptide to the subject. "$t_{max}$" is the time to reach maximum concentration of glucose-regulating peptide in a blood serum of a subject following administration of a single dosage of glucose-regulating peptide to the subject.

As used herein, "area under concentration vs. time curve (AUC) of glucose-regulating peptide in a blood plasma" is calculated according to the linear trapezoidal rule and with addition of the residual areas. A decrease of 23% or an increase of 30% between two dosages would be detected with a probability of 90% (type II error $\beta$ = 10%). The "delivery rate" or "rate of absorption" is estimated by comparison of the time ($t_{max}$) to reach the maximum concentration ($C_{max}$). Both $C_{max}$ and $t_{max}$ are analyzed using non-parametric methods. Comparisons of the pharmacokinetics of intramuscular, subcutaneous, intravenous and intranasal glucose-regulating peptide administrations were performed by analysis of variance (ANOVA). For pair wise comparisons a Bonferroni-Holmes sequential procedure is used to evaluate significance. The dose-response relationship between the three nasal doses is estimated by regression analysis. P <0.05 is considered significant. Results are given as mean values +/- SEM.

While the mechanism of absorption promotion may vary with different mucosal delivery-enhancing agents of the invention, useful reagents in this context will not substantially adversely affect the mucosal tissue and will be selected according to the physicochemical characteristics of the particular glucose-regulating peptide or other active or delivery-enhancing agent. In this context, delivery-enhancing agents that increase penetration or permeability of mucosal tissues will often result in some alteration of the protective permeability barrier of the mucosa. For such delivery-enhancing agents to be of value within the invention, it is generally desired that any significant changes in permeability of the mucosa be reversible within a time frame appropriate to the desired duration of drug delivery. Furthermore, there should be no substantial, cumulative toxicity, nor any permanent deleterious changes induced in the barrier properties of the mucosa with long-term use.

Within certain aspects of the invention, absorption-promoting agents for coordinate administration or combinatorial formulation with glucose-regulating peptide of the invention are selected from small hydrophilic molecules, including but not limited to, dimethyl sulfoxide (DMSO), dimethylformamide, ethanol, propylene glycol, and the 2-pyrrolidones. Alternatively, long-chain amphipathic molecules, for example, deacylmethyl sulfoxide, azone, sodium laurylsulfate, oleic acid; and the bile salts, may be employed to enhance mucosal penetration of the glucose-regulating peptide. In additional aspects, surfactants (e.g., polysorbates) are employed as adjunct compounds, processing agents, or formulation additives to enhance intranasal delivery of the glucose-regulating peptide. Agents such as DMSO, polyethylene glycol, and ethanol can, if present in sufficiently high concentrations in delivery environment (e.g., by pre-administration or incorporation in a therapeutic formulation), enter the aqueous phase of the mucosa and alter its solubilizing properties, thereby enhancing the partitioning of the glucose-regulating peptide from the vehicle into the mucosa.

Thus, some examples of solubilizing agents include cyclodextrins, hydroxypropyl-$\beta$-cyclodextrin, sulfobutylether-$\beta$-cyclodextrin, methyl-$\beta$-cyclodextrin, and mixtures thereof.

Additional mucosal delivery-enhancing agents that are useful within the coordinate administration and processing methods and combinatorial formulations of the invention include, but are not limited to, mixed micelles; enamines; nitric oxide donors (e.g., S-nitroso-N-acetyl-DL-penicillamine, NOR1, NOR4--which are preferably co-administered with an NO scavenger such as carboxy-PITO or doclofenac sodium); sodium salicylate; glycerol esters of acetoacetic acid (e.g., glyceryl-1,3-diacetoacetate or 1,2-isopropylideneglycerine-3-acetoacetate); and other release-diffusion or intra- or trans-epithelial penetration-promoting agents that are physiologically compatible for mucosal delivery. Other absorption-promoting agents are selected from a variety of carriers, bases and excipients that enhance mucosal delivery, stability, activity or trans-epithelial penetration of the glucose-regulating peptide. These include, *inter alia,* cyclodextrins and $\beta$-cyclodextrin derivatives (e.g., 2-hydroxypropyl-$\beta$-cyclodextrin and heptakis(2,6-di-O-methyl-$\beta$-cyclodextrin). These compounds, optionally conjugated with one or more of the active ingredients and further optionally formulated in an oleaginous base, enhance bioavailability in the mucosal formulations of the invention. Yet additional absorption-enhancing agents adapted for mucosal delivery include medium-chain fatty acids, including mono- and diglycerides (e.g., sodium caprate-extracts of coconut oil, Capmul), and triglycerides (e.g., amylodextrin, Estaram 299, Miglyol 810).

The mucosal therapeutic and prophylactic compositions of the present invention may be supplemented with any suitable penetration-promoting agent that facilitates absorption, diffusion, or penetration of glucose-regulating peptide across mucosal barriers. The penetration promoter may be any promoter that is pharmaceutically acceptable. Thus, in more detailed aspects of the invention compositions are provided that incorporate one or more penetration-promoting agents selected from sodium salicylate and salicylic acid derivatives (acetyl salicylate, choline salicylate, salicylanide, etc.); amino acids and salts thereof (e.g., monoaminocarboxlic acids such as glycine, alanine, phenylalanine, proline, hydroxyproline, etc.; hydroxyamino acids such as serine; acidic amino acids such as aspartic acid, glutamic acid, etc.; and basic amino acids such as lysine etc.—inclusive of their alkali metal or alkaline earth metal salts); and N-acetylamino acids (N-acetylalanine, N-acetylphenylalanine, N-acetylserine, N-acetylglycine, N-acetyllysine, N-acetylglutamic acid, N-acetylproline, N-acetylhydroxyproline, etc.) and their salts (alkali metal salts and alkaline earth metal salts). Also provided as penetration-promoting agents within the methods and compositions of the invention are substances which are generally used as emulsifiers (e.g., sodium oleyl phosphate, sodium lauryl phosphate, sodium lauryl sulfate, sodium myristyl sulfate, polyoxyethylene alkyl ethers, polyoxyethylene alkyl esters, etc.), caproic acid, lactic acid, malic acid and citric acid and alkali metal salts thereof, pyrrolidonecarboxylic acids, alkylpyrrolidonecarboxylic acid esters, N-alkylpyrrolidones, proline acyl esters, and the like.

Within various aspects of the invention, improved nasal mucosal delivery formulations and methods are provided that allow delivery of glucose-regulating peptide and other therapeutic agents within the invention across mucosal barriers between administration and selected target sites. Certain formulations are specifically adapted for a selected target cell, tissue or organ, or even a particular disease state. In other aspects, formulations and methods provide for efficient, selective endo- or transcytosis of glucose-regulating peptide specifically routed along a defined intracellular or intercellular pathway. Typically, the glucose-regulating peptide is efficiently loaded at effective concentration levels in a carrier or other delivery, vehicle, and is delivered and maintained in a stabilized form, e.g., at the nasal mucosa and/or during passage through intracellular compartments and membranes to a remote target site for drug action (e.g., the blood stream or a defined tissue, organ, or extracellular compartment). The glucose-regulating peptide may be provided in a delivery vehicle or otherwise modified (e.g., in the form of a prodrug), wherein release or activation of the glucose-regulating peptide is triggered by a physiological stimulus (e.g., pH change, lysosomal enzymes, etc.). Often, the glucose-regulating peptide is pharmacologically inactive until it reaches its target site for activity. In most cases, the glucose-regulating peptide and other formulation components are non-toxic and non-immunogenic. In this context, carriers and other formulation components are generally selected for their ability to be rapidly degraded and excreted under physiological conditions. At the same time, formulations are chemically and physically stable in dosage form for effective storage. A variety of additives, diluents, bases and delivery vehicles are provided within the invention which effectively control water content to enhance protein stability. These reagents and carrier materials effective as anti-aggregation agents in this sense include, for example, polymers of various functionalities, such as polyethylene glycol, dextran, diethylaminoethyl dextran, and carboxymethyl cellulose, which significantly increase the stability and reduce the solid-phase aggregation of peptides and proteins admixed therewith or linked thereto. In some instances, the activity or physical stability of proteins can also be enhanced by various additives to aqueous solutions of the peptide or protein drugs. For example, additives, such as polyols (including sugars), amino acids, proteins such as collagen and gelatin, and various salts may be used.

Certain additives, in particular sugars and other polyols, also impart significant physical stability to dry, e.g., lyoplilized proteins. These additives can also be used within the invention - to protect the proteins against aggregation not only during lyophilization but also during storage in the dry state. For example sucrose and Ficoll 70 (a polymer with sucrose units) exhibit significant protection against peptide or protein aggregation during solid-phase incubation under various conditions. These additives may also enhance the stability of solid proteins embedded within polymer matrices.

Yet additional additives, for example sucrose, stabilize proteins against solid-state aggregation in humid atmospheres at elevated temperatures, as may occur in certain sustained-release formulations of the invention. Proteins such as gelatin and collagen also serve as stabilizing or bulking agents to reduce denaturation and aggregation of unstable proteins in this context. These additives can be incorporated into polymeric melt processes and compositions within the invention. For example, polypeptide microparticles can be prepared by simply lyophilizing or spray drying a solution containing various stabilizing additives described above. Sustained release of unaggregated peptides and proteins can thereby be obtained over an extended period of time.

Various additional preparative components and methods, as well as specific formulation additives, are provided herein which yield formulations for mucosal delivery of aggregation-prone peptides and proteins, wherein the peptide or protein is stabilized in a substantially pure, unaggregated form using a solubilization agent. A range of components and additives are contemplated for use within these methods and formulations. Exemplary of these solubilization agents are cyclodextrins (CDs), which selectively bind hydrophobic side chains of polypeptides. These CDs have been found to bind to hydrophobic patches of proteins in a manner that significantly inhibits aggregation. This inhibition is selective with respect to both the CD and the protein involved. Such selective inhibition of protein aggregation provides additional advantages within the intranasal delivery methods and compositions of the invention. Additional agents for use in this context include CD dimers, trimers and tetramers with varying geometries controlled by the linkers that specifically block aggregation of peptides and protein. Yet solubilization agents and methods for incorporation within the invention involve the use of peptides and peptide mimetics to selectively block protein-protein interactions. In one aspect, the specific binding of hydrophobic side chains reported for CD multimers is extended to proteins via the use of peptides and peptide mimetics that similarly block protein aggregation. A wide range of suitable methods and anti-aggregation agents are available for incorporation within the compositions and procedures of the invention.

Charge Modifying and pH Control Agents and Methods

To improve the transport characteristics of biologically active agents (including glucose-regulating peptide, other active peptides and proteins, and macromolecular and small molecule drugs) for enhanced delivery across hydrophobic mucosal membrane barriers, the invention also provides techniques and reagents for charge modification of selected biologically active agents or delivery-enhancing agents described herein. In this regard, the relative permeabilities of macromolecules is generally be related to their partition coefficients. The degree of ionization of molecules, which is dependent on the $pK_a$ of the molecule and the pH at the mucosal membrane surface, also affects permeability of the molecules. Permeation and partitioning of biologically active agents, including glucose-regulating peptide and analogs of the invention, for mucosal delivery may be facilitated by charge alteration or charge spreading of the active agent or permeabilizing agent, which is achieved, for example, by alteration of charged functional groups, by modifying the pH of the delivery vehicle or solution in which the active agent is delivered, or by coordinate administration of a charge- or pH-altering reagent with the active agent.

Consistent with these general teachings, mucosal delivery of charged macromolecular species, including glucose-regulating peptide and other biologically active peptides and proteins, within the methods and compositions of the invention is substantially improved when the active agent is delivered to the mucosal surface in a substantially un-ionized, or neutral, electrical charge state.

Certain glucose-regulating peptide and other biologically active peptide and protein components of mucosal formulations for use within the invention will be charge modified to yield an increase in the positive charge density of the peptide or protein. These modifications extend also to cationization of peptide and protein conjugates, carriers and other delivery forms disclosed herein. Cationization offers a convenient means of altering the biodistribution and transport properties of proteins and macromolecules within the invention. Cationization is undertaken in a manner that substantially preserves the biological activity of the active agent and limits potentially adverse side effects, including tissue damage and toxicity.

A "buffer" is generally used to maintain the pH of a solution at a nearly constant value. A buffer maintains the pH of a solution, even when small amounts of strong acid or strong base are added to the solution, by preventing or neutralizing large changes in concentrations of hydrogen and hydroxide ions. A buffer generally consists of a weak acid and its appropriate salt (or a weak base and its appropriate salt). The appropriate salt for a weak acid contains the same negative ion as present in the weak acid (see Lagowski, Macmillan Encyclopedia of Chemistry, Vol. 1, Simon & Schuster, New York, 1997 at p. 273-4). The Henderson-Hasselbach Equation, $pH = pKa + log_{10} [A^-]/[HA]$, is used to describe a buffer, and is based on the standard equation for weak acid dissociation, $HA = H^+ + A^-$. Examples of commonly used buffer sources include the following: acetate, tartrate, citrate, phosphate, lactate, glycine, lysine, arginine, histidine, glutamate, methionine, formate, and glycolate.

The "buffer capacity" means the amount of acid or base that can be added to a buffer solution before a significant pH change will occur. If the pH lies within the range of pK-1 and pK+1 of the weak acid the buffer capacity is appreciable, but outside this range it falls off to such an extent as to be of little value. Therefore, a given system only has a useful buffer action in a range of one pH unit on either side of the pK of the weak acid (or weak base) (see Dawson, Data for Biochemical Research, Third Edition, Oxford Science Publications, 1986, p. 419). Generally, suitable concentrations are chosen so that the pH of the solution is close to the pKa of the weak acid (or weak base) (see Lide, CRC Handbook of Chemistry and Physics, 86th Edition, Taylor & Francis Group, 2005-2006, p. 2-41). Further, solutions of strong acids and bases are not normally classified as buffer solutions, and they do not display buffer capacity between pH values 2.4 to 11.6.

Degradative Enzyme Inhibitory Agents and Methods

Another excipient that may be included in a trans-mucosal preparation is a degradative enzyme inhibitor. Exemplary mucoadhesive polymer-enzyme inhibitor complexes that are useful within the mucosal delivery formulations and methods of the invention include, but are not limited to: Carboxymethylcellulose-pepstatin (with anti-pepsin activity); Poly(acrylic acid)-Bowman-Birk inhibitor (anti-chymotrypsin); Poly(acrylic acid)-chymostatin (anti-chymotrypsin); Poly(acrylic acid)-elastatinal (anti-elastase); Carboxymethylcellulose-elastatinal (anti-elastase); Polycarbophil—elastatinal (anti-elastase); Chitosan-antipain (anti-trypsin); Poly(acrylic acid)—bacitracin (anti-aminopeptidase N); Chitosan—EDTA (anti-aminopeptidase N, anti-carboxypeptidase A); Chitosan—EDTA—antipain (anti-trypsin, anti-chymotrypsin, anti-elastase). As described in further detail below, certain embodiments of the invention will optionally incorporate a novel chitosan derivative or chemically modified form of chitosan. One such novel derivative for use within the invention is denoted as a $\beta$-[1$\rightarrow$4]-2-guanidino-2-deoxy-D-glucose polymer (poly-GuD).

Any inhibitor that inhibits the activity of an enzyme to protect the biologically active agent(s) may be usefully employed in the compositions and methods of the invention. Useful enzyme inhibitors for the protection of biologically active proteins and peptides include, for example, soybean trypsin inhibitor, exendin trypsin inhibitor, chymotrypsin inhibitor and trypsin and chrymotrypsin inhibitor isolated from potato (solanum tuberosum L.) tubers. A combination or mixtures of inhibitors may be employed. Additional inhibitors of proteolytic enzymes for use within the invention include ovomucoid-enzyme, gabaxate mesylate, alphal-antitrypsin, aprotinin, amastatin, bestatin, puromycin, bacitracin, leupepsin, alpha2-macroglobulin, pepstatin and egg white or soybean trypsin inhibitor. These and other inhibitors can be used alone or in combination. The inhibitor(s) may be incorporated in or bound to carrier, e.g., a hydrophilic polymer, coated on the surface of the dosage form which is to contact the nasal mucosa, or incorporated in the superficial phase of the surface, in combination with the biologically active agent or in a separately administered (e.g., pre-administered) formulation.

The amount of the inhibitor, e.g., of a proteolytic enzyme inhibitor that is optionally incorporated in the compositions of the invention will vary depending on (a) the properties of the specific inhibitor, (b) the number of functional groups present in the molecule (which may be reacted to introduce ethylenic unsaturation necessary for copolymerization with hydrogel forming monomers), and (c) the number of lectin groups, such as glycosides, which are present in the inhibitor molecule. It may also depend on the specific therapeutic agent that is intended to be administered. Generally speaking, a useful amount of an enzyme inhibitor is from about 0.1 mg/ml to about 50 mg/ml, often from about 0.2 mg/ml to about 25 mg/ml, and more commonly from about 0.5 mg/ml to 5 mg/ml of the of the formulation (i.e., a separate protease inhibitor formulation or combined formulation with the inhibitor and biologically active agent).

In the case of trypsin inhibition, suitable inhibitors may be selected from, e.g., aprotinin, BBI, soybean trypsin inhibitor, chicken ovomucoid, chicken ovoinhibitor, human exendin trypsin inhibitor, camostat mesilate, flavonoid inhibitors, antipain, leupeptin, p-aminobenzaznidine, AEBSF, TLCK (tosyllysine chloromethylketone), APMSF, DFP, PMSP, and poly(acrylate) derivatives. In the case of chymotrypsin inhibition, suitable inhibitors may be selected from, e.g., aprotinin, BBI, soybean trypsin inhibitor, chymostatin, benzyloxycarbonyl-Pro-Phe-CHO, FK-448, chicken ovoinhibitor, sugar biphenylboronic acids complexes, DFP, PMSF, $\beta$-phenylpropionate, and poly(acrylate) derivatives. In the case of elastase inhibition, suitable inhibitors may be selected from, e.g., elastatinal, methoxysuccinyl-Ala-Ala-Pro-Val-chloromethylketone (MPCMK), BBI, soybean trypsin inhibitor, chicken ovoinhibitor, DFP, and PMSF.

Additional enzyme inhibitors for use within the invention are selected from a wide range of non-protein inhibitors that vary in their degree of potency and toxicity. As described in further detail below, immobilization of these adjunct agents to matrices or other delivery vehicles, or development of chemically modified analogues, may be readily implemented to reduce or even eliminate toxic effects, when they are encountered. Among this broad group of candidate enzyme inhibitors for use within the invention are organophosphorous inhibitors, such as diisopropylfluorophosphate (DFP) and phenylmethylsulfonyl fluoride (PMSF), which are potent, irreversible inhibitors of serine proteases (e.g., trypsin and chymotrypsin). The additional inhibition of acetylcholinesterase by these compounds makes them highly toxic in uncontrolled delivery settings. Another candidate inhibitor, 4-(2-Aminoethyl)-benzenesulfonyl fluoride (AEBSF), has an inhibitory activity comparable to DFP and PMSF, but it is markedly less toxic. (4-Aminophenyl)-methanesulfonyl fluoride hydrochloride (APMSF) is another potent inhibitor of trypsin, but is toxic in uncontrolled settings. In contrast to these inhibitors, 4-(4-isopropylpiperadinocarbonyl)phenyl 1, 2,3,4,-tetrahydro-1-naphthoate methanesulphonate (FK-448) is a low toxic substance, representing a potent and specific inhibitor of chymotrypsin. Further representatives of this non-protein group of inhibitor candidates, and also exhibiting low toxic risk, are camostat mesilate (N,N'-dimethyl carbamoylmethyl-p-(p'-guanidino-benzoyloxy)phenylacetate methane-sulphonate).

Yet another type of enzyme inhibitory agent for use within the methods and compositions of the invention are amino acids and modified amino acids that interfere with enzymatic degradation of specific therapeutic compounds. For use in this context, amino acids and modified amino acids are substantially non-toxic and can be produced at a low cost. However, due to their low molecular size and good solubility, they are readily diluted and absorbed in mucosal environments. Nevertheless, under proper conditions, amino acids can act as reversible, competitive inhibitors of protease enzymes. Certain modified amino acids can display a much stronger inhibitory activity. A desired modified amino acid in this context is known as a 'transition-state' inhibitor. The strong inhibitory activity of these compounds is based on their structural similarity to a substrate in its transition-state geometry, while they are generally selected to have a much higher affinity for the active site of an enzyme than the substrate itself. Transition-state inhibitors are reversible, competitive inhibitors. Examples of this type of inhibitor are $\alpha$-aminoboronic acid derivatives, such as boro-leucine, boro-valine and boro-alanine. The boron atom in these derivatives can form a tetrahedral boronate ion that is believed to resemble the transition state of peptides during their hydrolysis by aminopeptidases. These amino acid derivatives are potent and reversible inhibitors of aminopeptidases and it is reported that boro-leucine is more than 100-times more effective in enzyme inhibition than bestatin and more than 1000-times more effective than puromycin. Another modified amino acid for which a strong protease inhibitory activity has been reported is N-acetylcysteine, which inhibits enzymatic activity of aminopeptidase N. This adjunct agent also displays mucolytic properties that can be employed within the methods and compositions of the invention to reduce the effects of the mucus diffusion barrier.

Still other useful enzyme inhibitors for use within the coordinate administration methods and combinatorial formulations of the invention may be selected from peptides and modified peptide enzyme inhibitors. An important representative of this class of inhibitors is the cyclic dodecapeptide, bacitracin, obtained from Bacillus licheniformis. In addition to these types of peptides, certain dipeptides and tripeptides display weak, non-specific inhibitory activity towards some protease. By analogy with amino acids, their inhibitory activity can be improved by chemical modifications. For example, phosphinic acid dipeptide analogues are also 'transition-state' inhibitors with a strong inhibitory activity towards aminopeptidases. They have reportedly been used to stabilize nasally administered leucine enkephalin. Another example of a transition-state analogue is the modified pentapeptide pepstatin, which is a very potent inhibitor of pepsin. Structural analysis of pepstatin, by testing the inhibitory activity of several synthetic analogues, demonstrated the major structure-function characteristics of the molecule responsible for the inhibitory activity. Another special type of modified peptide includes inhibitors with a terminally located aldehyde function in their structure. For example, the sequence benzyloxycarbonyl-Pro-Phe-CHO, which fulfills the known primary and secondary specificity requirements of chymotrypsin, has been found to be a potent reversible inhibitor of this target proteinase. The chemical structures of further inhibitors with a terminally located aldehyde function, e.g., antipain, leupeptin, chymostatin and elastatinal, are also known in the art, as are the structures of other known, reversible, modified peptide inhibitors, such as phosphoramidon, bestatin, puromycin and amastatin.

Due to their comparably high molecular mass, polypeptide protease inhibitors are more amenable than smaller compounds to concentrated delivery in a drug-carrier matrix. Additional agents for protease inhibition within the formulations and methods of the invention involve the use of complexing agents. These agents mediate enzyme inhibition by depriving the intranasal environment (or preparative or therapeutic composition) of divalent cations, which are co-factors for many proteases. For instance, the complexing agents EDTA and DTPA as coordinately administered or combinatorially formulated adjunct agents, in suitable concentration, will be sufficient to inhibit selected proteases to thereby enhance intranasal delivery of biologically active agents according to the invention. Further representatives of this class of inhibitory agents are EGTA, 1,10-phenanthroline and hydroxychinoline. In addition, due to their propensity to chelate divalent cations, these and other complexing agents are useful within the invention as direct, absorption-promoting agents.

As noted in more detail elsewhere herein, it is also contemplated to use various polymers, particularly mucoadhesive polymers, as enzyme inhibiting agents within the coordinate administration, multi-processing and/or combinatorial formulation methods and compositions of the invention. For example, poly(acrylate) derivatives, such as poly(acrylic acid) and polycarbophil, can affect the activity of various proteases, including trypsin, chymotrypsin. The inhibitory effect of these polymers may also be based on the complexation of divalent cations such as $Ca^{2+}$ and $Zn^{2+}$. It is further contemplated that these polymers may serve as conjugate partners or carriers for additional enzyme inhibitory agents, as described above. For example, a chitosan-EDTA conjugate has been developed and is useful within the invention that exhibits a strong inhibitory effect towards the enzymatic activity of zinc-dependent proteases. The mucoadhesive properties of polymers following covalent attachment of other enzyme inhibitors in this context are not expected to be substantially compromised, nor is the general utility of such polymers as a delivery vehicle for biologically active agents within the invention expected to be diminished. On the contrary, the reduced distance between the delivery vehicle and mucosal surface afforded by the mucoadhesive mechanism will minimize presystemic metabolism of the active agent, while the covalently bound enzyme inhibitors remain concentrated at the site of drug delivery, minimizing undesired dilution effects of inhibitors as well as toxic and other side effects caused thereby. In this manner, the effective amount of a coordinately administered enzyme inhibitor can be reduced due to the exclusion of dilution effects.

Exemplary mucoadhesive polymer-enzyme inhibitor complexes that are useful within the mucosal formulations and methods of the invention include, but are not limited to: Carboxymethylcellulose-pepstatin (with anti-pepsin activity); Poly(acrylic acid)-Bowman-Birk inhibitor (anti-chymotrypsin); Poly(acrylic acid)-chymostatin (anti-chymotrypsin); Poly(acrylic acid)-elastatinal (anti-elastase); Carboxymethylcellulose-elastatinal (anti-elastase); Polycarboplul-elastatinal (anti-elastase); Chitosan-antipain (anti-trypsin); Poly(acrylic acid)-bacitracin (anti-aminopeptidase N); Chitosan-EDTA (anti-aminopeptidase N, anti-carboxypeptidase A); Chitosan-EDTA-antipain (anti-trypsin, anti-chymotrypsin, anti-elastase).

Mucolytic and Mucus-Clearing Agents and Methods

Effective delivery of biotherapeutic agents via intranasal administration must take into account the decreased drug transport rate across the protective mucus lining of the nasal mucosa, in addition to drug loss due to binding to glycoproteins of the mucus layer. Normal mucus is a viscoelastic, gel-like substance consisting of water, electrolytes, mucins, macromolecules, and sloughed epithelial cells. It serves primarily as a cytoprotective and lubricative covering for the underlying mucosal tissues. Mucus is secreted by randomly distributed secretory cells located in the nasal epithelium and in other mucosal epithelia. The structural unit of mucus is mucin. This glycoprotein is mainly responsible for the viscoelastic nature of mucus, although other macromolecules may also contribute to this property. In airway mucus, such macromolecules include locally produced secretory IgA, IgM, IgE, lysozyme, and bronchotransferrin, which also play an important role in host defense mechanisms.

The coordinate administration methods of the instant invention optionally incorporate effective mucolytic or mucus-clearing agents, which serve to degrade, thin or clear mucus from intranasal mucosal surfaces to facilitate absorption of intranasally administered biotherapeutic agents. Within these methods, a mucolytic or mucus-clearing agent is coordinately administered as an adjunct compound to enhance intranasal delivery of the biologically active agent. Alternatively, an effective amount of a mucolytic or mucus-clearing agent is incorporated as a processing agent within a multi-processing method of the invention, or as an additive within a combinatorial formulation of the invention, to provide an improved formulation that enhances intranasal delivery of biotherapeutic compounds by reducing the barrier effects of intranasal mucus.

A variety of mucolytic or mucus-clearing agents are available for incorporation within the methods and compositions of the invention. Based on their mechanisms of action, mucolytic and mucus clearing agents can often be classified into the following groups: proteases (e.g., pronase, papain) that cleave the protein core of mucin glycoproteins; sulfhydryl compounds that split mucoprotein disulfide linkages; and detergents (e.g., Triton X-100, Tween 20) that break non-covalent bonds within the mucus. Additional compounds in this context include, but are not limited to, bile salts and surfactants, for example, sodium deoxycholate, sodium taurodeoxycholate, sodium glycocholate, and lysophosphatidylcholine.

The effectiveness of bile salts in causing structural breakdown of mucus is in the order deoxycholate > taurocholate > glycocholate. Other effective agents that reduce mucus viscosity or adhesion to enhance intranasal delivery according to the methods of the invention include, e.g., short-chain fatty acids, and mucolytic agents that work by chelation, such as N-acylcollagen peptides, bile acids, and saponins (the latter function in part by chelating $Ca^{2+}$ and/or $Mg^{2+}$ which play an important role in maintaining mucus layer structure).

Additional mucolytic agents for use within the methods and compositions of the invention include N-acetyl-L-cysteine (ACS), a potent mucolytic agent that reduces both the viscosity and adherence of bronchopulmonary mucus and is reported to modestly increase nasal bioavailability of human growth hormone in anesthetized rats (from 7.5 to 12.2%). These and other mucolytic or mucus-clearing agents are contacted with the nasal mucosa, typically in a concentration range of about 0.2 to 20 mM, coordinately with administration of the biologically, active agent, to reduce the polar viscosity and/or elasticity of intranasal mucus.

Still other mucolytic or mucus-clearing agents may be selected from a range of glycosidase enzymes, which are able to cleave glycosidic bonds within the mucus glycoprotein. α-amylase and β-amylase are representative of this class of enzymes, although their mucolytic effect may be limited. In contrast, bacterial glycosidases which allow these microorganisms to permeate mucus layers of their hosts.

For combinatorial use with most biologically active agents within the invention, including peptide and protein therapeutics, non-ionogenic detergents are generally also useful as mucolytic or mucus-clearing agents. These agents typically will not modify or substantially impair the activity of therapeutic polypeptides.

Ciliostatic Agents and Methods

Because the self-cleaning capacity of certain mucosal tissues (e.g., nasal mucosal tissues) by mucociliary clearance is necessary as a protective function (e.g., to remove dust, allergens, and bacteria), it has been generally considered that this function should not be substantially impaired by mucosal medications. Mucociliary transport in the respiratory tract is a particularly important defense mechanism against infections. To achieve this function, ciliary beating in the nasal and airway passages moves a layer of mucus along the mucosa to removing inhaled particles and microorganisms.

Ciliostatic agents find use within the methods and compositions of the invention to increase the residence time of mucosally (e.g., intranasally) administered glucose-regulating peptide, analogs and mimetics, and other biologically active agents disclosed herein. In particular, the delivery these agents within the methods and compositions of the invention is significantly enhanced in certain aspects by the coordinate administration or combinatorial formulation of one or more ciliostatic agents that function to reversibly inhibit ciliary activity of mucosal cells, to provide for a temporary, reversible increase in the residence time of the mucosally administered active agent(s). For use within these aspects of the invention, the foregoing ciliostatic factors, either specific or indirect in their activity, are all candidates for successful employment as ciliostatic agents in appropriate amounts (depending on concentration, duration and mode of delivery) such that they yield a transient (i.e., reversible) reduction or cessation of mucociliary clearance at a mucosal site of administration to enhance delivery of glucose-regulating peptide, analogs and mimetics, and other biologically active agents disclosed herein, without unacceptable adverse side effects.

Within more detailed aspects, a specific ciliostatic factor is employed in a combined formulation or coordinate administration protocol with one or more glucose-regulating peptide proteins, analogs and mimetics, and/or other biologically active agents disclosed herein. Various bacterial ciliostatic factors isolated and characterized in the literature may be employed within these embodiments of the invention. Ciliostatic factors from the bacterium *Pseudomonas aeruginosa* include a phenazine derivative, a pyo compound (2-alkyl-4-hydroxyquinolines), and a rhamnolipid (also known as a hemolysin). The pyo compound produced ciliostasis at concentrations of 50 μg/ml and without obvious ultrastructural lesions. The phenazine derivative also inhibited ciliary motility but caused some membrane disruption, although at substantially greater concentrations of 400 μg/ml. Limited exposure of tracheal explants to the rhamnolipid resulted in ciliostasis, which is associated with altered ciliary membranes. More extensive exposure to rhamnolipid is associated with removal of dynein arms from axonemes.

Surface Active Agents and Methods

Within more detailed aspects of the invention, one or more membrane penetration-enhancing agents may be employed within a mucosal delivery method or formulation of the invention to enhance mucosal delivery of glucose-regulating peptide proteins, analogs and mimetics, and other biologically active agents disclosed herein. Membrane penetration enhancing agents in this context can be selected from: (i) a surfactant; (ii) a bile salt; (iii) a phospholipid additive, mixed micelle, liposome, or carrier; (iv) an alcohol; (v) an enamine; (vi) an NO donor compound; (vii) a long-chain amphipathic molecule; (viii) a small hydrophobic penetration enhancer; (ix) sodium or a salicylic acid derivative; (x) a glycerol ester of acetoacetic acid; (xi) a clyclodextrin or beta-cyclodextrin derivative; (xii) a medium-chain fatty acid; (xiii) a chelating agent; (xiv) an amino acid or salt thereof; (xv) an N-acetylamino acid or salt thereof; (xvi) an enzyme degradative to a selected membrane component; (xvii) an inhibitor of fatty acid synthesis; (xviii) an inhibitor of cholesterol synthesis; or (xix) any combination of the membrane penetration enhancing agents recited in (i)-(xviii).

Certain surface-active agents are readily incorporated within the mucosal delivery formulations and methods of the invention as mucosal absorption enhancing agents. These agents, which may be coordinately administered or combinatorially formulated with glucose-regulating peptide proteins, analogs and mimetics, and other biologically active agents disclosed herein, may be selected from a broad assemblage of known surfactants. Surfactants, which generally fall into three classes: (1) nonionic polyoxyethylene ethers; (2) bile salts such as sodium glycocholate (SGC) and deoxycholate (DOC); and (3) derivatives of fusidic acid such as sodium taurodihydrofusidate (STDHF). The mechanisms of action of these various classes of surface-active agents typically include solubilization of the biologically active agent. For proteins and peptides which often form aggregates, the surface active properties of these absorption promoters can allow interactions with proteins such that smaller units such as surfactant coated monomers may be more readily maintained in solution. Examples of other surface-active agents are L-α-Phosphatidylcholine Didecanoyl (DDPC), polysorbate 80 and polysorbate 20. These monomers are presumably more transportable units than aggregates. A second potential mechanism is the protection of the peptide or protein from proteolytic degradation by proteases in the mucosal environment. Both bile salts and some fusidic acid derivatives reportedly inhibit proteolytic degradation of proteins by nasal homogenates at concentrations less than or equivalent to those required to enhance protein absorption. This protease inhibition may be especially important for peptides with short biological half-lives.

Thus, some examples of surface active agents include nonionic polyoxyethylene ether, fusidic acid and its derivatives, sodium taurodihydrofusidate, L-α-phosphatidylcholine didecanoyl, polysorbate 80, polysorbate 20, polyethylene glycol, cetyl alcohol, polyvinylpyrolidone, polyvinyl alcohol, lanolin alcohol, sorbitan monooleate, and mixtures thereof.

Viscosity Enhancing Agents

Viscosity enhancing or suspending agents may affect the rate of release of a drug from the dosage formulation and absorption. Some examples of the materials which can serve as pharmaceutically acceptable viscosity enhancing agents are gelatin; methylcellulose (MC); hydroxypropylmethylcellulose (HPMC); carboxymethylcellulose (CMC); cellulose; starch; heta starch; poloxamers; pluronics; sodium CMC; sorbitol; acacia; povidone; carbomer; polycarbophil; chitosan; clutosan microspheres; alginate microspheres; chitosan glutamate; amberlite resin; hyaluronan; ethyl cellulose; maltodextrin DE; drum-dried way maize starch (DDWM); degradable starch microspheres (DSM); deoxyglycocholate (GDC); hydroxyethyl cellulose (HEC); hydroxypropyl cellulose (HPC); microcrystalline cellulose (MCC); polymethacrylic acid and polyethylene glycol; sulfobutylether B cyclodextrin; cross-linked eldexomer starch biospheres; sodiumtaurodihydrofusidate (STDHF); N-trimethyl chitosan chloride (TMC); degraded starch microspheres; amberlite resin; chistosan nanoparticles; spray-dried crospovidone; spray-dried dextran microspheres; spray-dried-microcrystalline cellulose; and cross-linked eldexomer starch microspheres. Other viscosity enhancing agents in Ugwoke, et al., Adv. Drug Deliv. Rev. 29:1656-57, 1998, are incorporated by reference.

Degradation Enzymes and Inhibitors of Fatty Acid and Cholesterol Syntheses

In related aspects of the invention, glucose-regulating peptide proteins, analogs and mimetics, and other biologically active agents for mucosal administration are formulated or coordinately administered with a penetration enhancing agent selected from a degradation enzyme, or a metabolic stimulatory agent or inhibitor of synthesis of fatty acids, sterols or other selected epithelial barrier components, U.S. Patent No. 6,190,894. For example, degradative enzymes such as phospholipase, hyaluronidase, neuraminidase, and chondroitinase may be employed to enhance mucosal penetration of glucose-regulating peptide proteins, analogs and mimetics, and other biologically active agent without causing irreversible damage to the mucosal barrier. In one embodiment, chondroitinase is employed within a method or composition as provided herein to alter glycoprotein or glycolipid constituents of the permeability barrier of the mucosa, thereby enhancing mucosal absorption of glucose-regulating peptide proteins, analogs and mimetics, and other biologically active agents disclosed herein.

With regard to inhibitors of synthesis of mucosal barrier constituents, it is noted that free fatty acids account for 20-25% of epithelial lipids by weight. Two rate-limiting enzymes in the biosynthesis of free fatty acids are acetyl CoA carboxylase and fatty acid synthetase. Through a series of steps, free fatty acids are metabolized into phospholipids. Thus, inhibitors of free fatty acid synthesis and metabolism for use within the methods and compositions of the invention include, but are not limited to, inhibitors of acetyl CoA carboxylase such as 5-tetradecyloxy-2-furancarboxylic acid (TOFA); inhibitors of fatty acid synthetase; inhibitors of phospholipase A such as gomisin A, 2-(p-amylcinnamyl)amino-4-chlorobenzoic acid, bromophenacyl bromide, monoalide, 7,7-dimethyl-5,8-eicosadienoic acid, nicergoline, cepharanthine, nicardipine, quercetin, dibutyryl-cyclic AMP, R-24571, N-oleoylethanolamine, N-(7-nitro-2,1,3-benzoxadiazol-4-yl) phosphostidyl serine, cyclosporine A, topical anesthetics, including dibucaine, prenylamine, retinoids, such as all-trans and 13-cis-retinoic acid, W-7, trifluoperazine, R-24571 (calmidazolium), 1-hexadocyl-3-trifluoroethyl glycero-sn-2-phosphomenthol (MJ33); calcium channel blockers including nicardipine, verapamil, diltiazem, nifedipine, and nimodipine; antimalarials including quinacrine, mepacrine, chloroquine and hydroxychloroquine; beta blockers including propanalol and labetalol; calmodulin antagonists; EGTA; thimersol; glucocorticosteroids including dexamethasone and prednisolone; and nonsteroidal antiinflammatory agents including indomethacin and naproxen.

Free sterols, primarily cholesterol, account for 20-25% of the epithelial lipids by weight. The rate limiting enzyme in the biosynthesis of cholesterol is 3-hydroxy-3-methylglutaryl (HMG) CoA reductase. Inhibitors of cholesterol synthesis for use within the methods and compositions of the invention include, but are not limited to, competitive inhibitors of (HMG) CoA reductase, such as simvastatin, lovastatin, fluindostatin (fluvastatin), pravastatin, mevastatin, as well as other HMG CoA reductase inhibitors, such as cholesterol oleate, cholesterol sulfate and phosphate, and oxygenated sterols, such as 25-OH-- and 26-OH-cholesterol; inhibitors of squalene synthetase; inhibitors of squalene epoxidase; inhibitors of DELTA7 or DELTA24 reductases such as 22,25-diazacholesterol, 20,25-diazacholestenol, AY9944, and triparanol.

Each of the inhibitors of fatty acid synthesis or the sterol synthesis inhibitors maybe coordinately administered or combinatorially formulated with one or more glucose-regulating peptide proteins, analogs and mimetics, and other biologically active agents disclosed herein to achieve enhanced epithelial penetration of the active agent(s). An effective concentration range for the sterol inhibitor in a therapeutic or adjunct formulation for mucosal delivery is generally from about 0.0001 % to about 20% by weight of the total, more typically from about 0.01 % to about 5%.

Nitric Oxide Donor Agents and Methods

Within other related aspects of the invention, a nitric oxide (NO) donor is selected as a membrane penetration-enhancing agent to enhance mucosal delivery of one or more glucose-regulating peptide proteins, analogs and mimetics, and other biologically active agents disclosed herein. Various NO donors are known in the art and are useful in effective concentrations within the methods and formulations of the invention. Exemplary NO donors include, but are not limited to, nitroglycerine, nitropruside, NOC5 [3-(2-hydroxy-1-(methyl-ethyl)-2-nitrosohydrazino)-1-propanamine], NOCl2 [N-ethyl-2-(1-ethyl-hydroxy-2-nitrosohydrazino)-ethanamine], SNAP [S-nitroso-N-acetyl-DL-penicillamine], NORI and NOR4. Within the methods and compositions of the invention, an effective amount of a selected NO donor is coordinately administered or combinatorially formulated with one or more glucose-regulating peptide proteins, analogs and mimetics, and/or other biologically active agents disclosed herein, into or through the mucosal epithelium.

Agents for Modulating Epithelial Junction Structure and/or Physiology

The present invention provides pharmaceutical composition that contains one or more glucose-regulating peptides, proteins, analogs or mimetics, and/or other biologically active agents in combination with mucosal delivery enhancing agents disclosed herein formulated in a pharmaceutical preparation for mucosal delivery.

The permeabilizing agent reversibly enhances mucosal epithelial paracellular transport, typically by modulating epithelial junctional structure and/or physiology at a mucosal epithelial surface in the subject. This effect typically involves inhibition by the permeabilizing agent of homotypic or heterotypic binding between epithelial membrane adhesive proteins of neighboring epithelial cells. Target proteins for this blockade of homotypic or heterotypic binding can be selected from various related junctional adhesion molecules (JAMs), occludins, or claudins. Examples of this are antibodies, antibody fragments or single-chain antibodies that bind to the extracellular domains of these proteins.

In yet additional detailed embodiments, the invention provides permeabilizing peptides and peptide analogs and mimetics for enhancing mucosal epithelial paracellular transport. The subject peptides and peptide analogs and mimetics typically work within the compositions and methods of the invention by modulating epithelial junctional structure and/or physiology in a mammalian subject. In certain embodiments, the peptides and peptide analogs and mimetics effectively inhibit homotypic and/or heterotypic binding of an epithelial membrane adhesive protein selected from a junctional adhesion molecule (JAM), occludin, or claudin.

One such agent that has been extensively studied is the bacterial toxin from *Vibrio cholerae* known as the "zonula occludens toxin" (ZOT). This toxin mediates increased intestinal mucosal permeability and causes disease symptoms including diarrhea in infected subjects. Fasano, et al., Proc. Nat. Acad. Sci., U.S.A. 8:5242-5246, 1991. When tested on rabbit ileal mucosa, ZOT increased the intestinal permeability by modulating the structure of intercellular tight junctions. More recently, it has been found that ZOT is capable of reversibly opening tight junctions in the intestinal mucosa. It has also been reported that ZOT is capable of reversibly opening tight junctions in the nasal mucosa. U.S. Patent No. 5,908,825.

Within the methods and compositions of the invention, ZOT, as well as various analogs and mimetics of ZOT that function as agonists or antagonists of ZOT activity, are useful for enhancing intranasal delivery of biologically active agent-by increasing paracellular absorption into and across the nasal mucosa. In this context, ZOT typically acts by causing a structural reorganization of tight junctions marked by altered localization of the junctional protein ZO1. Within these aspects of the invention, ZOT is coordinately administered or combinatorially formulated with the biologically active agent in an effective amount to yield significantly enhanced absorption of the active agent, by reversibly increasing nasal mucosal permeability without substantial adverse side effects.

Vasodilator Agents and Methods

Yet another class of absorption-promoting agents that shows beneficial utility within the coordinate administration and combinatorial formulation methods and compositions of the invention are vasoactive compounds, more specifically vasodilators. These compounds function within the invention to modulate the structure and physiology of the submucosal vasculature, increasing the transport rate of glucose-regulating peptide, analogs and mimetics, and other biologically active agents into or through the mucosal epithelium and/or to specific target tissues or compartments (e.g., the systemic circulation or central nervous system).

Vasodilator agents for use within the invention typically cause submucosal blood vessel relaxation by either a decrease in cytoplasmic calcium, an increase in nitric oxide (NO) or by inhibiting myosin light chain kinase. They are generally divided into 9 classes: calcium antagonists, potassium channel openers, ACE inhibitors, angiotensin-II receptor antagonists, α-adrenergic and imidazole receptor antagonists, β1-adrenergic agonists, phosphodiesterase inhibitors, eicosanoids and NO donors.

Despite chemical differences, the pharmacokinetic properties of calcium antagonists are similar. Absorption into the systemic circulation is high, and these agents therefore undergo considerable first-pass metabolism by the liver, resulting in individual variation in pharmacokinetics. Except for the newer drugs of the dihydropyridine type (amlodipine, felodipine, isradipine, nilvadipine, nisoldipine and nitrendipine), the half-life of calcium antagonists is short. Therefore, to maintain an effective drug concentration for many of these may require delivery by multiple dosing, or controlled release formulations, as described elsewhere herein. Treatment with the potassium channel opener minoxidil may also be limited in manner and level of administration due to potential adverse side effects.

ACE inhibitors prevent conversion of angiotemsin-I to angiotensin-II, and are most effective when renin production is increased. Since ACE is identical to kininase-II, which inactivates the potent endogenous vasodilator bradykinin, ACE inhibition causes a reduction in bradykinin degradation. ACE inhibitors provide the added advantage of cardioprotective and cardioreparative effects, by preventing and reversing cardiac fibrosis and ventricular hypertrophy in animal models. The predominant elimination pathway of most ACE inhibitors is via renal excretion. Therefore, renal impairment is associated with reduced elimination and a dosage reduction of 25 to 50% is recommended in patients with moderate to severe renal impairment.

With regard to NO donors, these compounds are particularly useful within the invention for their additional effects on mucosal permeability. In addition to the above-noted NO donors, complexes of NO with nucleophiles called NO/nucleophiles, or NONOates, spontaneously and nonenzymatically release NO when dissolved in aqueous solution at physiologic pH. In contrast, nitro vasodilators such as nitroglycerin require specific enzyme activity for NO release. NONOates release NO with a defined stoichiometry and at predictable rates ranging from <3 minutes for diethylamine/NO to approximately 20 hours for diethylenetriamine/NO (DETANO).

Within certain methods and compositions of the invention, a selected vasodilator agent is coordinately administered (e.g., systemically or intranasally, simultaneously or in combinatorially effective temporal association) or combinatorially formulated with one or more glucose-regulating peptide, analogs and mimetics, and other biologically active agent(s) in an amount effective to enhance the mucosal absorption of the active agent(s) to reach a target tissue or compartment in the subject (e.g., the liver, hepatic portal vein, CNS tissue or fluid, or blood plasma).

Selective Transport-Enhancing Agents and Methods

The compositions and delivery methods of the invention optionally incorporate a selective transport-enhancing agent that facilitates transport of one or more biologically active agents. These transport-enhancing agents may be employed in a combinatorial formulation or coordinate administration protocol with one or more of the glucose-regulating peptide proteins, analogs and mimetics disclosed herein, to coordinately enhance delivery of one or more additional biologically active agent(s) across mucosal transport barriers, to enhance mucosal delivery of the active agent(s) to reach a target tissue or compartment in the subject (e.g., the mucosal epithelium, liver, CNS tissue or fluid, or blood plasma). Alternatively, the transport-enhancing agents may be employed in a combinatorial formulation or coordinate administration protocol to directly enhance mucosal delivery of one or more of the glucose-regulating peptide proteins, analogs and mimetics, with or without enhanced delivery of an additional biologically active agent.

Exemplary selective transport-enhancing agents for use within this aspect of the invention include, but are not limited to, glycosides, sugar-containing molecules, and binding agents such as lectin binding agents, which are known to interact specifically with epithelial transport barrier components. For example, specific "bioadhesive" ligands, including various plant and bacterial lectins, which bind to cell surface sugar moieties by receptor-mediated interactions can be employed as carriers or conjugated transport mediators for enhancing mucosal, e.g., nasal delivery of biologically active agents within the invention. Certain bioadhesive ligands for use within the invention will mediate transmission of biological signals to epithelial target cells that trigger selective uptake of the adhesive ligand by specialized cellular transport processes (endocytosis or transcytosis). These transport mediators can therefore be employed as a "carrier system" to stimulate or direct selective uptake of one or more glucose-regulating peptide proteins, analogs and mimetics, and other biologically active agent(s) into and/or through mucosal epithelia. These and other selective transport-enhancing agents significantly enhance mucosal delivery of macromolecular biopharmaceuticals (particularly peptides, proteins, oligonucleotides and polynucleotide vectors) within the invention. Lectins are plant proteins that bind to specific sugars found on the surface of glycoproteins and glycolipids of eukaryotic cells. Concentrated solutions of lectins have a 'mucotractive' effect, and various studies have demonstrated rapid receptor mediated endocytocis (RME) of lectins and lectin conjugates (e.g., concanavalin A conjugated with colloidal gold particles) across mucosal surfaces. Additional studies have reported that the uptake mechanisms for lectins can be,utilized for intestinal drug targeting *in vivo.* In certain of these studies, polystyrene nanoparticles (500 nm) were covalently coupled to tomato lectin and reported yielded improved systemic uptake after oral administration to rats.

In addition to plant lectins, microbial adhesion and invasion factors provide a rich source of candidates for use as adhesive/selective transport carriers within the mucosal delivery methods and compositions of the invention. Two components are necessary for bacterial adherence processes, a bacterial 'adhesin' (adherence or colonization factor) and a receptor on the host cell surface. Bacteria causing mucosal infections need to penetrate the mucus layer before attaching themselves to the epithelial surface. This attachment is usually mediated by bacterial fimbriae or pilus structures, although other cell surface components may also take part in the process. Adherent bacteria colonize mucosal epithelia by multiplication and initiation of a series of biochemical reactions inside the target cell through signal transduction mechanisms (with or without the help of toxins). Associated with these invasive mechanisms, a wide diversity of bioadhesive proteins (e.g., invasin, internalin) originally produced by various bacteria and viruses are known. These allow for extracellular attachment of such microorganisms with an impressive selectivity for host species and even particular target tissues. Signals transmitted by such receptor-ligand interactions trigger the transport of intact, living microorganisms into, and eventually through, epithelial cells by endo- and transcytotic processes. Such naturally occurring phenomena may be harnessed (e.g., by complexing biologically active agents such as glucose-regulating peptide with an adhesin) according to the teachings herein for enhanced delivery of biologically active compounds into or across mucosal epithelia and/or to other designated target sites of drug action.

Various bacterial and plant toxins that bind epithelial surfaces in a specific, lectin-like manner are also useful within the methods and compositions of the invention. For example, diptheria toxin (DT) enters host cells rapidly by RME. Likewise, the B subunit of the *E. coli* heat labile toxin binds to the brush border of intestinal epithelial cells in a highly specific, lectin-like manner. Uptake of this toxin and transcytosis to the basolateral side of the enterocytes has been reported *in vivo* and *in vitro.* Other researches have expressed the transmembrane domain of diphtheria toxin in *E. coli* as a maltose-binding fusion protein and coupled it chemically to high-Mw poly-L-lysine. The resulting complex is successfully used to mediate internalization of a reporter gene *in vitro.* In addition to these examples, *Staphylococcus aureus* produces a set of proteins (e.g., staphylococcal enterotoxin A (SEA), SEB, toxic shock syndrome toxin 1 (TSST-1) which act both as superantigens and toxins. Studies relating to these proteins have reported dose-dependent, facilitated transcytosis of SEB and TSST- 1 in Caco-2 cells.

Viral haemagglutinins comprise another type of transport agent to facilitate mucosal delivery of biologically active agents within the methods and compositions of the invention. The initial step in many viral infections is the binding of surface proteins (haemagglutinins) to mucosal cells. These binding proteins have been identified for most viruses, including rotaviruses, varicella zoster virus, semliki forest virus, adenoviruses, potato leafroll virus, and reovirus. These and other exemplary viral hemagglutinins can be employed in a combinatorial formulation (e.g., a mixture or conjugate formulation) or coordinate administration protocol with one or more of the glucose-regulating peptide, analogs and mimetics disclosed herein, to coordinately enhance mucosal delivery of one or more additional biologically active agent (s). Alternatively, viral hemagglutinins can be employed in a combinatorial formulation or coordinate administration protocol to directly enhance mucosal delivery of one or more of the glucose-regulating peptide proteins, analogs and mimetics, with or without enhanced delivery of an additional biologically active agent.

A variety of endogenous, selective transport-mediating factors are also available for use within the invention. Mammalian cells have developed an assortment of mechanisms to facilitate the internalization of specific substrates and target these to defined compartments. Collectively, these processes of membrane deformations are termed 'endocytosis' and comprise phagocytosis, pinocytosis, receptor-mediated endocytosis (clathrin-mediated RME), and potocytosis (non-clathrin-mediated RME). RME is a highly specific cellular biologic process by which, as its name implies, various ligands bind to cell surface receptors and are subsequently internalized and trafficked within the cell. In many cells the process of endocytosis is so active that the entire membrane surface is internalized and replaced in less than a half hour. Two classes of receptors are proposed based on their orientation in the cell membrane; the amino terminus of Type I receptors is located on the extracellular side of the membrane, whereas Type II receptors have this same protein tail in the intracellular milieu.

Still other embodiments of the invention utilize transferrin as a carrier or stimulant of RME of mucosally delivered biologically active agents. Transferrin, an 80 kDa iron-transporting glycoprotein, is efficiently taken up into cells by RME. Transferrin receptors are found on the surface of most proliferating cells, in elevated numbers on erythroblasts and on many kinds of tumors. The transcytosis of transferrin (Tf) and transferrin conjugates is reportedly enhanced in the presence of Brefeldin A (BFA), a fungal metabolite. In other studies, BFA treatment has been reported to rapidly increase apical endocytosis of both ricin and HRP in MDCK cells. Thus, BFA and other agents that stimulate receptor-mediated transport can be employed within the methods of the invention as combinatorially formulated (e.g., conjugated) and/or coordinately administered agents to enhance receptor-mediated transport of biologically active agents, including glucose-regulating peptide proteins, analogs and mimetics.

Polymeric Delivery Vehicles and Methods

Within certain aspects of the invention, glucose-regulating peptide proteins, analogs and mimetics, other biologically active agents disclosed herein, and delivery-enhancing agents as described above, are, individually or combinatorially, incorporated within a mucosally (e.g., nasally) administered formulation that includes a biocompatible polymer functioning as a carrier or base. Such polymer carriers include polymeric powders, matrices or microparticulate delivery vehicles, among other polymer forms. The polymer can be of plant, animal, or synthetic origin. Often the polymer is crosslinked. Additionally, in these delivery systems the glucose-regulating peptide, analog or mimetic, can be functionalized in a manner where it can be covalently bound to the polymer and rendered inseparable from the polymer by simple ishing. In other embodiments, the polymer is chemically modified with an inhibitor of enzymes or other agents which may degrade or inactivate the biologically active agent(s) and/or delivery enhancing agent(s). In certain formulations, the polymer is a partially or completely water insoluble but water swellable polymer, e.g., a hydrogel. Polymers useful in this aspect of the invention are desirably water interactive and/or hydrophilic in nature to absorb significant quantities of water, and they often form hydrogels when placed in contact with water or aqueous media for a period of time sufficient to reach equilibrium with water. In more detailed embodiments, the polymer is a hydrogel which, when placed in contact with excess water, absorbs at least two times its weight of water at equilibrium when exposed to water at room temperature, U.S. Patent No. 6,004,583.

Drug delivery systems based on biodegradable polymers are preferred in many biomedical applications because such systems are broken down either by hydrolysis or by enzymatic reaction into non-toxic molecules. The rate of degradation is controlled by manipulating the composition of the biodegradable polymer matrix. These types of systems can therefore be employed in certain settings for long-term release of biologically active agents. Biodegradable polymers such as poly(glycolic acid) (PGA), poly-(lactic acid) (PLA), and poly(D,L-lactic-co-glycolic acid) (PLGA), have received considerable attention as possible drug delivery carriers, since the degradation products of these polymers have been found to have low toxicity. During the normal metabolic function, of the body these polymers degrade into carbon dioxide and water. These polymers have also exhibited excellent biocompatibility.

For prolonging the biological activity of glucose-regulating peptide, analogs and mimetics, and other biologically active agents disclosed herein, as well as optional delivery-enhancing agents, these agents may be incorporated into polymeric matrices, e.g., polyorthoesters, polyanhydrides, or polyesters. This yields sustained activity and release of the active agent(s), e.g., as determined by the degradation of the polymer matrix. Although the encapsulation ofbiotherapeutic molecules inside synthetic polymers may stabilize them during storage and delivery, the largest obstacle of polymer-based release technology is the activity loss of the therapeutic molecules during the formulation processes that often involve heat, sonication or organic solvents.

Absorption-promoting polymers contemplated for use within the invention may include derivatives and chemically or physically modified versions of the foregoing types of polymers, in addition to other naturally occurring or synthetic polymers, gums, resins, and other agents, as well as blends of these materials with each other or other polymers, so long as the alterations, modifications or blending do not adversely affect the desired properties, such as water absorption, hydrogel formation, and/or chemical stability for useful application. In more detailed aspects of the invention, polymers such as nylon, acrylan and other normally hydrophobic synthetic polymers may be sufficiently modified by reaction to become water swellable and/or form stable gels in aqueous media.

Absorption-promoting polymers of the invention may include polymers from the group of homo- and copolymers based on various combinations of the following vinyl monomers: acrylic and methacrylic acids, acrylamide, methacrylamide, hydroxyethylacrylate or methacrylate, vinylpyn-olidones, as well as polyvinylalcohol and its co- and terpolymers, polyvinylacetate, its co- and terpolymers with the above listed monomers and 2-acrylamido-2-methyl-propanesulfonic acid (AMPS®). Very useful are copolymers of the above listed monomers with copolymerizable functional monomers such as acryl or methacryl amide acrylate or methacrylate esters where the ester groups are derived from straight or branched chain alkyl, aryl having up to four aromatic rings which may contain alkyl substituents of 1 to 6 carbons; steroidal, sulfates, phosphates or cationic monomers such as N,N-dimethylaminoalkyl(meth)acrylamide, dimethylaminoalkyl(meth)acrylate, (meth)acryloxyalkyltrimethylammonium chloride, (meth)acryloxyalkyldimethylbenzyl ammonium chloride.

Additional absorption-promoting polymers for use within the invention are those classified as dextrans, dextrins, and from the class of materials classified as natural gums and resins, or from the class of natural polymers such as processed collagen, chitin, chitosan, , pullalan, zooglan, alginates and modified alginates such as "Kelcoloid" (a polypropylene glycol modified alginate) gellan gums such as "Kelocogel", Xanathan gums such as "Keltrol", estastin, alpha hydroxy butyrate and its copolymers, hyaluronic acid and its derivatives, polylactic and glycolic acids.

A very useful class of polymers applicable within the instant invention are olefinically-unsaturated carboxylic acids containing at least one activated carbon-to-carbon olefinic double bond, and at least one carboxyl group; that is, an acid or functional group readily converted to an acid containing an olefinic double bond which readily functions in polymerization because of its presence in the monomer molecule, either in the alpha-beta position with respect to a carboxyl group, or as part of a terminal methylene grouping. Olefinically-unsaturated acids of this class include such materials as the acrylic acids typified by the acrylic acid itself, alpha-cyano acrylic acid, beta methylacrylic acid (crotonic acid), alpha-phenyl acrylic acid, beta-acryloxy propionic acid, cinnamic acid, p-chloro cinnamic acid, 1-carboxy-4-phenyl butadiene-1,3, itaconic acid, citraconic acid, mesaconic acid, glutaconic acid, aconitic acid, maleic acid, fumaric acid, and tricarboxy ethylene. As used herein, the term "carboxylic acid" includes the polycarboxylic acids and those acid anhydrides, such as maleic anhydride, wherein the anhydride group is formed by the elimination of one molecule of water from two carboxyl groups located on the same carboxylic acid molecule.

Representative acrylates useful as absorption-promoting agents within the invention include methyl acrylate, ethyl acrylate, propyl acrylate, isopropyl acrylate, butyl acrylate, isobutyl acrylate, methyl methacrylate, methyl ethacrylate, ethyl methacrylate, octyl acrylate, heptyl acrylate, octyl methacrylate, isopropyl methacrylate, 2-ethylhexyl methacrylate, nonyl acrylate, hexyl acrylate, n-hexyl methacrylate, and the like. Higher alkyl acrylic esters are decyl acrylate, isodecyl methacrylate, lauryl acrylate, stearyl acrylate, behenyl acrylate and melissyl acrylate and methacrylate versions thereof. Mixtures of two or three or more long chain acrylic esters may be successfully polymerized with one of the carboxylic monomers. Other comonomers include olefins, including alpha olefins, vinyl ethers, vinyl esters, and mixtures thereof.

Other vinylidene monomers, including the acrylic nitriles, may also be used as absorption-promoting agents within the methods and compositions of the invention to enhance delivery and absorption of one or more glucose-regulating peptide proteins, analogs and mimetics, and other biologically active agent(s), including to enhance delivery of the active agent(s) to a target tissue or compartment in the subject (e.g., the liver, hepatic portal vein, CNS tissue or fluid, or blood plasma). Useful alpha, beta-olefinically unsaturated nitriles are preferably monoolefinically unsaturated nitriles having from 3 to 10 carbon atoms such as acrylonitrile, methacrylonitrile, and the like. Most preferred are acrylonitrile and methacrylonitrile. Acrylic amides containing from 3 to 35 carbon atoms including monoolefinically unsaturated amides also may be used. Representative amides include acrylamide, methacrylamide, N-t-butyl acrylamide, N-cyclohexyl acrylamide, higher alkyl amides, where the alkyl group on the nitrogen contains from 8 to 32 carbon atoms, acrylic amides including N-alkylol amides of alpha, beta-olefinically unsaturated carboxylic acids including those having from 4 to 10 carbon atoms such as N-methylol acrylamide, N-propanol acrylamide, N-methylol methacrylamide, N-methylol maleimide, N-methylol maleamic acid esters, N-methylol-p-vinyl benzamide, and the like.

Yet additional useful absorption promoting materials are alpha-olefins containing from 2 to 18 carbon atoms, more preferably from 2 to 8 carbon atoms; dienes containing from 4 to 10 carbon atoms; vinyl esters and allyl esters such as vinyl acetate; vinyl aromatics such as styrene, methyl styrene and chloro-styrene; vinyl and allyl ethers and ketones such as vinyl methyl ether and methyl vinyl ketone; chloroacrylates; cyanoalkyl acrylates such as alpha-cyanomethyl acrylate, and the alpha-, beta-, and gamma-cyanopropyl acrylates; alkoxyacrylates such as methoxy ethyl acrylate; haloacrylates as chloroethyl acrylate; vinyl halides and vinyl chloride, vinylidene chloride and the like; divinyls, diacrylates and other polyfunctional monomers such as divinyl ether, diethylene glycol diacrylate, ethylene glycol dimethacrylate, methylene-bis-acrylamide, allylpentaerythritol, and the like; and bis (beta-haloalkyl) alkenyl phosphonates such as bis(beta-chloroethyl) vinyl phosphonate and the like as are known to those skilled in the art. Copolymers wherein the carboxy containing monomer is a minor constituent, and the other vinylidene monomers present as major components are readily prepared in accordance with the methods disclosed herein.

When hydrogels are employed as absorption promoting agents within the invention, these maybe composed of synthetic copolymers from the group of acrylic and methacrylic acids, acrylamide, methacrylamide, hydroxyethylacrylate (HEA) or methacrylate (HEMA), and vinylpyrrolidones which are water interactive and swellable. Specific illustrative examples of useful polymers, especially for the delivery of peptides or proteins, are the following types of polymers: (meth)acrylamide and 0.1 to 99 wt. % (meth)acrylic acid; (meth)acrylamides and 0.1-75 wt % (meth)acryloxyethyl trimethyammonium chloride; (meth)acrylamide and 0.1-75 wt % (meth)acrylamide; acrylic acid and 0.1-75 wt % alkyl(meth) acrylates; (meth)acrylamide and 0.1-75 wt % AMPS.RTM. (trademark of Lubrizol Corp.); (meth)acrylamide and 0 to 30 wt % alkyl(meth)acrylamides and 0.1-75 wt % AMPS.RTM.; (meth)acrylamide and 0.1-99 wt. % HEMA; (metb)acrylamide and 0.1 to 75 wt % HEMA and 0.1 to 99%(meth)acrylic acid; (meth)acrylic acid and 0.1-99 wt % HEMA; 50 mole % vinyl ether and 50 mole % maleic anhydride; (meth)acrylamide and 0.1 to 75 wt % (meth)acryloxyalky dimethyl benzylammonium chloride; (meth)acrylamide and 0.1 to 99 wt % vinyl pyrrolidone; (meth)acrylamide and 50 wt % vinyl pyrrolidone and 0.1-99.9 wt % (meth)acrylic acid; (meth)acrylic acid and 0.1 to 75 wt % AMPS.RTM. and 0.1-75 wt % alkyl(meth) acrylamide. In the above examples, alkyl means $C_1$ to $C_{30}$, preferably $C_1$ to $C_{22}$, linear and branched and $C_4$ to $C_{16}$ cyclic; where (meth) is used, it means that the monomers with and without the methyl group are included. Other very useful hydrogel polymers are swellable, but insoluble versions of poly(vinyl pyrrolidone) starch, carboxymethyl cellulose and polyvinyl alcohol.

Additional polymeric hydrogel materials useful within the invention include (poly) hydroxyalkyl (meth)acrylate: anionic and cationic hydrogels: poly(electrolyte) complexes; poly(vinyl alcohols) having a low acetate residual: a swellable mixture of crosslinked agar and crosslinked carboxymethyl cellulose: a swellable composition comprising methyl cellulose mixed with a sparingly crosslinked agar; a water swellable copolymer produced by a dispersion of finely divided copolymer of maleic anhydride with styrene, ethylene, propylene, or isobutylene; a water swellable polymer of N-vinyl lactams; swellable sodium salts of carboxymethyl cellulose; and the like.

Other gelable, fluid imbibing and retaining polymers useful for forming the hydrophilic hydrogel for mucosal delivery of biologically active agents within the invention include pectin; polysaccharides such as agar, acacia, karaya, tragacenth, algins and guar and their crosslinked versions; acrylic acid polymers, copolymers and salt derivatives, polyacrylamides; water swellable indene maleic anhydride polymers; starch graft copolymers; acrylate type polymers and copolymers with water absorbability of about 2 to 400 times its original weight; diesters of polyglucan; a mixture of crosslinked poly(vinyl alcohol) and poly(N-vinyl-2-pyrrolidone); polyoxybutylene-polyethylene block copolymer gels; carob gum; polyester gels; poly urea gels; polyether gels; polyamide gels; polyimide gels; polypeptide gels; polyamino acid gels; poly cellulosic gels; crosslinked indene-maleic anhydride acrylate polymers; and polysaccharides.

Synthetic hydrogel polymers for use within the invention may be made by an infinite combination of several monomers in several ratios. The hydrogel can be crosslinked and generally possesses the ability to imbibe and absorb fluid and swell or expand to an enlarged equilibrium state. The hydrogel typically swells or expands upon delivery to the nasal mucosal surface, absorbing about 2-5, 5-10,10-50, up to 50-100 or more times fold its weight of water. The optimum degree of swellability for a given hydrogel will be determined for different biologically active agents depending upon such factors as molecular weight, size, solubility and diffusion characteristics of the active agent carried by or entrapped or encapsulated within the polymer, and the specific spacing and cooperative chain motion associated with each individual polymer.

Hydrophilic polymers useful within the invention are water insoluble but water swellable. Such water-swollen polymers as typically referred to as hydrogels or gels. Such gels may be conveniently produced from water-soluble polymer by the process of cross-linking the polymers by a suitable cross-linking agent. However, stable hydrogels may also be formed from specific polymers under defined conditions of pH, temperature and/or ionic concentration, according to known methods in the art. Typically the polymers are cross-linked, that is, cross-linked to the extent that the polymers possess good hydrophilic properties, have improved physical integrity (as compared to non cross-linked polymers of the same or similar type) and exhibit improved ability to retain within the gel network both the biologically active agent of interest and additional compounds for coadministration therewith such as a cytokine or enzyme inhibitor, while retaining the ability to release the active agent(s) at the appropriate location and time.

Generally hydrogel polymers for use within the invention are cross-linked with a difunctional cross-linking in the amount of from 0.01 to 25 weight percent, based on the weight of the monomers forming the copolymer, and more preferably from 0.1 to 20 weight percent and more often from 0.1 to 15 weight percent of the cross-linking agent. Another useful amount of a cross-linking agent is 0.1 to 10 weight percent. Tri, tetra or higher multifunctional cross-linking agents may also be employed. When such reagents are utilized, lower amounts may be required to attain equivalent crosslinking density, i.e., the degree of cross-linking, or network properties that are sufficient to contain effectively the biologically active agent(s).

The cross-links can be covalent, ionic or hydrogen bonds with the polymer possessing the ability to swell in the presence of water containing fluids. Such crosslinkers and cross-linking reactions are known to those skilled in the art and in many cases are dependent upon the polymer system. Thus a crosslinked network may be formed by free radical copolymerization of unsaturated monomers. Polymeric hydrogels may also be formed by cross-linking preformed polymers by reacting functional groups found on the polymers such as alcohols, acids, amines with such groups as glyoxal, formaldehyde or glutaraldehyde, bis anhydrides and the like.

The polymers also may be cross-linked with any polyene, e.g. decadiene or trivinyl cyclohexane; acrylamides, such as N,N-methylene-bis (acrylamide); polyfunctional acrylates, such as trimethylol propane triacrylate; or polyfunctional vinylidene monomer containing at least 2 terminal $CH_2 <$ groups, including, for example, divinyl benzene, divinyl naphthlene, allyl acrylates and the like. In certain embodiments, cross-linking monomers for use in preparing the copolymers are polyalkenyl polyethers having more than one alkenyl ether grouping per molecule, which may optionally possess alkenyl groups in which an olefinic double bond is present attached to a terminal methylene grouping (e.g., made by the etherification of a polyhydric alcohol containing at least 2 carbon atoms and at least 2 hydroxyl groups). Compounds of this class may be produced by reacting an alkenyl halide, such as allyl chloride or allyl bromide, with a strongly alkaline aqueous solution of one or more polyhydric alcohols. The product may be a complex mixture of polyethers with varying numbers of ether groups. Efficiency of the polyether cross-linking agent increases with the number of potentially polymerizable groups on the molecule. Typically, polyethers containing an average of two or more alkenyl ether groupings per molecule are used. Other cross-linking monomers include for example, diallyl esters, dimethallyl ethers, allyl or methallyl acrylates and acrylamides, tetravinyl silane, polyalkenyl methanes, diacrylates, and dimethacrylates, divinyl compounds such as divinyl benzene, polyallyl phosphate, diallyloxy compounds and phosphine esters and the like. Typical agents are allyl pentaerythritol, allyl sucrose, trimethylolpropane triacrylate, 1,6-hexanediol diacrylate, trimethylolpropane diallyl ether, pentaerythritol triacrylate, tetramethylene dimethacrylate, ethylene diacrylate, ethylene dimethaerylate, triethylene glycol dimethacrylate, and the like. Allyl pentaerythritol, trimethylolpropane diallylether and allyl sucrose provide suitable polymers. When the cross-linking agent is present, the polymeric mixtures usually contain between about 0.01 to 20 weight percent, e.g., 1%, 5%, or 10% or more by weight of cross-linking monomer based on the total of carboxylic acid monomer, plus other monomers.

In more detailed aspects of the invention, mucosal delivery of glucose-regulating peptide, analogs and mimetics, and other biologically active agents disclosed herein, is enhanced by retaining the active agent(s) in a slow-release or enzymatically or physiologically protective carrier or vehicle, for example a hydrogel that shields the active agent from the action of the degradative enzymes. In certain embodiments, the active agent is bound by chemical means to the carrier or vehicle, to which may also be admixed or bound additional agents such as enzyme inhibitors, cytokines, etc. The active agent may alternately be immobilized through sufficient physical entrapment within the carrier or vehicle, e.g., a polymer matrix.

Polymers such as hydrogels useful within the invention may incorporate functional linked agents such as glycosides chemically incorporated into the polymer for enhancing intranasal bioavailability of active agents formulated therewith. Examples of such glycosides are glucosides, fructosides, galactosides, arabiposides, mannosides and their alkyl substituted derivatives and natural glycosides such as arbutin, phlorizin, amygdalin, digitonin, saponin, and indican. There are several ways in which a typical glycoside may be bound to a polymer. For example, the hydrogen of the hydroxyl groups of a glycoside or other similar carbohydrate may be replaced by the alkyl group from a hydrogel polymer to form an ether. Also, the hydroxyl groups of the glycosides may be reacted to esterify the carboxyl groups of a polymeric hydrogel to form polymeric esters *in situ.* Another approach is to employ condensation of acetobromoglucose with cholest-5-en-3beta-ol on a copolymer of maleic acid. N-substituted polyacrylamides can be synthesized by the reaction of activated Polymers with omega-aminoalkylglycosides: (1) (carbohydrate-spacer)(n)-polyacrylamide, pseudopolysaccharides (2) (carbohydrate spacer)(n)-phosphatidylethanolamine(m)-polyacrylamide, neoglycolipids, derivatives of phosphatidylethanolamine; and (3) (carbohydrate-spacer)(n)-biotin(m)-polyacrylamide. These biotinylated derivatives may attach to lectins on the mucosal surface to facilitate absorption of the biologically active agent(s), e.g., a polymer-encapsulated glucose-regulating peptide.

Within more detailed aspects of the invention, one or more glucose-regulating peptide, analogs and mimetics, and/or other biologically active agents, disclosed herein, optionally including secondary active agents such as protease inhibitor(s), cytokine(s), additional modulator(s) of intercellular junctional physiology, etc., are modified and bound to a polymeric carrier or matrix. For example, this may be accomplished by chemically binding a peptide or protein active agent and other optional agent(s) within a crosslinked polymer network. It is also possible to chemically modify the polymer separately with an interactive agent such as a glycosidal containing molecule. In certain aspects, the biologically active agent(s), and optional secondary active agent(s), may be functionalized, i.e., wherein an appropriate reactive group is identified or is chemically added to the active agent(s). Most often an ethylenic polymerizable group is added, and the functionalized active agent is then copolymerized with monomers and a crosslinking agent using a standard polymerization method such as solution polymerization (usually in water), emulsion, suspension or dispersion polymerization. Often, the functionalizing agent is provided with a high enough concentration of functional or polymerizable groups to insure that several sites on the active agent(s) are functionalized. For example, in a polypeptide comprising 16 amine sites, it is generally desired to functionalize at least 2, 4, 5, 7, and up to 8 or more of the sites.

After functionalization, the functionalized active agent(s) is/are mixed with monomers and a crosslinking agent that comprise the reagents from which the polymer of interest is formed. Polymerization is then induced in this medium to create a polymer containing the bound active agent(s). The polymer is then ished with water or other appropriate solvents and otherwise purified to remove trace unreacted impurities and, if necessary, ground or broken up by physical means such as by stirring, forcing it through a mesh, ultrasonication or other suitable means to a desired particle size. The solvent, usually water, is then removed in such a manner as to not denature or otherwise degrade the active agent(s). One desired method is lyophilization (freeze drying) but other methods are available and may be used (e.g., vacuum drying, air drying, spray, drying, etc.).

To introduce polymerizable groups in peptides, proteins and other active agents within the invention, it is possible to react available amino, hydroxyl, thiol and other reactive groups with electrophiles containing unsaturated groups. For example, unsaturated monomers containing N-hydroxy succinimidyl groups, active carbonates such as p-nitrophenyl carbonate, trichlorophenyl carbonates, tresylate, oxycarbonylimidazoles, epoxide, isocyanates and aldehyde, and unsaturated carboxymethyl azides and unsaturated orthopyridyl-disulfide belong to this category of reagents. Illustrative Examples of unsaturated reagents are allyl glycidyl ether, allyl chloride, allylbromide, allyl iodide, acryloyl chloride, allyl isocyanate, allylsulfonyl chloride, maleic anhydride, copolymers of maleic anhydride and allyl ether, and the like.

All of the lysine active derivatives, except aldehyde, can generally react with other amino acids such as imidazole groups of histidine and hydroxyl groups of tyrosine and the thiol groups of cystine if the local environment enhances nucleophilicity of these groups. Aldehyde-containing functionalizing reagents are specific to lysine. These types of reactions with available groups from lysines, cysteines, tyrosine have been extensively documented in the literature and are known to those skilled in the art.

In the case of biologically active agents that contain amine groups, it is convenient to react such groups with an acyloyl chloride, such as acryloyl chloride, and introduce the polymerizable acrylic group onto the reacted agent. Then during preparation of the polymer, such as during the crosslinking of the copolymer of acrylamide and acrylic acid, the functionalized active agent, through the acrylic groups, is attached to the polymer and becomes bound thereto.

In additional aspects of the invention, biologically active agents, including peptides, proteins, nucleosides, and other molecules which are bioactive *in vivo*, are conjugation-stabilized by covalently bonding one or more active agent(s) to a polymer incorporating as an integral part thereof both a hydrophilic moiety, e.g., a linear polyalkylene glycol, a lipophilic moiety (see, e.g., U.S. Patent No. 5,681,811). In one aspect, a biologically active agent is covalently coupled with a polymer comprising (i) a linear polyalkylene glycol moiety, and (ii) a lipophilic moiety, wherein the active agent, linear polyalkylene glycol moiety, and the lipophilic moiety are conformationally arranged in relation to one another such that the active therapeutic agent has an enhanced *in vivo* resistance to enzymatic degradation (i.e., relative to its stability under similar conditions in an unconjugated form devoid of the polymer coupled thereto).: In another aspect, the conjugation-stabilized formulation has a three-dimensional conformation comprising the biologically active agent covalently coupled with a polysorbate complex comprising (i) a linear polyalkylene glycol moiety, and (ii) a lipophilic moiety, wherein the active agent, the linear polyalkylene glycol moiety and the lipophilic moiety are conformationally arranged in relation to one another such that (a) the lipophilic moiety is exteriorly available in the three-dimensional conformation, and (b) the active agent in the composition has an enhanced *in vivo* resistance to enzymatic degradation.

In a further related aspect, a multiligand conjugated complex is provided which comprises a biologically active agent covalently coupled with a triglyceride backbone moiety through a polyalkylene glycol spacer group bonded at a carbon atom of the triglyceride backbone moiety, and at least one fatty acid moiety covalently attached either directly to a carbon atom of the triglyceride backbone moiety or covalently joined through a polyalkylene glycol spacer moiety (see, e.g., U.S. Patent No. 5,681,811). In such a multiligand conjugated therapeutic agent complex, the alpha' and beta carbon atoms of the triglyceride bioactive moiety may have fatty acid moieties attached by covalently bonding either directly thereto, or indirectly covalently bonded thereto through polyalkylene glycol spacer moieties. Alternatively, a fatty acid moiety may be covalently attached either directly or through a polyalkylene glycol spacer moiety to the alpha and alpha' carbons of the triglyceride backbone moiety, with the bioactive therapeutic agent being covalently coupled with the gamma-carbon of the triglyceride backbone moiety, either being directly covalently bonded thereto or indirectly bonded thereto through a polyalkylene spacer moiety. It will be recognized that a wide variety of structural, compositional, and conformational forms are possible for the multiligand conjugated therapeutic agent complex comprising the triglyceride backbone moiety, within the scope of the invention. It is further noted that in such a multiligand conjugated therapeutic agent complex, the biologically active agent(s) may advantageously be covalently coupled with the triglyceride modified backbone moiety through alkyl spacer groups, or alternatively other acceptable spacer groups, within the scope of the invention. As used in such context, acceptability of the spacer group refers to steric, compositional, and end use application specific acceptability characteristics.

In yet additional aspects of the invention, a conjugation-stabilized complex is provided which comprises a polysorbate complex comprising a polysorbate moiety including a triglyceride backbone having covalently coupled to alpha, alpha' and beta carbon atoms thereof functionalizing groups including (i) a fatty acid group; and (ii) a polyethylene glycol group having a biologically active agent or moiety covalently bonded thereto, e.g., bonded to an appropriate functionality of the polyethylene glycol group. Such covalent bonding may be either direct, e.g., to a hydroxy terminal functionality of the polyethylene glycol group, or alternatively, the covalent bonding may be indirect, e.g., by reactively capping the hydroxy terminus of the polyethylene glycol group with a terminal carboxy functionality spacer group, so that the resulting capped polyethylene glycol group has a terminal carboxy functionality to which the biologically active agent or moiety may be covalently bonded.

In yet additional aspects of the invention, a stable, aqueously soluble, conjugation-stabilized complex is provided which comprises one or more glucose-regulating peptide proteins, analogs and mimetics, and/or other biologically active agent(s)+ disclosed herein covalently coupled to a physiologically compatible polyethylene glycol (PEG) modified glycolipid moiety. In such complex, the biologically active agent(s) may be covalently coupled to the physiologically compatible PEG modified glycolipid moiety by a labile covalent bond at a free amino acid group of the active agent, wherein the labile covalent bond is scissionable *in vivo* by biochemical hydrolysis and/or proteolysis. The physiologically compatible PEG modified glycolipid moiety may advantageously comprise a polysorbate polymer, e.g., a polysorbate polymer comprising fatty acid ester groups selected from the group consisting of monopalmitate, dipalmitate, monolaurate, dilaurate, trilaurate, monoleate, dioleate, trioleate, monostearate, distearate, and tristearate. In such complex, the physiologically compatible PEG modified glycolipid moiety may suitably comprise a polymer selected from the group consisting of polyethylene glycol ethers of fatty acids, and polyethylene glycol esters of fatty acids, wherein the fatty acids for example comprise a fatty acid selected from the group consisting of lauric, palmitic, oleic, and stearic acids.

Storage and Manufacturing of Material

In certain aspects of the invention, the combinatorial formulations and/or coordinate administration methods herein incorporate an effective amount of peptides and proteins which may adhere to charged glass thereby reducing the effective concentration in the container. Silanized containers, for example, silanized glass containers, are used to store the finished product to reduce adsorption of the polypeptide or protein to a glass container.

In yet additional aspects of the invention, a kit for treatment of a mammalian subject comprises a stable pharmaceutical composition of one or more glucose-regulating peptide compound(s) formulated for mucosal delivery to the mammalian subject wherein the composition is effective to alleviate one or more symptom(s) of obesity, cancer, or malnutrition or isting related to cancer in said subject without unacceptable adverse side effects. The kit further comprises a pharmaceutical reagent vial to contain the one or more glucose-regulating peptide compounds. The pharmaceutical reagent vial is composed of pharmaceutical grade polymer, glass or other suitable material. The pharmaceutical reagent vial is, for example, a silanized glass vial. The kit further comprises an aperture for delivery of the composition to a nasal mucosal surface of the subject. The delivery aperture is composed of a pharmaceutical grade polymer, glass or other suitable material. The delivery aperture is, for example, a silanized glass.

A silanization technique combines a special cleaning technique for the surfaces to be silanized with a silanization process at low pressure. The silane is in the gas phase and at an enhanced temperature of the surfaces to be silanized. The method provides reproducible surfaces with stable, homogeneous and functional silane layers having characteristics of a monolayer. The silanized surfaces prevent binding to the glass of polypeptides or mucosal delivery enhancing agents of the present invention.

The procedure is useful to prepare silanized pharmaceutical reagent vials to hold glucose-regulating peptide compositions of the present invention. Glass trays are cleaned by rinsing with double distilled water (ddH$_2$O) before using. The silane tray is then be rinsed with 95% EtOH, and the acetone tray is rinsed with acetone. Pharmaceutical reagent vials are sonicated in acetone for 10 minutes. After the acetone sonication, reagent vials are washed in ddH$_2$O tray at least twice. Reagent vials are sonicated in 0.1M NaOH for 10 minutes. While the reagent vials are sonicating in NaOH, the silane solution is made under a hood. (Silane solution: 800 mL of 95% ethanol; 96 L of glacial acetic acid; 25 mL of glycidoxypropyltrimethoxy silane). After the NaOH sonication, reagent vials are washed in ddH$_2$O tray at least twice. The reagent vials are sonicated in silane solution for 3 to 5 minutes. The reagent vials are ished in 100% EtOH tray. The reagent vials are dried with prepurified N$_2$ gas and stored in a 100°C oven for at least 2 hours before using.

The nasal spray product manufacturing process may include the preparation of a diluent for the nasal spray, which includes ~85% water plus the components of the nasal spray formulation without the gluclose-regulating peptide. The pH of the diluent is then measured and adjusted to pH 4.0±0.3 with sodium hydroxide or hydrochloric acid, if necessary. The nasal spray is prepared by the non-aseptic transfer of ~85% of the final target volume of the diluent to a screw cap bottle. An appropriate amount of gluclose-regulating peptide is added and mixed until completely dissolved. The pH is measured and adjusted to pH 7.0±0.3 with sodium hydroxide or hydrochloric acid, if necessary. A sufficient quantity of diluent is added to reach the final target volume. Screw-cap bottles are filled and caps affixed. The above description of the manufacturing process represents a method used to prepare the initial clinical batches of drug product. This method may be modified during the development process to optimize the manufacturing process.

Currently marketed injectable gluclose-regulating peptide requires sterile manufacturing conditions for compliance with FDA regulations. Parenteral administration, including insulin for injection or infusion, requires a sterile (aseptic) manufacturing process. Current Good Manufacturing Practices (GMP) for sterile drug manufacturing include standards for design and construction features (21 CFR § 211.42 (April 1 , 2005)); standards for testing and approval or rejection of components, drug product containers, and closures (§ 211.84); standards for control of microbiological contamination (§ 211.113); and other special testing requirements (§ 211.167). Non-parenteral (non-aseptic) products, such as the intranasal product of the invention, do not require these specialized sterile manufacturing conditions. As can be readily appreciated, the requirements for a sterile manufacturing process are substantially higher and correspondingly more costly than those required for a non-sterile product manufacturing process. These costs include much greater capitalization costs for facilities, as well as a more costly manufacturing cost: extra facilites for sterile manufacturing include additional rooms and ventilation; extra costs associated with sterile manufacturing include greater manpower, extensive quality control and quality assurance, and administrative support. As a result, manufacturing costs of an intranasal glucose-regulating peptide product, such as that of the invention, are far less than those of a parenterally administered glucose-regulating peptide product. The present invention satisfies the need for a non-sterile manufacturing process for a glucose-regulating peptide.

Sterile solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders, methods of preparation include vacuum drying and freeze-drying which yields a powder of the active ingredient plus any additional desired ingredient from a previously sterile-filtered solution thereof. The prevention of the action of microorganisms can be accomplished by various antibacterial and antifungal agents, for example, parabens, chlorobutanol, phenol, sorbic acid, thimerosal, and the like.

Mucosal administration according to the invention allows effective self-administration of treatment by patients, provided that sufficient safeguards are in place to control and monitor dosing and side effects. Mucosal administration also overcomes certain drawbacks of other administration forms, such as injections, that are painful and expose the patient to possible infections and may present drug bioavailability problems.

Bioadhesive Delivery Vehicles and Methods

In certain aspects of the invention, the combinatorial formulations and/or coordinate administration methods herein incorporate an effective amount of a nontoxic bioadhesive as an adjunct compound or carrier to enhance mucosal delivery of one or more biologically active agent(s). Bioadhesive agents in this context exhibit general or specific adhesion to one or more components or surfaces of the targeted mucosa. The bioadhesive maintains a desired concentration gradient of the biologically active agent into or across the mucosa to ensure penetration of even large molecules (e.g., peptides and proteins) into or through the mucosal epithelium. Typically, employment of a bioadhesive within the methods and compositions of the invention yields a two- to five- fold, often a five- to ten-fold increase in permeability for peptides and proteins into or through the mucosal epithelium. This enhancement of epithelial permeation often permits effective transmucosal delivery of large macromolecules, for example to the basal portion of the nasal epithelium or into the adjacent extracellular compartments or a blood plasma or CNS tissue or fluid.

This enhanced delivery provides for greatly improved effectiveness of delivery of bioactive peptides, proteins and other macromolecular therapeutic species. These results will depend in part on the hydrophilicity of the compound, whereby greater penetration will be achieved with hydrophilic species compared to water insoluble compounds. In addition to these effects, employment of bioadhesives to enhance drug persistence at the mucosal surface can elicit a reservoir mechanism for protracted drug delivery, whereby compounds not only penetrate across the mucosal tissue but also back-diffuse toward the mucosal surface once the material at the surface is depleted.

A variety of suitable bioadhesives are disclosed in the art for oral administration, U.S. Patent Nos. 3,972,995; 4,259,314; 4,680,323; 4,740,365; 4,573,996; 4,292,299; 4,715,369; 4,876,092; 4,855,142; 4,250,163; 4,226,848; 4,948,580; and U.S. Patent Reissue No. 33,093, which find use within the novel methods and compositions of the invention. The potential of various bioadhesive polymers as a mucosal, e.g., nasal, delivery platform within the methods and compositions of the invention can be readily assessed by determining their ability to retain and release glucose-regulating peptide, as well as by their capacity to interact with the mucosal surfaces following incorporation of the active agent therein. In addition, well known methods will be applied to determine the biocompatibility of selected polymers with the tissue at the site of mucosal administration. When the target mucosa is covered by mucus (i.e., in the absence of mucolytic or mucus-clearing treatment), it can serve as a connecting link to the underlying mucosal epithelium. Therefore, the term "bioadhesive" as used herein also covers mucoadhesive compounds useful for enhancing mucosal delivery of biologically active agents within the invention. However, adhesive contact to mucosal tissue mediated through adhesion to a mucus gel layer may be limited by incomplete or transient attachment between the mucus layer and the underlying tissue, particularly at nasal surfaces where rapid mucus clearance occurs. In this regard, mucin glycoproteins are continuously secreted and, immediately after their release from cells or glands, form a viscoelastic gel. The luminal surface of the adherent gel layer, however, is continuously eroded by mechanical, enzymatic and/or ciliary action. Where such activities are more prominent or where longer adhesion times are desired, the coordinate administration methods and combinatorial formulation methods of the invention may further incorporate mucolytic and/or ciliostatic methods or agents as disclosed herein above.

Typically, mucoadhesive polymers for use within the invention are natural or synthetic macromolecules which adhere to wet mucosal tissue surfaces by complex, but non-specific, mechanisms. In addition to these mucoadhesive polymers, the invention also provides methods and compositions incorporating bioadhesives that adhere directly to a cell surface, rather than to mucus, by means of specific, including receptor-mediated, interactions. One example of bioadhesives that function in this specific manner is the group of compounds known as lectins. These are glycoproteins with an ability to specifically recognize and bind to sugar molecules, e.g., glycoproteins or glycolipids, which form part of intranasal epithelial cell membranes and can be considered as "lectin receptors."

In certain aspects of the invention, bioadhesive materials for enhancing intranasal delivery of biologically active agents comprise a matrix of a hydrophilic, e.g., water soluble or swellable, polymer or a mixture of polymers that can adhere to a wet mucous surface. These adhesives may be formulated as ointments, hydrogels (see above) thin films, and other application forms. Often, these adhesives have the biologically active agent mixed therewith to effectuate slow release or local delivery of the active agent. Some are formulated with additional ingredients to facilitate penetration of the active agent through the nasal mucosa, e.g., into the circulatory system of the individual.

Various polymers, both natural and synthetic ones, show significant binding to mucus and/or mucosal epithelial surfaces under physiological conditions. The strength of this interaction can readily be measured by mechanical peel or shear tests. When applied to a humid mucosal surface, many dry materials will spontaneously adhere, at least slightly. After such an initial contact, some hydrophilic materials start to attract water by adsorption, swelling or capillary forces, and if this water is absorbed from the underlying substrate or from the polymer-tissue interface, the adhesion may be sufficient to achieve the goal of enhancing mucosal absorption of biologically active agents. Such 'adhesion by hydration' can be quite strong, but formulations adapted to employ this mechanism must account for swelling which continues as the dosage transforms into a hydrated mucilage. This is projected for many hydrocolloids useful within the invention, especially some cellulose-derivatives, which are generally non-adhesive when applied in pre-hydrated state. Nevertheless, bioadhesive drug delivery systems for mucosal administration are effective within the invention when such materials are applied in the form of a dry polymeric powder, microsphere, or film-type delivery form.

Other polymers adhere to mucosal surfaces not only when applied in dry, but also in fully hydrated state, and in the presence of excess amounts of water. The selection of a mucoadhesive thus requires due consideration of the conditions, physiological as well as physico-chemical, under which the contact to the tissue will be formed and maintained. In particular, the amount of water or humidity usually present at the intended site of adhesion, and the prevailing pH, are known to largely affect the mucoadhesive binding strength of different polymers.

Several polymeric bioadhesive drug delivery systems have been fabricated and studied in the past 20 years, not always with success. A variety of such carriers are, however, currently used in clinical applications involving dental, orthopedic, ophthalmological, and surgical uses. For example, acrylic-based hydrogels have been used extensively for bioadhesive devices. Acrylic-based hydrogels are well suited for bioadhesion due to their flexibility and nonabrasive characteristics in the partially swollen state, which reduce damage-causing attrition to the tissues in contact. Furthermore, their high permeability in the swollen state allows unreacted monomer, un-crosslinked polymer chains, and the initiator to be washed out of the matrix after polymerization, which is an important feature for selection of bioadhesive materials for use within the invention. Acrylic-based polymer devices exhibit very high adhesive bond strength. For controlled mucosal delivery of peptide and protein drugs, the methods and compositions of the invention optionally include the use of carriers, e.g., polymeric delivery vehicles that function in part to shield the biologically active agent from proteolytic breakdown, while at the same time providing for enhanced penetration of the peptide or protein into or through the nasal mucosa. In this context, bioadhesive polymers have demonstrated considerable potential for enhancing oral drug delivery. As an example, the bioavailability of 9-desglycinamide, 8-arginine vasopressin (DGAVP) intraduodenally administered to rats together with a 1% (w/v) saline dispersion of the mucoadhesive poly(acrylic acid) derivative polycarbophil, is 3-5-fold increased compared to an aqueous solution of the peptide drug without this polymer.

Mucoadhesive polymers of the poly(acrylic acid)-type are potent inhibitors of some intestinal proteases. The mechanism of enzyme inhibition is explained by the strong affinity of this class of polymers for divalent cations, such as calcium or zinc, which are essential cofactors of metallo-proteinases, such as trypsin and chymotrypsin. Depriving the

24

proteases of their cofactors by poly(acrylic acid) is reported to induce irreversible structural changes of the enzyme proteins which were accompanied by a loss of enzyme activity. At the same time, other mucoadhesive polymers (e.g., some cellulose derivatives and chitosan) may not inhibit proteolytic enzymes under certain conditions. In contrast to other enzyme inhibitors contemplated for use within the invention (e.g., aprotinin, bestatin), which are relatively small molecules, the trans-nasal absorption of inhibitory polymers is likely to be minimal in light of the size of these molecules, and thereby eliminate possible adverse side effects. Thus, mucoadhesive polymers, particularly of the poly(acrylic acid)-type, may serve both as an absorption-promoting adhesive and enzyme-protective agent to enhance controlled delivery of peptide and protein drugs, especially when safety concerns are considered.

In addition to protecting against enzymatic degradation, bioadhesives and other polymeric or non-polymeric absorption-promoting agents for use within the invention may directly increase mucosal permeability to biologically active agents. To facilitate the transport of large and hydrophilic molecules, such as peptides and proteins, across the nasal epithelial barrier, mucoadhesive polymers and other agents have been postulate to yield enhanced permeation effects beyond what is accounted for by prolonged premucosal residence time of the delivery system. The time course of drug plasma concentrations reportedly suggested that the bioadhesive microspheres caused an acute, but transient increase of insulin permeability across the nasal mucosa. Other mucoadhesive polymers for use within the invention, for example chitosan, reportedly enhance the permeability of certain mucosal epithelia even when they are applied as an aqueous solution or gel. Another mucoadhesive polymer reported to directly affect epithelial permeability is hyaluronic acid and ester derivatives thereof. A particularly useful bioadhesive agent within the coordinate administration, and/or combinatorial formulation methods and compositions of the invention is chitosan, as well as its analogs and derivatives. Chitosan is a non-toxic, biocompatible and biodegradable polymer that is widely used for pharmaceutical and medical applications because of its favorable properties of low toxicity and good biocompatibility. It is a natural polyaminosaccharide prepared from chitin by N-deacetylation with alkali. As used within the methods and compositions of the invention, chitosan increases the retention of glucose-regulating peptide proteins, analogs and mimetics, and other biologically active agents disclosed herein at a mucosal site of application. This mode of administration can also improve patient compliance and acceptance. As further provided herein, the methods and compositions of the invention will optionally include a novel chitosan derivative or chemically modified form of chitosan. One such novel derivative for use within the invention is denoted as a $\beta$-[1$\rightarrow$4]-2-guanidino-2-deoxy-D-glucose polymer (poly-GuD). Chitosan is the N-deacetylated product of chitin, a naturally occurring polymer that has been used extensively to prepare microspheres for oral and intra-nasal formulations. The chitosan polymer has also been proposed as a soluble carrier for parenteral drug delivery. Within one aspect of the invention, o-methylisourea is used to convert a chitosan amine to its guanidinium moiety. The guanidinium compound is prepared, for example, by the reaction between equinormal solutions of chitosan and o-methylisourea at pH above 8.0.

Additional compounds classified as bioadhesive agents for use within the present invention act by mediating specific interactions, typically classified as "receptor-ligand interactions" between complementary structures of the bioadhesive compound and a component of the mucosal epithelial surface. Many natural examples illustrate this form of specific binding bioadhesion, as exemplified by lectin-sugar interactions. Lectins are (glyco) proteins of non-immune origin which bind to polysaccharides or glycoconjugates.

Several plant lectins have been investigated as possible pharmaceutical absorption-promoting agents. One plant lectin, Phaseolus vulgaris hemagglutinin (PHA), exhibits high oral bioavailability of more than 10% after feeding to rats. Tomato *(Lycopersicon esculeuctum)* lectin (TL) appears safe for various modes of adminstration.

In summary, the foregoing bioadhesive agents are useful in the combinatorial formulations and coordinate administration methods of the instant invention, which optionally incorporate an effective amount and form of a bioadhesive agent to prolong persistence or otherwise increase mucosal absorption of one or more glucose-regulating peptide proteins, analogs and mimetics, and other biologically active agents. The bioadhesive agents may be coordinately administered as adjunct compounds or as additives within the combinatorial formulations of the invention. In certain embodiments, the bioadhesive agent acts as a pharmaceutical glue', whereas in other embodiments adjunct delivery or combinatorial formulation of the bioadhesive agent serves to intensify contact of the biologically active agent with the nasal mucosa, in some cases by promoting specific receptor-ligand interactions with epithelial cell "receptors", and in others by increasing epithelial permeability to significantly increase the drug concentration gradient measured at a target site of delivery (e.g., liver, blood plasma, or CNS tissue or fluid). Yet additional bioadhesive agents for use within the invention act as enzyme (e.g., protease) inhibitors to enhance the stability of mucosally administered biotherapeutic agents delivered coordinately or in a combinatorial formulation with the bioadhesive agent.

Liposomes and Micellar Delivery Vehicles

The coordinate administration methods and combinatorial formulations of the instant invention optionally incorporate effective lipid or fatty acid based carriers, processing agents, or delivery vehicles, to provide improved formulations for mucosal delivery of glucose-regulating peptide proteins, analogs and mimetics, and other biologically active agents. For example, a variety of formulations and methods are provided for mucosal delivery which comprise one or more of these active agents, such as a peptide or protein, admixed or encapsulated by, or coordinately administered with, a liposome, mixed micellar carrier, or emulsion, to enhance chemical and physical stability and increase the half life of the biologically active agents (e.g., by reducing susceptibility to proteolysis, chemical modification and/or denaturation) upon mucosal delivery.

Within certain aspects of the invention, specialized delivery systems for biologically active agents comprise small lipid vesicles known as liposomes. These are typically made from natural, biodegradable, non-toxic, and non-immunogenic lipid molecules, and can efficiently entrap or bind drug molecules, including peptides and proteins, into, or onto, their membranes. The attractiveness of liposomes as a peptide and protein delivery system within the invention is increased by the fact that the encapsulated proteins can remain in their preferred aqueous environment within the vesicles, while the liposomal membrane protects them against proteolysis and other destabilizing factors. Even though not all liposome preparation methods known are feasible in the encapsulation of peptides and proteins due to their unique physical and chemical properties, several methods allow the encapsulation of these macromolecules without substantial deactivation.

A variety of methods are available for preparing liposomes for use within the invention, U.S. Patent Nos. 4,235,871; 4,501,728; and 4,837,028. For use with liposome delivery, the biologically active agent is typically entrapped within the liposome, or lipid vesicle, or is bound to the outside of the vesicle.

Like liposomes, unsaturated long chain fatty acids, which also have enhancing activity for mucosal absorption, can form closed vesicles with bilayer-like structures (so called "ufasomes"). These can be formed, for example, using oleic acid to entrap biologically active peptides and proteins for mucosal, e.g., intranasal, delivery within the invention.

Other delivery systems for use within the invention combine the use of polymers and liposomes to ally the advantageous properties of both vehicles such as encapsulation inside the natural polymer fibrin. In addition, release of biotherapeutic compounds from this delivery system is controllable through the use of covalent crosslinking and the addition of antifibrinolytic agents to the fibrin polymer.

More simplified delivery systems for use within the invention include the use of cationic lipids as delivery vehicles or carriers, which can be effectively employed to provide an electrostatic interaction between the lipid carrier and such charged biologically active agents as proteins and polyanionc nucleic acids. This allows efficient packaging of the drugs into a form, suitable for mucosal administration and/or subsequent delivery to systemic compartments.

Additional delivery vehicles for use within the invention include long and medium chain fatty acids, as well as surfactant mixed micelles with fatty acids. Most naturally occurring lipids-in the form of esters have important implications with regard to their own transport across mucosal surfaces. Free fatty acids and their monoglycerides which have polar groups attached have been demonstrated in the form of mixed micelles to act on the intestinal barrier as penetration enhancers. This discovery of barrier modifying function of free fatty acids (carboxylic acids with a chain length varying from 12 to 20 carbon atoms) and their polar derivatives has stimulated extensive research on the application of these agents as mucosal absorption enhancers.

For use within the methods of the invention, long chain fatty acids, especially fusogenic lipids (unsaturated fatty acids and monoglycerides such as oleic acid, linoleic acid, linoleic acid, monoolein, etc.) provide useful carriers to enhance mucosal delivery of glucose-regulating peptide, analogs and mimetics, and other biologically active agents disclosed herein. Medium chain fatty acids (C6 to C12) and monoglycerides have also been shown to have enhancing activity in intestinal drug absorption and can be adapted for use within the mocosal delivery formulations and methods of the invention. In addition, sodium salts of medium and long chain fatty acids are effective delivery vehicles and absorption-enhancing agents for mucosal delivery of biologically active agents within the invention. Thus, fatty acids can be employed in soluble forms of sodium salts or by the addition of non-toxic surfactants, e.g., polyoxyethylated hydrogenated castor oil, sodium taurocholate, etc. Other fatty acid and mixed micellar preparations that are useful within the invention include, but are not limited to, Na caprylate (C8), Na caprate (C10), Na laurate (C12) or Na oleate (C18), optionally combined with bile salts, such as glycocholate and taurocholate.

Pegylation

Additional methods and compositions provided within the invention involve chemical modification of biologically active peptides and proteins by covalent attachment of polymeric materials, for example dextrans, polyvinyl pyrrolidones, glycopeptides, polyethylene glycol and polyamino acids. The resulting conjugated peptides and proteins retain their biological activities and solubility for mucosal administration. In alternate embodiments, glucose-regulating peptide proteins, analogs and mimetics, and other biologically active peptides and proteins, are conjugated to polyalkylene oxide polymers, particularly polyethylene glycols (PEG). U.S. Patent No. 4,179,337.

Amine-reactive PEG polymers for use within the invention include SC-PEG with molecular masses of 2000, 5000, 10000, 12000, and 20 000; U-PEG-10000; NHS-PEG-3400-biotin; T-PEG-5000; T-PEG-12000; and TPC-PEG-5000. PEGylation of biologically active peptides and proteins may be achieved by modification of carboxyl sites (e.g.,

aspartic acid or glutamic acid groups in addition to the carboxyl terminus). The utility of PEG-hydrazide in selective modification of carbodiimide-activated protein carboxyl groups under acidic conditions has been described. Alternatively, bifunctional PEG modification of biologically active peptides and proteins can be employed. In some procedures, charged amino acid residues, including lysine, aspartic acid, and glutamic acid, have a marked tendency to be solvent accessible on protein surfaces.

Other Stabilizing Modifications of Active Agents

In addition to PEGylation, biologically active agents such as peptides and proteins for use within the invention can be modified to enhance circulating half-life by shielding the active agent via conjugation to other known protecting or stabilizing compounds, for example by the creation of fusion proteins with an active peptide, protein, analog or mimetic linked to one or more carrier proteins, such as one or more immunoglobulin chains.

Formulation and Administration

Mucosal delivery formulations of the present invention comprise glucose-regulating peptide, analogs and mimetics, typically combined together with one or more pharmaceutically acceptable carriers and, optionally, other therapeutic ingredients. The carrier(s) must be "pharmaceutically acceptable" in the sense of being compatible with the other ingredients of the formulation and not eliciting an unacceptable deleterious effect in the subject. Such carriers are described herein above or are otherwise well known to those skilled in the art of pharmacology. Desirably, the formulation should not include substances such as enzymes or oxidizing agents with which the biologically active agent to be administered is known to be incompatible. The formulations may be prepared by any of the methods well known in the art of pharmacy.

Within the compositions and methods of the invention, the glucose-regulating peptide proteins, analogs and mimetics, and other biologically active agents disclosed herein may be administered to subjects by a variety of mucosal administration modes, including by oral, rectal, vaginal, intranasal, intrapulmonary, or transdermal delivery, or by topical delivery to the eyes, ears, skin or other mucosal surfaces. Optionally, glucose-regulating peptide proteins, analogs and mimetics, and other biologically active agents disclosed herein can be coordinately or adjunctively administered by non-mucosal routes, including by intramuscular, subcutaneous, intravenous, intra-atrial, intra-articular, intraperitoneal, or parenteral routes. In other alternative embodiments, the biologically active agent(s) can be administered *ex vivo* by direct exposure to cells, tissues or organs originating from a mammalian subject, for example as a component of an *ex vivo* tissue or organ treatment formulation that contains the biologically active agent in a suitable, liquid or solid carrier.

Compositions according to the present invention may be administered in an aqueous solution as a nasal or pulmonary spray and may be dispensed in spray form by a variety of methods known to those skilled in the art. Preferred systems for dispensing liquids as a nasal spray are disclosed in U.S. Patent No. 4,511,069. The formulations may be presented in multi-dose containers, for example in the sealed dispensing system disclosed in U.S. Patent No. 4,511,069. Additional aerosol delivery forms may include, e.g., compressed air-, jet-, ultrasonic-, and piezoelectric nebulizers, which deliver the biologically active agent dissolved or suspended in a pharmaceutical solvent, e.g., water, ethanol, or a mixture thereof. An aerosol formulation of this invention may have droplets having diameters from 1 to 700 microns in size.

Compositions and formulations of this invention may have an osmolarity of from 50 to 350 mOsm/L, or from 50 to 300 mOsm/L. A tonicifier may be used to adjust the osmolarity, osmolality, or tonicity of a formulation.

Nasal and pulmonary spray solutions of the present invention typically comprise the drug or drug to be delivered, optionally formulated with a surface-active agent, such as a nonionic surfactant (e.g., polysorbate-80), and one or more buffers. In some embodiments of the present invention, the nasal spray solution further comprises a propellant. The pH of the nasal spray solution is optionally between about pH 3.0 and 9, preferably 7.0 $\pm$0.5. Suitable buffers for use within these compositions are as described above or as otherwise known in the art. Other components may be added to enhance or maintain chemical stability, including preservatives, surfactants, dispersants, or gases. Suitable preservatives include, but are not limited to, phenol, methyl paraben, paraben, m-cresol, thiomersal, chlorobutanol, benzylalkonimum chloride, sodium benzoate, and the like. Suitable surfactants include, but are not limited to, oleic acid, sorbitan trioleate, polysorbates, lecithin, phosphotidyl cholines, and various long chain diglycerides and phospholipids. Suitable dispersants include, but are not limited to, ethylenediaminetetraacetic acid, and the like. Suitable gases include, but are not limited to, nitrogen, helium, chlorofluorocarbons (CFCs), hydrofluorocarbons (HFCs), carbon dioxide, air, and the like.

Within alternate embodiments, mucosal formulations are administered as dry powder formulations comprising the biologically active agent in a dry, usually lyophilized, form of an appropriate particle size, or within an appropriate particle size range, for intranasal, delivery. Minimum particle size appropriate for deposition within the nasal or pulmonary passages is often about 0.5 $\mu$ mass median equivalent aerodynamic diameter (MMEAD), commonly about 1 $\mu$ MMEAD, and more typically about 2 $\mu$ MMEAD. Maximum particle size appropriate for deposition within the nasal passages is often about 10 $\mu$MMEAD, commonly about 8 $\mu$ MMEAD, and more typically about 4 $\mu$ MMEAD. Intranasally respirable

powders within these size ranges can be produced by a variety of conventional techniques, such as jet milling, spray drying, solvent precipitation, supercritical fluid condensation, and the like. These dry powders of appropriate MMEAD can be administered to a patient via a conventional dry powder inhaler (DPI), which rely on the patient's breath, upon pulmonary or nasal inhalation, to disperse the power into an aerosolized amount. Alternatively, the dry powder may be administered via air-assisted devices that use an external power source to disperse the powder into an aerosolized amount, e.g., a piston pump.

Dry powder devices typically require a powder mass in the range from about 1 mg to 20 mg to produce a single aerosolized dose ("puff"). If the required or desired dose of the biologically active agent is lower than this amount, the powdered active agent will typically be combined with a pharmaceutical dry bulking powder to provide the required total powder mass. Preferred dry bulking powders include sucrose, lactose, dextrose, mannitol, glycine, trehalose, human serum albumin (HSA), and starch. Other suitable dry bulking powders include cellobiose, dextrans, maltotriose, pectin, sodium citrate, sodium ascorbate, and the like.

To formulated compositions for mucosal delivery within the present invention, the biologically active agent can be combined with various pharmaceutically acceptable additives, as well as a base or carrier for dispersion of the active agent(s). Desired additives include, but are not limited to, pH control agents, such as arginine, sodium hydroxide, glycine, hydrochloric acid, citric acid, acetic acid, etc. In addition, local anesthetics (e.g., benzyl alcohol), isotonizing agents (e.g., sodium chloride, mannitol, sorbitol), adsorption inhibitors (e.g., Tween 80), solubility enhancing agents (e.g., cyclodextrins and derivatives thereof), stabilizers (e.g., serum albumin), and reducing agents (e.g., glutathione) can be included. When the composition for mucosal delivery is a liquid, the tonicity of the formulation, as measured with reference to the tonicity of 0.9% (w/v) physiological saline solution taken as unity, is typically adjusted to a value at which no substantial, irreversible tissue damage will be induced in the nasal mucosa at the site of administration. Generally, the tonicity of the solution is adjusted to a value of about 1/3 to 3, more typically 1/2 to 2, and most often 3/4 to 1.7.

The biologically active agent may be dispersed in a base or vehicle, which may comprise a hydrophilic compound having a capacity to disperse the active agent and any desired additives. The base may be selected from a wide range of suitable carriers, including but not limited to, copolymers of polycarboxylic acids or salts thereof, carboxylic anhydrides (e.g., maleic anhydride) with other monomers (e.g., methyl (meth)acrylate, acrylic acid, etc.), hydrophilic vinyl polymers such as polyvinyl acetate, polyvinyl alcohol, polyvinylpyrrolidone, cellulose derivatives such as hydroxymethylcellulose, hydroxypropylcellulose, etc., and natural polymers such as chitosan, collagen, sodium alginate, gelatin, hyaluronic acid, and nontoxic metal salts thereof. Often, a biodegradable polymer is selected as a base or carrier, for example, polylactic acid, poly(lactic acid-glycolic acid) copolymer, polyhydroxybutyric acid, poly(hydroxybutyric acid-glycolic acid) copolymer and mixtures thereof. Alternatively or additionally, synthetic fatty acid esters such as polyglycerin fatty acid esters, sucrose fatty acid esters, etc., can be employed as carriers. Hydrophilic polymers and other carriers can be used alone or in combination, and enhanced structural integrity can be imparted to the carrier by partial crystallization, ionic bonding, crosslinking and the like. The carrier can be provided in a variety of forms, including, fluid or viscous solutions, gels, pastes, powders, microspheres and films for direct application to the nasal mucosa. The use of a selected carrier in this context may result in promotion of absorption of the biologically active agent.

The biologically active agent can be combined with the base or carrier according to a variety of methods, and release of the active agent may be by diffusion, disintegration of the carrier, or associated formulation of water channels. In some circumstances, the active agent is dispersed in microcapsules (microspheres) or nanocapsules (nanospheres) prepared from a suitable polymer, e.g., isobutyl 2-cyanoacrylate and dispersed in a biocompatible dispersing medium applied to the nasal mucosa, which yields sustained delivery and biological activity over a protracted time.

To further enhance mucosal delivery of pharmaceutical agents within the invention, formulations comprising the active agent may also contain a hydrophilic low molecular weight compound as a base or excipient. Such hydrophilic low molecular weight compounds provide a passage medium through which a water-soluble active agent, such as a physiologically active peptide or protein, may diffuse through the base to the body surface where the active agent is absorbed. The hydrophilic low molecular weight compound optionally absorbs moisture from the mucosa or the administration atmosphere and dissolves the water-soluble active peptide. The molecular weight of the hydrophilic low molecular weight compound is generally not more than 10000 and preferably not more than 3000. Exemplary hydrophilic low molecular weight compound include polyol compounds, such as oligo-, di- and monosaccharides such as sucrose, mannitol, sorbitol, lactose, L-arabinose, D-erythrose, D-ribose, D-xylose, D-mannose, trehalose, D-galactose, lactulose, cellobiose, gentibiose, glycerin and polyethylene glycol. Other examples of hydrophilic low molecular weight compounds useful as carriers within the invention include N-methylpyrrolidone, and alcohols (e.g. oligovinyl alcohol, ethanol, ethylene glycol, propylene glycol, etc.). These hydrophilic low molecular weight compounds can be used alone or in combination with one another or with other active or inactive components of the intranasal formulation.

The compositions of the invention may alternatively contain as pharmaceutically acceptable carriers substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc. For solid compositions, conventional nontoxic pharmaceutically acceptable carriers can be used which include, for example, pharmaceutical grades of mannitol, lactose, starch, magnesium stearate; sodium saccharin, talcum, cellulose, glucose, sucrose, magnesium carbonate, and the like.

Therapeutic compositions for administering the biologically active agent can also be formulated as a solution, microemulsion, or other ordered structure suitable for high concentration of active ingredients. The carrier can be a solvent or dispersion medium containing, for example, water, ethanol, polyol (for example, glycerol, propylene glycol, and liquid polyethylene glycol, and the like), and suitable mixtures thereof. Proper fluidity for solutions can be maintained, for example, by the use of a coating such as lecithin, by the maintenance of a desired particle size in the case of dispersible formulations, and by the use of surfactants. In many cases, it will be desirable to include isotonic agents, for example, sugars, polyalcohols such as mannitol, sorbitol, or sodium chloride in the composition. Prolonged absorption of the biologically active agent can be brought about by including in the composition ' an agent which delays absorption, for example, monostearate salts and gelatin.

In certain embodiments of the invention, the biologically active agent is administered in a time-release formulation, for example in a composition which includes a slow release polymer. The active agent can be prepared with carriers that will protect against rapid release, for example a controlled release vehicle such as a polymer, microencapsulated delivery system or bioadhesive gel. Prolonged delivery of the active agent, in various compositions of the invention can be brought about by including in the composition agents that delay absorption, for example, aluminum monosterate hydrogels and gelatin. When controlled release formulations of the biologically active agent is desired, controlled release binders suitable for use in accordance with the invention include any biocompatible controlled-release material which is inert to the active agent and which is capable of incorporating the biologically active agent. Numerous such materials are known in the art. Useful controlled-release binders are materials that are metabolized slowly under physiological conditions following their intranasal delivery (e.g., at the nasal mucosal surface, or in the presence of bodily fluids following transmucosal delivery). Appropriate binders include but are not limited to biocompatible polymers and copolymers previously used in the art in sustained release formulations. Such biocompatible compounds are non-toxic and inert to surrounding tissues, and do not trigger significant adverse side effects such as nasal irritation, immune response, inflammation, or the like. They are metabolized into metabolic products that are also biocompatible and easily eliminated from the body.

Exemplary polymeric materials for use in this context include, but are not limited to, polymeric matrices derived from copolymeric and homopolymeric polyesters having hydrolysable ester linkages. A number of these are known in the art to be biodegradable and to lead to degradation products having no or low toxicity. Exemplary polymers include polyglycolic acids (PGA) and polylactic acids (PLA), poly(DL-lactic acid-co-glycolic acid)(DL PLGA), poly(D-lactic acid-coglycolic acid)(D PLGA) and poly(L-lactic acid-co-glycolic acid)(L PLGA). Other useful biodegradable or bioerodable polymers include but are not limited to such polymers as poly(epsilon-caprolactone), poly(epsilon-aprolactone-CO-lactic acid), poly(ε-aprolactone-CO-glycolic acid), poly(beta-hydroxy butyric acid), poly(alkyl-2-cyanoacrilate), hydrogels such as poly(hydroxyethyl methacrylate), polyamides, poly(amino acids) (i.e., L-leucine, glutamic acid, L-aspartic acid and the like), poly (ester urea), poly (2-hydroxyethyl DL-aspartamide), polyacetal polymers, polyorthoesters, polycarbonate, polymaleamides, polysaccharides and copolymers thereof. Many methods for preparing such formulations are generally known to those skilled in the art. Other useful formulations include-controlled-release compositions e.g., microcapsules, U.S. Patent Nos. 4,652,441 and 4,917,893, lactic acid-glycolic acid copolymers useful in making microcapsules and other formulations, U.S. Patent Nos. 4,677,191 and 4,728,721, and sustained-release compositions for water-soluble peptides, U.S. Patent No. 4,675,189.

For nasal and pulmonary delivery, systems for controlled aerosol dispensing of therapeutic liquids as a spray are well known. In one embodiment, metered doses of active agent are delivered by means of a specially constructed mechanical pump valve, U.S. Patent No. 4,511,069.

Dosage

For prophylactic and treatment purposes, the biologically active agent(s) disclosed herein may be administered to the subject in a single bolus delivery, via continuous delivery (e.g., continuous transdennal, mucosal, or intravenous delivery) over an extended time period, or in a repeated administration protocol (e.g., by an hourly, daily or weekly, repeated administration protocol). In this context, a therapeutically effective dosage of the glucose-regulating peptide may include repeated doses within a prolonged prophylaxis or treatment regimen that will yield clinically significant results to alleviate one or more symptoms or detectable conditions associated with a targeted disease or condition as set forth above. Determination of effective dosages in this context is typically based on animal model studies followed up by human clinical trials and is guided by determining effective dosages and administration protocols that significantly reduce the occurrence or severity of targeted disease symptoms or conditions in the subject. Suitable models in this regard include, for example, murine, rat, porcine, feline, non-human primate, and other accepted animal model subjects known in the art. Alternatively, effective dosages can be determined using *in vitro* models (e.g., immunologic and histopathologic assays). Using such models, only ordinary calculations and adjustments are typically required to determine an appropriate concentration and dose to administer a therapeutically effective amount of the biologically active agent (s) (e.g., amounts that are intranasally effective, transdermally effective, intravenously effective, or intramuscularly effective to elicit a desired response).

In an alternative embodiment, the invention provides compositions and methods for intranasal delivery of glucose-regulating peptide, wherein the glucose-regulating peptide compound(s) is/are repeatedly administered through an intranasal effective dosage regimen that involves multiple administrations of the glucose-regulating peptide to the subject during a daily or weekly schedule to maintain a therapeutically effective elevated and lowered pulsatile level of glucose-regulating peptide during an extended dosing period. The compositions and method provide glucose-regulating peptide compound(s) that are self-administered by the subject in a nasal formulation between one and six times daily to maintain a therapeutically effective elevated and lowered pulsatile level of glucose-regulating peptide during an 8 hour to 24 hour extended dosing period.

<u>Kits</u>

The instant invention also includes kits, packages and multicontainer units containing the above described pharmaceutical compositions, active ingredients, and/or means for administering the same for use in the prevention and treatment of diseases and other conditions in mammalian subjects. Briefly, these kits include a container or formulation that contains one or more glucose-regulating peptide proteins, analogs or mimetics, and/or other biologically active agents in combination with mucosal delivery enhancing agents disclosed herein formulated in a pharmaceutical preparation for mucosal delivery.

The intranasal formulations of the present invention can be administered using any spray bottle or syringe, or by instillation. An example of a nasal spray bottle is the, "Nasal Spray Pump w/ Safety Clip," Pfeiffer SAP # 60548, which delivers a dose of 0.1 mL per squirt and has a diptube length of 36.05 mm. It can be purchased from Pfeiffer of America of Princeton, NJ.

<u>Aerosol Nasal Administration of a Glucose-regulating Peptide</u>

We have discovered that the GRPs can be administered intranasally using a nasal spray or aerosol. This is surprising because many proteins and peptides have been shown to be sheared or denatured due to the mechanical forces generated by the actuator in producing the spray or aerosol. In this area the following definitions are useful.

1. Aerosol - A product that is packaged under pressure and contains therapeutically active ingredients that are released upon activation of an appropriate valve system.

2. Metered aerosol - A pressurized dosage form comprised of metered dose valves, which allow for the delivery of a uniform quantity of spray upon each activation.

3. Powder aerosol - A product that is packaged under pressure and contains therapeutically active ingredients in the form of a powder, which are released upon activation of an appropriate valve system.

4. Spray aerosol - An aerosol product that utilizes a compressed gas as the propellant to provide the force necessary to expel the product as a wet spray; it generally applicable to solutions of medicinal agents in aqueous solvents.

5. Spray-A liquid minutely divided as by a jet of air or steam. Nasal spray drug products contain therapeutically active ingredients dissolved or suspended in solutions or mixtures of excipients in nonpressurized dispensers.

6. Metered spray - A non-pressurized dosage form consisting of valves that allow the dispensing of a specified quantity of spray upon each activation.

7. Suspension spray- A liquid preparation containing solid particles dispersed in a liquid vehicle and in the form of course droplets or as finely divided solids.

The fluid dynamic characterization of the aerosol spray emitted by metered nasal spray pumps as a drug delivery device ("DDD"). Spray characterization is an integral part of the regulatory submissions necessary for Food and Drug Administration ("FDA") approval of research and development, quality assurance and stability testing procedures for new and existing nasal spray pumps.

Thorough characterization of the spray's geometry has been found to be the best indicator of the overall performance of nasal spray pumps. In particular, measurements of the spray's divergence angle (plume geometry) as it exits the device; the spray's cross-sectional ellipticity, uniformity and particle/droplet distribution (spray pattern); and the time evolution of the developing spray have been found to be the most representative performance quantities in the characterization of a nasal spray pump. During quality assurance and stability testing, plume geometry and spray pattern measurements are key identifiers for verifying consistency and conformity with the approved data criteria for the nasal spray pumps.

Definitions

Plume Height - the measurement from the actuator tip to the point at which the plume angle becomes non-linear because of the breakdown of linear flow. Based on a visual examination of digital images, and to establish a measurement point for width that is consistent with the farthest measurement point of spray pattern, a height of 30 mm is defined for this study.

Major Axis - the largest chord that can be drawn within the fitted spray pattern that crosses the COMw in base units (mm).

Minor Axis - the smallest chord that can be drawn within the fitted spray pattern that crosses the COMw in base units (mm).

Ellipticity Ratio - the ratio of the major axis to the minor axis, preferably between 1.0 and 1.5, and most preferably between 1.0 and 1.3.

$D_{10}$- the diameter of droplet for which 10% of the total liquid volume of sample consists of droplets of a smaller diameter ($\mu$m).

$D_{50}$ - the diameter of droplet for which 50% of the total liquid volume of sample consists of droplets of a smaller diameter ($\mu$m), also known as the mass median diameter.

$D_{90}$- the diameter of droplet for which 90% of the total liquid volume of sample consists of droplets of a smaller diameter ($\mu$m).

Span - measurement of the width of the distribution, the smaller the value, the narrower the distribution. Span is calculated as: $\frac{(D_{90} - D_{10})}{D_{50}}$.

% RSD - percent relative standard deviation, the standard deviation divided by the mean of the series and multiplied by 100, also known as % CV.

Volume- the volume of liquid or powder discharged from the delivery device with each actuation, preferably between 0.01 mL and about 2.5 mL and most preferably between 0.02 mL and 0.25 mL.

All publications, references, patents, patent publications and patent applications cited herein are each hereby specifically incorporated by reference in their entirety.

While this invention has been described in relation to certain embodiments, and many details have been set forth for purposes of illustration, it will be apparent to those skilled in the art that this invention includes additional embodiments, and that some of the details described herein may be varied considerably without departing from this invention. This invention includes such additional embodiments, modifications and equivalents. In particular, this invention includes any combination of the features, terms, or elements of the various illustrative components and examples.

The use herein of the terms "a," "an," "the," and similar terms in describing the invention, and in the claims, are to be construed to include both the singular and the plural. The terms "comprising," "having," "including," and "containing" are to be constl-ued as open-ended terms which mean, for example, "including, but not limited to." Recitation of a range of values herein refers individually to each separate value falling within the range as if it were individually recited herein, whether or not some of the values within the range are expressly recited. Specific values employed herein will be understood as exemplary and not to limit the scope of the invention.

The examples given herein, and the exemplary language used herein are solely for the purpose of illustration, and are not intended to limit the scope of the invention.

EXAMPLES

EXAMPLE 1

Insulin Aspart Formulations

Table 1 describes the twelve insulin aspart formulations tested using the *in vitro* EpiAirway Model System for the transepithelial resistance assay (TER), cell viability assay (MTT), lactate dehydrogenase cell death assay (LDH), and tissue permeation assay. The results were used to determine which formulation achieved the greatest degree of tissue permeation and TER reduction while resulting in no significant cell toxicity.

Insulin aspart is an insulin analog which is homologous with regular human insulin except for a single substitution of aspartic acid for proline at position B28. NovoLog (NovoLog™; Novo Nordisk Pharmaceuticals) is a sterile, aqueous, clear, and colorless solution, that contains insulin aspart (B28 asp regular human insulin analog) 100 Units/mL, glycerin 16 mg/mL, phenol 1.50 mg/mL, metacresol 1.72 mg/mL, zinc 19.6 $\mu$g/mL, disodium hydrogen phosphate dihydrate 1.25 mg/mL, and sodium chloride 0.58 mg/mL. NovoLog has a pH of 7.2-7.6. New insulin aspart formulations were generated. A total volume of 0.5 mL was manufactured for each formulation. The formulations contained varying concentrations of insulin aspart, NovoLog diluent, and the exciepients methyl-β-cyclodextrin (M-β-CD), L-α-phosphatidylcholine didecanoyl (DDPC), and disodium edetate (EDTA), alone or in combination. Controls without excipients were also included in the study. Small amounts of 2N HCl or NaOH was added, when necessary, to the formulations until the desired pH was achieved. The reagents used to prepare the formulations are shown in Table 2.

Table 1:

| Insulin Aspart Formulations for *In Vitro* Studies | | | | |
|---|---|---|---|---|
| Sample | Insulin aspart (U/mL) | Me-β-CD/ DDPC/EDTA (mg/mL) | % of NovoLog Diluent Remaining in Formulation | pH |
| 1 | 5 | 45/1/1 | 5% of NovoLog Diluent | 7 |
| 2 | 5 | 45/1/1 | 5% of NovoLog Diluent | 4 |
| 3 | 5 | 0 | 5% of NovoLog Diluent | 7 |
| 4 | 5 | 0 | 5% of NovoLog Diluent | 4 |
| 5 | 5 | 45/1/1 | 100% of NovoLog Diluent | 7 |
| 6 | 5 | 45/1/1 | 100% of NovoLog Diluent | 4 |
| 7 | 5 | 0 | 100% of NovoLog Diluent | 7 |
| 8 | 5 | 0 | 100% of NovoLog Diluent | 4 |
| 9 | 20 | 45/1/1 | 20% of NovoLog Diluent | 4 |
| 10 | 20 | 45/1/1 | 20% of NovoLog Diluent | 3 |
| 11 | 5 | 0/0/10 | 5% of NovoLog Diluent | 4 |
| 12 | 5 | 45/2//10 | 5% of NovoLog Diluent | 4 |

Table 2:

| Insulin Aspart Formulations Reagents | | | | |
|---|---|---|---|---|
| Reagent | Grade | Vendor | Cat # | Nastech Lot #/ Vendor Lot # |
| NovoLog | n/a | Novo Nordisk | n/a | PW51706 |
| Methyl-β-Cyclodextrin | Parma | Wacker | 60007005 | 71P018 |
| L-α-Phosphatidycholine didecanoyl | GMP | NOF | MC-1010 | 0412101 |
| Edetate Disodium | USP | Spectrum | ED150 | TF0419 |
| Sterile Water For Irrigation | USP | Spectrum/Braun | S1944 | J5C225 |
| 2N Hydrochloric Acid | Research | JT Baker | 5616-02 | B18512 |
| 2N Sodium Hydroxide | Research | JT Baker | 5633-02 | B06503 |

EXAMPLE 2

Nasal Mucosal Delivery - Permeation Kinetics and Cytotoxicity

The following methods are generally useful for evaluating nasal mucosal delivery parameters, kinetics and side effects for insulin within the formulations and method of the invention, as well as for determining the efficacy and characteristics of the various mucosal delivery-enhancing agents disclosed herein for combinatorial formulation or coordinate administration with insulin aspart. In one exemplary protocol, permeation kinetics and lack of unacceptable cytotoxicity are demonstrated for an intranasal delivery-enhancing agent as disclosed above in combination with a biologically active therapeutic agent, exemplified by insulin aspart.

Cell Cultures

The EpiAirway system was developed by MatTek Corp (Ashland, MA) as a model of the pseudostratified epithelium lining the respiratory tract. The epithelial cells are grown on porous membrane-bottomed cell culture inserts at an air-liquid interface, which results in differentiation of the cells to a highly polarized morphology. The apical surface is ciliated with a microvillous ultrastructure and the epithelium produces mucus (the presence of mucin has been confirmed by immunoblotting). The inserts have a diameter of 0.875 cm, providing a surface area of 0.6 cm$^2$. The cells are plated onto the inserts at the factory approximately three weeks before shipping.

EpiAirway™ culture membranes were received the day before the experiments started. They were shipped in phenol red-free and hydrocortisone-free Dulbecco's Modified Eagle's Medium (DMEM). Each tissue insert was placed into a well of a 6-well plate containing 0.9 ml of serum free DMEM. The membranes were then cultured for 24 hrs at 37 °C/5% $CO_2$ to allow tissues to equilibrate. Inserts are feed for each day of recovery. The DMEM-based medium is serum free but is supplemented with epidermal growth factor and other factors. The medium was tested for endogenous levels of any cytokine or growth factor considered for intranasal delivery, and was free of all cytokines and factors studied to date except insulin. The volume was sufficient to provide contact to the bottoms of the units on their stands, but the apical surface of the epithelium was allowed to remain in direct contact with air. Sterile tweezers were used in this step and in all subsequent steps involving transfer of units to liquid-containing wells to ensure that no air was trapped between the bottoms of the units and the medium.

The EpiAirway™ model system was used to evaluate the effect of each NovoLog containing formulation on TER, cell viability (MTT), cytotoxicity (LDH) and permeation. These assays are described below in detail. In all experiments, the nasal mucosal delivery formulation to be studied was applied to the apical surface of each unit in a volume of 100 μL, which was sufficient to cover the entire apical surface. An appropriate volume of the test formulation at the concentration applied to the apical surface (no more than 100 μL is generally needed) was set aside for subsequent determination of concentration of the active material by ELISA or other designated assay.

Transepithelial Electrical Resistance (TER)

TER measurements were read using a Tissue Resistance Measurement Chamber connected to an Epithelial Voltohmeter with the electrode leads, both from World Precision Instruments. First, background TER was read for each insert on the day the experiment began. After TER was read, 1 mL fresh media was placed in the bottom of each well in a 6-well plate. Inserts were drained on paper towel and placed into the new wells with fresh media, while keeping the inserts numbered to correlate with background TER measurements. 100 μL of experimental formulation was added to each insert. Inserts were placed in a shaking incubator at 100 rpm and 37°C for 1 hr.

The electrodes and a tissue culture blank insert were equilibrated for at least 20 minutes in fresh media with the power off prior to checking calibration. The background resistance was measured with 1.5 mL media in the Endohm tissue chamber and 300 μL media in a blank Millicell-CM insert. The top electrode was adjusted so that it was submerged in the media but not making contact with the top surface of the insert membrane. Background resistance of the blank insert was 5-20 ohms. For each TER determination, 300 μL media was added to the insert followed by a 20 minute incubation at RT before placement in the Endohm chamber to read TER. Resistance was expressed as (resistance measured - blank) x 0.6 cm$^2$. All TER values were reported as a function of the surface area of the tissue.

TER was calculated as:

$$\mathrm{TER} = (R_I - R_b) \times A$$

Where $R_I$ is resistance of the insert with a membrane, $R_b$ is the resistance of the blank insert, and A is the area of the membrane (0.6 cm2). A decrease in TER value relative to the control value (control = approximately 1000 ohms-cm$^2$; normalized to 100) indicates a decrease in cell membrane resistance and an increase in mucosal epithelial cell permeability. After the 1 hour incubation was complete, the tissue inserts were removed from the incubator. 200 μL fresh media was placed in each well of a 24-well plate and tissue inserts were transferred to the 24-well plate. 200 μL fresh media was gently added to each tissue insert. TER was again measured for each insert.

After the tissue culture inserts were transferred from the 6-well plate to the 24-well plate, the basal media was subdivided into three parts and stored in eppendorfs. All three subdivisions were placed at -80°C until use.

Lactate Dehydrogenase (LDH) Assay

The amount of cell death was assayed by measuring the release of LDH from the cells using a CytoTox 96 Cytotoxicity Assay Kit, from Promega Corp. Triplicate samples were performed for each tissue culture insert in the study. 50 μL harvested media (stored at 4°C) was loaded in triplicate in a 96-well plate. Fresh, cell-free media was used as a blank. 50 μL substrate solution, (12 mL Assay Buffer added to a fresh bottle of Substrate Mix, made according to the kit), was added to each well and the plates were incubates for 30 minutes at RT in the dark. Following incubation, 50 μL of stop solution was added to each well and the plates were read on a μQuant optical density plate reader at 490 nm using KCJr software.

### MTT Assay

The cell viability of each tissue culture insert was tested by MTT assay (MTT-100, MatTek kit) which tests mitochondrial reductase activity. This kit measures the uptake and transformation of tetrazolium salt to formazan dye. MTT concentrate was thawed and diluted with media at a ratio of 2 mL MTT: 8 mL media. The diluted MTT concentrate was pipetted (300 μL) into a 24-well plate. Tissue inserts were gently dried, placed into the plate wells, and incubated for three hours in the dark at 37°C. After incubation, each insert was removed from the plate, blotted gently, and placed into a 24-well extraction plate. The cell culture inserts were then immersed in 2.0 mL of the extractant solution per well (to completely cover the sample). The extraction plate was covered and sealed to reduce evaporation of extractant. After an overnight incubation at room temperature in the dark, the liquid within each insert was decanted back into the well from which it was taken, and the inserts discarded. The extractant solution (50 μL) from each well was pipetted in triplicate into a 96-well microtiter plate, along with extract blanks and diluted with the addition of 150 μL of fresh extractant solution. The optical density of the samples was measured at 550 nm on a μQuant optical density plate reader using KCJr software.

### TER Results

The TER measurements (ohms x cm2) before and after the incubation of 1 hour at 37°C for the experimental formulations were compared to the controls. The results show that the formulations containing enhancers, except for #7 and #8, have a significant TER reduction after one hour incubation.

### MTT Results

Nearly all formulations showed fair to good cell viability compared to the controls. The % MTT for most formulations was greater than 80% (except #1, #6, and #12).

### LDH Results

Most of the formulations tested showed very little LDH, indicating very low cyctotoxicity.

### Summary

The results of the TER, MTT and LDH assays indicate that formulation #2, #6, #9, #10, and #11 all show a significant reduction in TER without increased toxicity.

### EXAMPLE 3

### Insulin Aspart Permeability

ELISA was used to quantitate the amount of insulin or insulin analog that permeated across the apical to the basolateral side of the insert. Insulin is present in the MaTtek media so raw data was corrected by subtracting the average concentration present in the media sample from all other samples.

Iso-Insulin ELISA kits are purchased from Alpco Diagnostics, (Windham, NH, Cat #08-10-1128-01). Samples were diluted with assay buffer that was provided with the kit. Dilution was mixed into clear silanized HPLC vials with Teflon coated covers by gently inverted. The optical density of the samples was measured at 450 nm (as indicated in the protocol) on μQuant optical density plate reader using KCJr software.

The loading volume was 100 μL per insert and the permeation sampling time was 60 minutes. Each formulation; as well as controls, were tested using n= 3 inserts. Controls for this study included MatTek basal media and 9% Triton X -100. Each tissue insert was placed in an individual well containing 0.95 mL of MatTek basal media. On the apical surface of the inserts, 100 μL of test formulations was applied according to study design, and the samples were placed on a shaker (~100 rpm) for 1 hour at 37°C. At the end of the incubation period, 50 μL of ~20,000 KIUnits of aprotnin was added to each underlying culture media samples and stored at 2-8°C for ELISA analysis.

Table 3 shows the permeability results from the basolateral samples assayed by ELISA. Averages were corrected by subtracting the average amount of insulin present in the media alone samples from the experimental samples.

Table 3:

% Permeation Results Testing Insulin Aspart Formulations

| Sample # | μU/mL | | | | | | % | |
|---|---|---|---|---|---|---|---|---|
| | Insert a | Insert b | Insert c | Avg | Avg Corrected * | StDev | Permeation | StDev |
| 1 | 143346 | 121821 | 149478 | 138215 | 76124 | 14525 | 15.2 | 2.9 |
| 2 | 109255 | 130398 | 130532 | 123395 | 61304 | 12246 | 12.3 | 2.4 |
| 3 | 56179 | 68222 | 73138 | 65846 | 3755 | 8726 | 0.8 | 1.7 |
| 4 | 49425 | 65647 | 75448 | 63507 | 1415 | 13143 | 0.3 | 2.6 |
| 5 | 60867 | 61155 | 60857 | 60960 | -1132 | 169 | -0.2 | 0.0 |
| 6 | 51795 | 62832 | 65177 | 59935 | -2157 | 7146 | -0.4 | 1.4 |
| 7 | 38615 | 58850 | 69527 | 55664 | -6428 | 15700 | -1.3 | 3.1 |
| 8 | 50687 | 67669 | 63726 | 60694 | -1398 | 8888 | -0.3 | 1.8 |
| 9 | 248232 | 204930 | 281721 | 244961 | 182870 | 38500 | 9.1 | 1.9 |
| 10 | 246821 | 288253 | 265140 | 266738 | 204647 | 20762 | 10.2 | 1.0 |
| 11 | 113468 | 171342 | 171056 | 151955 | 89864 | 33331 | 18.0 | 6.7 |
| 12 | 190538 | 148489 | 169822 | 169616 | 107525 | 21025 | 21.5 | 4.2 |
| Media | | 51745 | 72438 | 62092 | 0 | 14632 | 0.0 | 2.9 |

These permeation results show that permeation enhancers can be employed to deliver insulin aspart via intranasal formulations. All enhancer containing formulations resulted in at least 9% (~9-21%) permeation compared to formulations without enhancers which yielded at most, ~1-2 %. The samples with the greatest enhancement in permeability included #1, #2, #9, #10, #11, and #12. When taken together with the TER, MTT, and LDH results, samples #2 (5 U/mL Insulin aspart, 45 mg/mL Me-β-CD, 1 mg/mL DDPC, 1 mg/mL EDTA, 5% NovoLog Diluent, pH 4); #9 (20 U/mL Insulin aspart, 45 mg/mL Me-β-CD, 1 mg/mL DDPC, 1 mg/mL EDTA, 20% NovoLog Diluent, pH 4); #10 (20 U/mL Insulin aspart, 45 mg/mL Me-β-CD, 1 mg/mL DDPC, 1 mg/mL EDTA, 20% NovoLog Diluent, pH 3); and #11 (5 U/mL Insulin aspart, 0 mg/mL Me-β-CD, 0 mg/mL DDPC, 10 mg/mL EDTA, 5% NovoLog Diluent, pH 4) have the greatest enhancement in permeability with the least cell toxicity.

EXAMPLE 4

NPG and PN159 Effects on Insulin Permeability

*In vitro* experiments evaluated the effect of both small molecule and peptide-based permeation enhancers on permeability of insulin. Seven different treatments were applied to EpiAirway 96 well plates for 1 hr at 37°C with 0.1 U insulin applied on the apical side. The standard curve and high low controls were as expected, and the insulin spike in the basolateral media showed nearly 100% recovery. Treatments included: PBS + insulin 0.1 U; 25 μM PN159 + insulin 0.1 U; 50 μM PN159 + insulin 0.1 U; PDF + insulin 0.1 U; PBS + insulin 0.1 U+ NTPG 150 mM; 25 uM PN159 + insulin 0.1U+NPG 150 mM; and PDF + insulin 0.1 U + NPG 150 mM.

The insulin used in this study is the Sigma recombinant (yeast derived) human insulin, natural sequence. This recombinant human insulin is derived from pro-insulin and is chemically, physically, and biologically identical to pancreatic human insulin. PBS is phosphate buffered saline. PDF is a mixture consisting of 45 mg/mL methyl-β-cyclodextrine, 1 mg/mL ethylenediamine tetracetate, 1 mg/mL didecanoylphosphatidle choline, and 10 mM acetate, pH 5.5. The monnomeric stabilizer (NPG) is N-pivaloyl glucosamine. PN159 is a peptide, described in copending U.S. Patent Application No. 11/233,239. The results are shown in Table 4, as follows:

Table 4:

| Permeability Results With PDF, PN1 59, and NPG Formulations | | | |
|---|---|---|---|
| | Mean % Permeability | SD | Fold Enhancement |
| PBS | 0.254 | 0.028 | 1.00 |
| PN159 25 $\mu$M | 0.807 | 0.289 | 3.173 |
| PN159 50 $\mu$M | 2.504 | 0.814 | 9.849 |
| PDF | 3.110 | 2.007 | 12.235 |
| NPG | 0.256 | 0.045 | 1.009 |
| PN159 25 $\mu$M / NPG | 1.839 | 1.080 | 7.233 |
| PDF / NPG | 7.673 | 0.817 | 30.184 |

PN159 increased permeability of insulin: 25 $\mu$M PN159 resulted in 0.8% permeability, while 50 $\mu$M PN159 increased permeability to 2.5%. Combined with NPG, 25 $\mu$M PN159 resulted in 1.8% permeability. The results showed that PDF alone provided 3% permeability, approximately 12-fold greater than PBS alone. When PDF was combined with NPG the permeability increased to 7.6%, a 30-fold increase over PBS.

A further study was conducted to test the TER, LDH, MTT, and permeability of the formulations shown in Table 5, including formulations containing PN159. All formulations were tested with n=3 inserts in the EpiAirway model. Regular insulin was approximately 28 U/mg (i.e., 200 U/mL = ~7.14 mg/mL).

Table 5:

| # | Regular Insulin (U/mL) | Me-β-CD (mg/mL) | EDTA (mg/mL) | Tween 80 (mg/mL) | PN159 (μM) | Arg Buffer (mM) | NaCl (mg/mL) | MP (mg/mL) | PP (mg/mL) | PG (mg/mL) | pH |
|---|---|---|---|---|---|---|---|---|---|---|---|
| | | | | | Formulations | | | | | | |
| 1 | 200 | 45 | 1 | 10 | 0 | 10 | 0 | 0.33 | 0.17 | 10 | 7.3 |
| 2 | 1000 | 45 | 1 | 10 | 0 | 10 | 0 | 0.33 | 0.17 | 10 | 7.3 |
| 3 | 1200 | 45 | 1 | 10 | 0 | 10 | 0 | 0.33 | 0.17 | 10 | 7.3 |
| 4 | 400 | 45 | 1 | 10 | 0 | 10 | 0 | 0.33 | 0.17 | 10 | 7.3 |
| 5 | 400 | 0 | 0 | 0 | 25[+] | 10 | 4 | 0 | 0 | 0 | 7.3 |
| 6 | 400 | 0 | 0 | 0 | 50[+] | 10 | 4 | 0 | 0 | 0 | 7.3 |
| 7 | 400 | 45 | 0 | 10 | 50[+] | 10 | 4 | 0 | 0 | 0 | 7.3 |
| 8 | 400 | 0 | 0 | 0 | 0 | 10 | 7 | 0 | 0 | 0 | 7.3 |
| 9 | PBS Control | | | | | | | | | | |
| 10 | 9% Triton X 100 Control | | | | | | | | | | |
| Abbreviations: Arg=Arginine, Me-β-CD=methyl-beta-cyclodextrin, EDTA=disodium edetate, NaCl = sodium chloride, MP = methylparaben sodium, PP = propylparaben sodium, PG = propylene glycol | | | | | | | | | | | |

The results of the TER measurements (ohms x cm2) before and after the incubation of 1 hour at 37 °C for the experimental formulations were compared to the controls. The results show that the formulations containing enhancers, had a significant TER reduction after one hour incubation. Formulations #5, #6, and #8 showed had cell viability and all formulations, except #7, had minimal cell toxicity, similar to PBS control. Permeation results are shown in Table 6.

Table 6:

| # | Avg % perm | STDEV |
|---|---|---|
| | Permeation Results | |
| 1 | 4.47 | 0.70 |
| 2 | 1.99 | 0.12 |
| 3 | 2.40 | 0.25 |
| 4 | 2.17 | 0.13 |
| 5 | 1.42 | 0.34 |
| 6 | 1.48 | 0.17 |
| 7 | 4.66 | 1.68 |
| 8 | 0.00 | 0.00 |

Formulations #1, (4.47%) and #7 (4.66%) had the highest percent permeability. These data show that addition of PN159 did not significantly enhance permeation of insulin over the PDF (Me-$\beta$-CD, DDPC, and EDTA) formulation *in vitro.*

EXAMPLE 5

Effect of Alternative Buffers on Insulin Permeability

A permeability study to compare alternative buffers in combination with the PDF formulation (Me-$\beta$-CD, DDPC, and EDTA) was performed. These permeability data were generated by ELISA (from LINCO Research, Inc. Catalogue # EZHI-14K) after a 60-minute incubation and using a 50 $\mu$L loading volume. All formulations listed in Table 7 had good cell viability and low cytotoxicity as measured by MTT and LDH assays.

Table 7:

Permeability Results With Alternative Buffers

| # | Insulin Conc. (U/mL) | Me-β-CD/DDPC/ EDTA (mg/mL) | Cremophor EL conc. (mg/mL) | Tween 80 conc. (mg/mL) | Phosphate buffer conc. (mM) | Acetate buffer conc. (mM) | Arginine buffer conc. (mM) | Sodium Chloride conc. (mg/mL) | pH | % Perm | StDev |
|---|---|---|---|---|---|---|---|---|---|---|---|
| 1 | 280 | 45/1/1 | 0 | 0 | 0 | 10 | 0 | 4 | 3 | 9.25 | 1.06 |
| 2 | 280 | 45/1/1 | 0 | 0 | 0 | 10 | 0 | 4 | 4 | 10.88 | 3.29 |
| 3 | 280 | 22.5/.5/.5 | 0 | 0 | 0 | 10 | 0 | 4 | 3 | 11.76 | 1.14 |
| 4 | 280 | 45/1/1 | 0 | 0 | 10 | 0 | 0 | 4 | 7 | 28.3 | 9.54 |
| 5 | 280 | 45/1/1 | 0 | 0 | 0 | 10 | 0 | 4 | 3.5 | 12.82 | 2.29 |
| 6 | 280 | 45/1/1 | 5 | 0 | 0 | 10 | 0 | 4 | 3.5 | 12.28 | 4.6 |
| 7 | 280 | 45/1/1 | 10 | 0 | 0 | 10 | 0 | 4 | 3.5 | 11.99 | 2.16 |
| 8 | 280 | 45/1/1 | 0 | 1 | 0 | 10 | 0 | 4 | 3.5 | 11.32 | 2.73 |
| 9 | 280 | 45/1/1 | 0 | 10 | 0 | 10 | 0 | 4 | 3.5 | 7.61 | 1.15 |
| 10 | 840 | 45/1/1 | 0 | 0 | 10 | 0 | 0 | 4 | 7 | 12.82 | 1.57 |
| 11 | 840 | 45/1/1 | 5 | 0 | 10 | 0 | 0 | 4 | 7 | 13.86 | 5.72 |
| 12 | 280 | 45/1/1 | 0 | 0 | 10 | 0 | 0 | 4 | 7 | 7.14 | 4.5 |
| 13 | 840 | 45/1/1 | 0 | 10 | 10 | 0 | 0 | 4 | 7 | 5.45 | 3.12 |
| 14 | 280 | 45/1/1 | 0 | 0 | 0 | 0 | 10 | 4 | 7 | 5.1 | 2.2 |
| 15 | 840 | 45/1/1 | 0 | 0 | 0 | 0 | 10 | 4 | 7 | 3.72 | 2.69 |
| 16 | 280 | 0/0/0 | 0 | 0 | 0 | 10 | 0 | 7 | 3.5 | 0.000 03 | 0.000 02 |

Arginine buffer was a modest performing buffer with the percent permeability of insulin at 3% to 6%. Acetate buffer formulations achieved % permeability of 7% to 12%. The % permeability for phosphate buffer formulations ranged from 5% to 28%. The highest % permeability, 28%, was achieved with 10mM phosphate buffer, 45mg/mL Me-β-CD, 1mg/mL DDPC, 1mg/mL EDTA, 280 U/mL Insulin at pH 7 (#4).

EXAMPLE 6

In vitro Screening Studies for Optimal Intranasal Insulin Formulation

A preliminary *in vitro* screen for 280 U/mL and 840 U/mL insulin formulations at varying component concentrations and pH ranges was performed. The base 1X PDF formulation included 45 mg/mL Me-β-CD, 1 mg/mL DDPC, 1 mg/mL EDTA, 10 mM Acetate, 10 mM Phosphatase, and 220 mOsm/kg·NaCl. The *in vitro* Study 1 formulation components are shown in Table 8.

Table 8:

| In Vitro Study 1 Formulation Components | | | |
|---|---|---|---|
| Component | Concentration (mg/mL) | | |
| Regular Insulin | 10 (280 U/mL) | | 30 (840 U/mL) |
| Me-β-CD | 45 | | 90 |
| DDPC | 0.5 | 1 | 2 |
| EDTA | 0.5 | 1 | 2 |
| Acetate | 0.6 (10 mM) | | |
| Phosphatase | 1.4 (10 mM) | | |
| NaCl | Qs to ~220 mOsm/kg $H_2O$ | | |
| pH | 3 | 4 | 7 |

The time course tested for the first *in vitro* experiment included 30, 60, and 120-minute incubations. Incubations were done in PBS with $Ca^{++}$ and $Mg^{++}$ (note insulin is present in the standard MatTek media). Assay conditions included 100 rpm spins at 37°C and application of 50 μL of the tested formulation. A significant TER reduction was observed with all formulations. High toxicity was observed with the MTT assay, and low cell viability was observed with the LDH assays.

Permeation data for the 280 U/mL insulin concentration was taken at 30 and 60 minutes and compared 1X PDF, pH 3; 2X PDF, pH 3; 1X PDF, pH 4; and 0.5X PDF, pH 3. Approximately 10% permeation was achieved with 1X PDF, pH 3; 1X PDF, pH 4; and 0.5X PDF, pH 3 at 60 minutes. 2X PDF, pH 3.resulted in 2% permeation. The no enhancer control showed less than 1% permeation.

Permeation data for the 280 U/mL insulin and 840 U/mL insulin concentrations were compared at 30 and 60 minutes in the 2X PDF, pH 3 formulation. No significant differences in permeation were observed due to the high variability, possibly caused by precipitation at the high insulin concentration.

Permeation data for the 280 U/mL insulin concentration taken at 30 and 60 minutes in 1X PDF, pH 3 was compared to 1X PDF, pH 7. The results showed that the formulation at pH 7 performed better than at pH 3. Percent permeation for 1X PDF, pH 3 was 10% while permeation for 1X PDF, pH 7 was 35%.

Summary of *In Vitro* Study 1

Permeation studies with 30, 60, and 120 minute incubations in PBS (with $Ca^{++}$ and $Mg^{++}$) resulted in high cytoxicity and low cell viability. 1X and 0.5X·PDF resulted in better permeation than 2X PDF. PDF was able to solubilize 10 mg/mL (i.e., 280 U/mL), but not 30 mg/mL (i.e., 840 U/mL). Percent permeation results were higher for pH 7 than pH 3.

A second study was conducted using 1X PDF (45 mg/mL Me-β-CD, 1 mg/mL DDPC, 1 mg/mL EDTA, 10 mM Acetate, 10 mM Phosphatase, and 220 mOsm/kg NaCl as the base formulation. The *in vitro* Study 2 formulation components are shown in Table 9.

Table 9:

| In Vitro Study 2 Formulation Components | | | |
|---|---|---|---|
| Component | Concentration (mg/mL) | | |
| Regular Insulin | 10 (280 U/mL) | | 30 (840 U/mL) |
| Me-β-CD | 45 | | |
| DDPC | 1 | | |
| EDTA | 1 | | |
| Polysorbate 80 (Tween 80) | 0 | 1 | 10 |
| Cremophor EL (CEL) | 0 | 5 | 10 |
| Acetate | 0.6 (10 mM) | | |
| Phosphatase | 1.4 (10 mM) | | |
| NaCl | Qs to ~220 mOsm/kg $H_2O$ | | |

(continued)

| In Vitro Study 2 Formulation Components | | |
|---|---|---|
| Component | Concentration (mg/mL) | |
| pH | 3.5 | 7 |

The time course for the second *in vitro* experiment included a 60 minute incubation. The incubation was done in PBS with $Ca^{++}$ and $Mg^{++}$. Assay conditions included 100 rpm spins at 37°C and application of 50 μL of the tested formulation. A significant TER reduction was observed with all formulations. High toxicity was observed with the MTT assay, and low cell viability was observed with the LDH assays, even without solubolizers.

Permeation data for the 280 U/mL insulin concentration was taken at 60 minutes and compared in 1X PDF, pH 3.5; 1X PDF, pH 7; 1X PDF + 0.5% CEL, pH 3.5; 1X PDF + 1% CEL, pH 3.5; 1X PDF + 0.1% Tween 80, pH 3.5; and 1X PDF + 1% Tween 80, pH 3.5. At least 10% permeation was achieved with all formulations at pH 3.5. Permeation was increased in the formulations at pH 7 compared to pH 3.5.

Permeation data for the 280 U/mL and 840 U/mL insulin concentrations were compared at 60 minutes in the 1X PDF, pH 7; 1X PDF + 0.5% CEL, pH 3.5; 1X PDF + 0.5% CEL, pH 7; 1X PDF + 1% CEL, pH 3.5; 1X PDF + 0.1% Tween 80, pH 3.5; and 1X PDF + 1% Tween 80, pH 3.5 formulations. The 840 U/mL insulin formulations were visually soluble on the surface of the insert when solubilizers were present. All formulations had similar permeability, with the exception of formulations at pH 7 having a higher permeability than formulations at pH 3.5.

Summary of *In Vitro* Study 2

High concentration insulin formulations (840 U/mL) were successfully stabilized (and solubilized) in the presence of an additional surface active agent (i.e., Tween or Cremophor). Even at 60 minute incubation, increased cytoxicity and decreased cell viability were observed for all formulations *in vitro.*

A third study was conducted to determine the *in vitro* effects on permeation of three, different surface active agents (Tween 80, Tween 20, and Pluronic F68) using 280 U/mL and 840 U/mL 1X PDF insulin formulations at pH 7. The *in vitro* Study 3 formulation components are shown in Table 10.

Table 10:

| In Vitro Study 3 Formulation Components | | | |
|---|---|---|---|
| Component | Concentration (mg/mL) | | |
| Regular Insulin | 10 (280 U/mL) | 30 (840 U/mL) | |
| Me-β-CD | **45** | | |
| DDPC | 0 | 1 | |
| EDTA | 1 | 10 | |
| Tween 80 | 0 | 1 | 10 |
| Tween 20 | 0 | 1 | 10 |
| Pluronic F68 | 0 | 0.1 | 1 |
| Arginine | 2.1(10mM) | | |
| NaCl | Qs to ~220 mOsm/kg $H_2O$ | | |
| pH | 7 | | |

The time course for the third *in vitro* experiment included a 60 minute incubation. The incubation was done in PBS with $Ca^{++}$ and $Mg^{++}$. Assay conditions included 100 rpm spins at 37°C and application of 50 μL of the tested formulation. A significant TER reduction was observed with all formulations. High toxicity was observed with the MTT assay, and low cell viability was observed with the LDH assays.

Permeation data for the 280 U/mL and 840 U/mL insulin concentration were taken at 60 minutes and compared 1X PDF + 1% Tween 80, pH 7; 1X PDF + 0.01 % Pluronic F68, pH 7; and 1X PDF + 0.1% Pluronic F68, pH 7 formulations. Results showed that pluronic F68 does not solubilize insulin sufficiently to enhance permeation.

The effects of Tween 80 and Tween 20 on insulin permeation in the 1X PDF formulation (insulin concentration 280 U/mL and 840 U/mL) were tested. Percent permeation data were compared at 60 minutes in the 1X PDF + 1% Tween 80, pH 7; 1X PDF + 0.1% Tween 80, pH 7; 1X PDF (no DDPC) + 1% Tween 80, pH 7; 1X PDF (no DDPC) + 0.1% Tween 80, pH 7; 1X PDF + 1% Tween 20, pH 7; 1X PDF + 0.1% Tween 20, pH 7; and 1X PDF + 1% Tween 80, pH 7 (Hypotonic) formulations. Results showed that 1% Tween (both Tween 80 and Tween 20) provides greater permeation than 0.1% Tween. Permeation results for Tween 20 were the same as Tween 80. Removal of DDPC had no effect upon % permeation in these formulations.

Increasing amounts of Tween 80 (0.01%, 0.1%, 0.5%, and 1 %) were tested for the effect on permeation for 280 U/mL insulin in the 1X PDF formulations at pH 7. The amount of Tween 80 in the formulation effected the % permeation. Permeation was increased with increasing concentration of Tween 80. Further analysis of the effect of Tween concentration on permeation was assayed in the 1X PDF formulations at 1%, 2%, and 5% Tween 80. In addition, permeation with the 2 X PDF formulations was tested with 1% and 2% Tween 80. The results showed that once the Tween 80 concentration was above 1%, in either 1X or 2X PDF, there was no further enhancement of *in vitro* permeation.

The effect of removing Me-β-CD on permeation was tested with the 0.1 % Tween and 1% Tween formulations (containing 1 mg/mL and 10 mg/mL EDTA). Removal of Me-β-CD from the formulation resulted in a dramatic decrease in permeation. The results were observed , with both 280 U/mL and 840 U/mL formulations.

Summary of *In Vitro* Sturdy 3

Tween 80 and Tween 20 both resulted in good permeation *in vitro* when used in combination with the 1X PDF formulations. Pluronic F68 did not enhance permeation Tween 80 or 20 alone is not sufficient to achieve increased permeation of insulin *in vitro.* Removal of Me-β-CD from the formulation resulted in a significant decrease in insulin permeation. Increasing Tween up to 1% resulted in increased permeation, but above 1% no additional benefit was observed. A 1 % Tween formulation is lower than some marketed Tween containing nasal products.

EXAMPLE 7

Insulin Formulation Stability Data

An in-use stability study for up to 28 days at 5 °C, 25 °C, 40 °C, and 50 °C was conducted for 1X PDF (45 mg/mL Me-β-CD, 1 mg/mL DDPC, 1 mg/mL EDTA, 10 mM Acetate, 10 mM Phosphatase, and 220 mOsm/kg NaCl) insulin spray formulations. HPLC was used to assay % peptide recovery. The formulation parameters evaluated in the study are shown in Table 11.

Table 11:

| Preliminary Insulin Stability Study Formulation Parameters | | | |
|---|---|---|---|
| Component | Concentration (mg/mL) | | |
| Regular Insulin | 10 (280 U/mL) | 30 (840 U/mL) | |
| Me-β-CD | 0 | 45 | |
| DDPC | 0 | 1 | |
| EDTA | 0 | 1 | |
| Tween 80 | 0 | 1 | 10 |
| Arginine | 2.1 (10mM) | | |
| NaCl | Qs to ~220 mOsm/kg $H_2O$ | | |
| pH | 3.5 | 7 | |

1X PDF insulin spray formulations remained more stable compared to insulin stored with salt and buffer alone. The presence of Tween did not affect stability of the 1X PDF formulations. Formulations at pH 7.0 maintained much better stability than formulations at pH 3.5. Stability of insulin stored in 1X PDF was very good out to 28 days at 5°C, 25°C, 40°C, and 50°C, approximately 100% label claim recovery was observed for both 280 U/mL and 840 U/mL insulin concentrations.

Further in-use assays (unit dose and 8-day) were conducted to evaluate the stability of 1X PDF insulin spray formulations. "Unit dose" was used to assess the stability of insulin spray after priming and one actuation. The conditions for the 8-day in-use study included 8-day, thrice daily (TID) actuation at 5°C and 30°C storage. The studies assayed for peptide content. The results of the in-use peptide content studies showed that the PDF insulin spray formulations demonstrate good stability for both unit dose and for 8-day in-use. Stability at the storage temperature 30°C appears to be as stable as storage temperature 5°C in this study.

EXAMPLE 8

Intranasally Administered Insulin Pharmacokinetics Results in Rabbits

Pharmacokinetic (PK; i.e., insulin measurements) values were measured for insulin treated New Zealand White Rabbits at specified time-points up to 240 minutes. Four (4) intranasal (IN) groups, one (1) subcutaneous (SC) group, and one (1) intravenous (IV) group were included in the study that generated bioavailability data. Each group included 5 male rabbits. All data calculations are dose normalized and the PK data was baseline corrected. Treatment and dosage details for PK Study 1 are shown in Table 12.

Table 12:

| Treatment and Dosage Details for Rabbit PK (and PD) Study 1 | | | | | | | | |
|---|---|---|---|---|---|---|---|---|
| Route/ Group ID | Insulin Dose Level (IU/kg) | Me-β-CD (mg/mL) | DDPC (mg/mL) | EDTA (mg/mL) | Tween 80 (mg/mL) | Arg Buffer (mM) | NaCl (mg/mL) | pH |
| IN/1XPDF (1) | 3 | 45 | 1 | 1 | 0 | 10 | 4 | 7 |
| IN/1X PDF-Tween (2) | 3 | 45 | 1 | 1 | 10 | 10 | 4 | 7 |
| IN/1X PDF-Tween (3) | 6 | 45 | 1 | 1 | 10 | 10 | 4 | 7 |
| IN-Control (4) | 3 | 0 | 0 | 0 | 0 | 10 | 7 | 7 |
| SC (5) | 0.6 | 0 | 0 | 0 | 0 | 10 | 9 | 7 |
| IV-Infusion (6) | 0.3 | 0 | 0 | 0 | 0 | 10 | 9 | 7 |

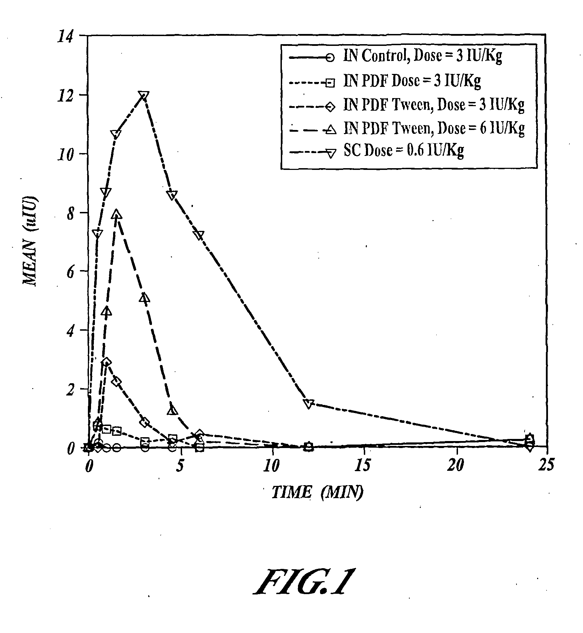

The results of the PK Study 1 are shown in Table 13 and Figure 1. IN administration of insulin resulted in a quicker $T_{max}$ than regular SC insulin. IN/1X PDF with 1% Tween (dose 6 IU/kg), #3, showed the highest peak of the intranasal formulations. Percent bioavailability (BA) of insulin was ~3-5 % for both IN/1X PDF with 1 % Tween formulations, #2 and #3, (relative to SC). Absolute % BA for SC was 30% while IN was 1%. % CV for IN was 50% while SC was 20%, based on AUC.

Table 13:

| PK Study 1 Results in Rabbit | | | | |
|---|---|---|---|---|
| Formulation (Group) | Dose (IU/kg) | $T_{max}$ (min) | $C_{max}$ (uIU/mL) | $AUC_{last}$ (min*uIU/mL) |
| IN/1X PDF (1) | 3 | 16.00 | 11.02 | 411.20 |
| IN/1X PDF-Tween(2) | 3 | 11.67 | 35.38 | 543.44 |
| IN/1X PDF-Tween (3) | 6 | 18.00 | 81.00 | 2118.50 |
| IN-Control (4) | 3 | 240.00 | 2.32 | 164.00 |
| SC-Regular (5) | 0.6 | 30.00 | 128.28 | 7744.40 |
| IV-Infusion (6) | 0.3 | 10.00 | 1352.30 | 12701.90 |

A second PK study was conducted to compare intranasal PDF plus Tween formulations with the NovoLog rapid-acting formulation (NovoLog diluent consists of: 16 mg/mL glycerin, 1.5 mg/mL phenol, 1.72 mg/mL m-cresol, 19.6 μg/mL zinc, 1.25 mg/mL disodium hydrogen phosphate dihydrate, and 0.58 mg/mL NaCl, pH 7.2 - 7.6). The parameters ofPK Study 2 are shown in Table 14.

Table 14:

| Formulation (Group) | insulin Dose (IU/kg) | Me-β-CD (mg/mL) | DDPC (mg/mL) | EDTA (mg/mL) | Tween 80 (mg/mL) | Arg Buffer (mM) | NaCl (mg/mL) | pH |
|---|---|---|---|---|---|---|---|---|
| Formulation Parameters for PK (and PD) Study 2 | | | | | | | | |
| IN/1X PDF 1% Tween | 6 | 45 | 1 | 1 | 10 | 10 | 4 | 7 |
| IN/1X PDF (no DDPC) | 6 | 45 | 0 | 1 | 10 | 10 | 4 | 7 |
| IN/1X PDF 2% Tween | 6 | 45 | 1 | 1 | 20 | 10 | 4 | 7 |
| IN/1X PDF 5% Tween | 6 | 45 | 1 | 1 | 50 | 10 | 4 | 7 |
| IN/2X PDF 1% Tween | 6 | 90 | 2 | 2 | 10 | 10 | 4 | 7 |
| IN 2X PDF 2% Tween | 6 | 90 | 2 | 2 | 20 | 10 | 4 | 7 |
| SC-PDF | 0.6 | 45 | 1 | 1 | 10 | 10 | 4 | 7 |
| SC-NovoLog | 0.6 IU/kg (3 U/mL) NovoLog in NovoLog Dilutent | | | | | | | 7.4 |

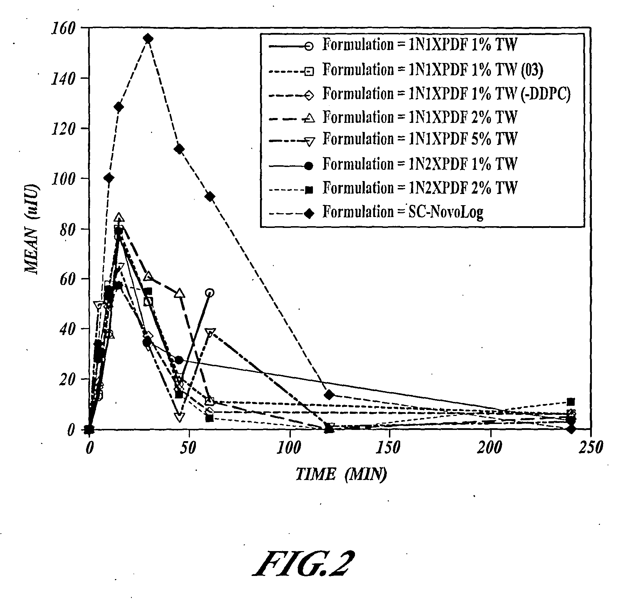

Shown in Table 15 are the $T_{max}$, % $C_{max}$, $AUC_{last}$, $AUC_{inf}$, and % BA relative to SC-Novolog results for Study 2 with PK baseline subtracted; also included are Study 1 results for the IN/1X PDF 1% Tween (#3) and SC-Regular (#5) formulations. The PK Study 2 results are shown in Figure 2. The PK curve for Study 2 is similar to the curve seen in Study 1, showing that IN/1X PDF results in a rapid-acting PK profile. A second peak of insulin was observed in some IN treated animals.

Table15:

| Formulation (Group) | $T_{max}$ (min) | %$C_{max}$ (uIU/mL) | $AUC_{last}$ (min*ul,U/mL) | $AUC_{inf}$ (min*uIU/mL) | %BA |
|---|---|---|---|---|---|
| PK Study 2 Results in Rabbit | | | | | |
| IN/1X PDF 1% Tween | 30 | 73.84 | 1766.20 | 3445.22 | 2.2 |
| IN/1X PDF 1% Tween* | 18 | 81.00 | 2397.00 | 4192.93 | 2.9 |
| IN/1X PDF (no DDPC) | 19 | 56.32 | 1549.00 | 2868.44 | 1.9 |
| IN/1X PDF 2% Tween | 27 | 97.65 | 4106.48 | 2436.22 | 5.0 |
| IN/1X PDF 5% Tween | 24 | 65.30 | 1412.40 | 2253.16 | 1.7 |
| IN/2X PDF 1% Tween | 15 | 79.24 | 2744.00 | 4173.69 | 3.4 |
| IN 2X PDF 2% Tween | 22.5 | 73.28 | 2283.34 | 7819.15 | 2.8 |
| SC-Regular* | 30 | 128.38 | 7750.15 | 8982.12 | 95.0 |
| SC-PDF | 29 | 141.60 | 5830.50 | 8821.04 | 71.4 |

(continued)

| PK Study 2 Results in Rabbit | | | | | |
|---|---|---|---|---|---|
| Formulation (Group) | $T_{max}$ (min) | $\%C_{max}$ (uIU/mL) | $AUCl_{ast}$ (min*ul,U/mL) | $AUC_{inf}$ (min*uIU/mL) | %BA |
| SC NovoLog | 23 | 168.84 | 8160.70 | 12338.64 | |
| *Results from PK Study 1 | | | | | |

The results from PK Study 2 show that the IN/1X PDF 2% Tween has the highest % BA, $C_{max}$ and $AUC_{last}$ of the intranasal formulations tested. The % BA, $C_{max}$ and $AUC_{last}$ were decreased when DDPC was removed. The IN/1X PDF 1% Tween results for Study 2 were consistent with the results from Study 1. SC-Regular, SC-Novolog, and SC-PDF insulin resulted in similar bioavailability. For the % BA, intranasal formulations resulted in approximately 2-5% bioavailability. IN/1X PDF 2% Tween showed the highest bioavailability at 5%.

EXAMPLE 9

Intranasally Administered Insulin Pharmacodynamics Data in Rabbits

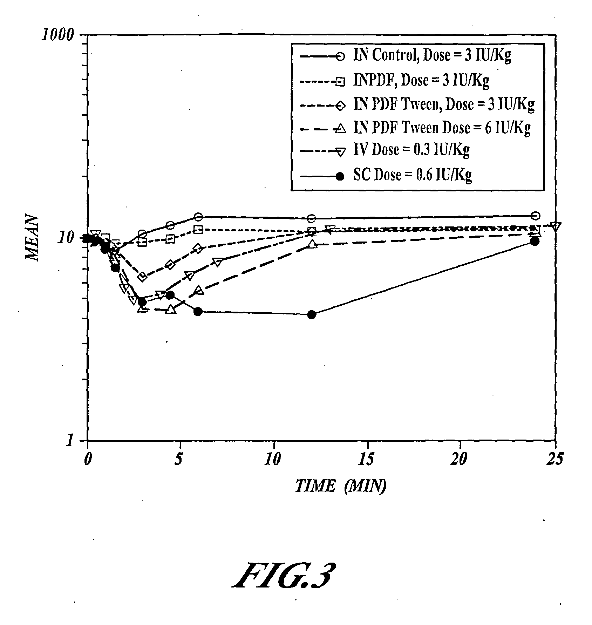

Pharmarmacodynamic (PD; i.e., glucose measurements) values were measured for insulin treated New Zealand White Rabbits at specified time-points up to 240 minutes. Glucose was measured at every time-point in duplicate with a Glucometer (One-Touch Ultra). The results of the PD Study 1 (test groups are shown in Example 8, Table 12 above) are shown in Table 16 and Figure 3. All data calculations were dose normalized, % BA was based on nominal assay values of test article.

Table 16:

| PD Study 1 Results | | | | | |
|---|---|---|---|---|---|
| Formulation (Group) | Dose (IU/kg) | $T_{min}$ (min) | $C_{min}$(%) | AUC (% glucose*min) | % BA Glucose |
| IN/1X PDF (1) | 3 | 15 | 93.5 | 420.79 | 1.3 |
| IN/1X PDF-Tween (2) | 3 | 30 | 64.5 | 2777.92 | 8.3 |
| IN/1X PDF-Tween (3) | 6 | 45 | 43.6 | 5205.66 | 7.8 |
| IN-Control (4) | 3 | 15 | 87 | 197.15 | 0.6 |
| SC-Regular (5) | 0.6 | 120 | 41.4 | 6706.55 | 100.0 |
| IV-Infusion (6) | 0.3 | 25 | 49.6 | 3179.51 | 94.8 |

In Study 1, the % $C_{min}$ for IN/PDF-Tween (#2 and #3), SC (#5), and IV (#6) insulin formulations was about 40%. $T_{min}$ was faster for IN/PDF-Tween (30-45 min) than SC (120 min). The % BA Glucose was ~ 8 % for IN PDF with 1% Tween (relative to SC).

A second PD study was conducted to compare intranasal PDF formulations with the NovoLog rapid-acting formulation (NovoLog diluent consists of: 16 mg/mL glycerin, 1.5 mg/mL phenol, 1.72 mg/mL m-cresol, 19.6 µg/mL zinc, 1.25 mg/mL disodium hydrogen phosphate dihydrate, and 0.58 mg/mL NaCl, pH 7.2-7.6). The parameters of PD Study 2 are shown above in Example 8, Table 14. PD Study 2 $C_{min}$ and $T_{min}$ results are shown in Table 17.

Table 17:

| $C_{min}$ and $T_{min}$ Results for PD Study 2 | | |
|---|---|---|
| Formulation (Group) | $T_{min}$ (min) | % $C_{min}$ |
| IN/1X PDF 1% Tween | 45 | 65.9 |
| IN/1X PDF 1% Tween* | 45 | 43.6 |
| IN/1X PDF (no DDPC) | 45 | 66.9 |
| IN/1X PDF 2% Tween | 45 | 57.1 |
| IN/1X PDF 5% Tween | 30 | 71 |

(continued)

| $C_{min}$ and $T_{min}$ Results for PD Study 2 | | |
|---|---|---|
| Formulation (Group) | $T_{min}$ (min) | % $C_{min}$ |
| TN/2X PDF 1% Tween | 30 | 50.3 |
| IN 2X PDF 2% Tween | 45 | 71.6 |
| SC-Regular* | 41.4 | 120 |
| SC-PDF | 49.5 | 30 |
| SC NovoLog | 34.8 | 120 |
| *Results from PD Study 1 | | |

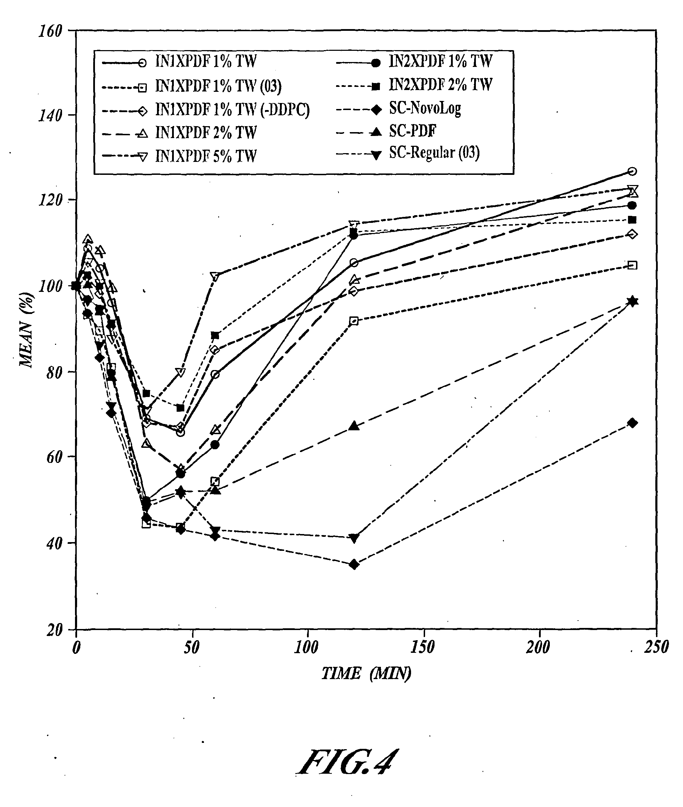

In Figures 4, the PD results for Study 2 are compared to Study 1. $T_{min}$ for IN/1X PDF 5% Tween, IN/2X PDF 1% Tween, SC NovoLog was about 30 min. $T_{min}$ for SC-Regular* was about 40 min. The $T_{min}$ for the other formulation was about 45 minutes. Results of PD Study 2 showed that 2X PDF 1% Tween had the greatest effect on PD of all the intranasal formulations. Presence of DDPC in the formulation did not affect the PD results.

Intranasal irritation was absent or silent in the rabbits for IN administration. The described PD data are supportive of intranasal formulations delivering insulin for a rapid-acting profile. The best performing formulations contained a solubolizing agent and surface active agent. A description of IN insulin formulations for further in vivo administration is shown in Table 18.

Table 18:

| Formulations for *In Vivo* Studies | | | | |
|---|---|---|---|---|
| **Formulation Number** | 094-1-0 | 094-1-250 | 094-1-500 | 094-1-1000 |
| **Insulin (U/mL)** | 0 | 250 | 500 | 1000 |
| **Me-B-CD (mg/mL)** | 45 | 45 | 45 | 45 |
| **DDPC (mg/mL)** | 1 | 1 | 1 | 1 |
| **EDTA (mg/mL)** | 1 | 1 | 1 | 1 |
| **Tween 80 (mg/mL)** | 10 | 10 | 10 | 10 |
| **Arginine (mM)** | 10 | 10 | 10 | 10 |
| **Sodium Chloride (mg/mL)** | 4 | 4 | 4 | 4 |
| **Propylparaben Sodium (mg/mL)** | 0.17 | 0.17 | 0.17 | 0.17 |
| **Methylparaben Sodium (mg/mL)** | 0.33 | 0.33 | 0.33 | 0.33 |
| **Propylene Glycol (mg/mL)** | 1 | 1 | 1 | 1 |
| **pH** | 7 | 7 | 7 | 7 |

EXAMPLE 10

Preclinical Study 3: PK and PD Results Following Intravenous. Subcutaneous, and Intranasal Administration of Insulin in Rabbits

Table 19 shows the dosage groups in Study 3. The following abbreviations were used: PDF= 45 mg/mL Me-$\beta$-CD, 1 mg/mL DDPC, 1 mg/mL EDTA, 10 mM arginine pH 7.0 with NaCl added to achieve about 220 mOsm/kg; 2X PDF = 90 mg/mL Me-$\beta$-CD, 2 mg/mL DDPC, 2 mg/mL EDTA (other components remain same as in PDF); preservative (Pre), in this case was a combination of 10 mg/mL propylene glycol, 0.33 mg/mL methyl paraben, and 0.17 mg/mL propyl paraben. Polysorabte 80 (Tween) was added to various formulations at 1% or 2% (10 or 20 mg/mL) as indicated. Two SC groups were dosed, one with regular insulin in absence of enhancers, and one with regular insulin in presence of PDF.

Table 19:

| Description of Groups Dosed in Preclinical Study 3 | |
|---|---|
| Formulation | Dose (IU/kg) |
| 1XPDF 1% Tween - | 6 |
| 1XPDF 1% Tween (-DDPC) | 6 |
| 1XPDF 2% Tween | 6 |
| 1XPDF 2% Tween (-DDPC) | 6 |
| 1XPDF 1% Tween (-Pre) | 6 |
| 1XPDF 1% Tween (-PreDDPC) | 6 |
| SC-Regular PDF | 0.6 |
| SC-Regular Saline | 0.6 |

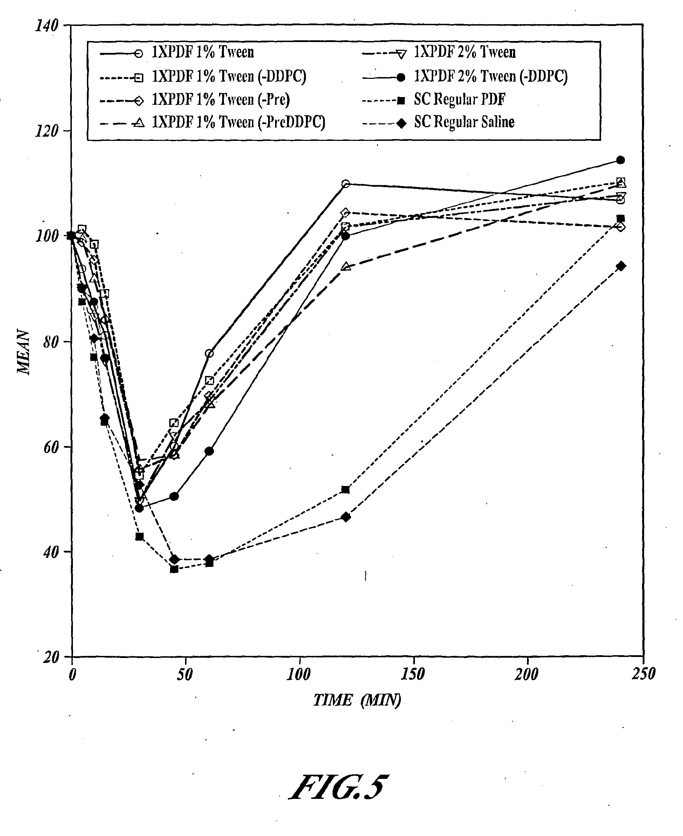

The PD data for the groups dosed in Preclinical Study 3 are shown in Table 20 and Figure 5.

Table 20:

| PD Data for Groups Dosed in Preclinical Study 3 | | | |
|---|---|---|---|
| Formulation | Dose (IU/kg) | $T_{min}$ | $\%C_{min}$ |
| 1XPDF 1 % Tween | 6 | 30 | 49.8 |
| 1XPDF 1% Tween (-DDPC) | 6 | 30 | 54.6 |
| 1XPDF 2% Tween | 6 | 30 | 49.5 |
| 1XPDF 2% Tween (-DDPC) | 6 | 30 | 48.4 |
| 1XPDF 1% Tween (-Pre) | 6 | 30 | 55.6 |
| 1XPDF 1 % Tween (-PreDDPC) | 6 | 3 0 | 57.3 |
| SC-Regular PDF | 0.6 | 45 | 36.4 |
| SC-Regular Saline | 0.6 | 60 | 38.4 |

All intranasal groups demonstrated about the same PD effect ($T_{min}$ and $\%C_{min}$). Subcutaneously delivered regular insulin in the absence and presence of PDF had similar PD effect (and a slower $T_{min}$ and greater $\%C_{min}$ as expected). The data showed that the onset (as indicated by $T_{min}$) is faster for regular insulin in the PDF formulations (45 min for SC; 30 min for intranasal) compared to the control formulation (60 min for SC). The data shows that the regular insulin in the intranasal PDF formulations is consistent with a rapid-acting insulin profile.

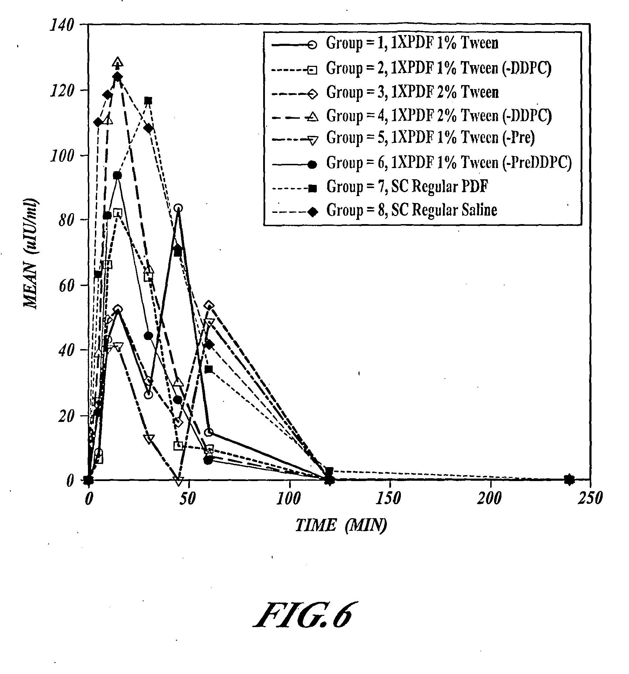

The PK data for the groups dosed in Preclinical Study 3 are shown in Figure 6 and Table 21, Table 22 and Table 23.

Table 21:

| PK Parameters for Groups Dosed in Preclinical Study 3 | | | | |
|---|---|---|---|---|
| Formulation | Group # | Tmax (min) | Cmax (uIU/mL) | AUClast (min*uIU/mL) |
| 1XPDF 1%Tween | 1 | 29.0 | 108.4 | 2504.2 |
| 1XPDF 1%Tween (-DDPC) | 2 | 16.3 | 95.7 | 2284.8 |
| 1XPDF 2%Tween | 3 | 36.3 | 88.1 | 2122.7 |
| 1XPDF 2%Tween (-DDPC) | 4 | 12.0 | 138.5 | 3387.4 |
| 1NPDF 1%Tween (-Pre) | 5 | 29.0 | 79.0 | 1174.5 |
| 1XPDF 1%Tween (-PreDDPC) | 6 | 13.0 | 94.7 | 2453.3 |
| SC Regular PDF | 7 | 19.0 | 129.7 | 5014.3 |

(continued)

| PK Parameters for Groups Dosed in Preclinical Study 3 | | | | |
|---|---|---|---|---|
| Formulation | Group # | Tmax (min) | Cmax (uIU/mL) | AUClast (min*uIU/mL) |
| SC Regular Saline . | 8 | 17.0 | 144.2 | 5885.5 |

Table 22:

| PK Data (bioavailability) for Groups Dosed in Preclinical Study 3 | | |
|---|---|---|
| Formulation | Group # | %F |
| 1XPDF 1% Tween | 1 | 4.3 |
| 1XPDF 1% Tween (-DDPC) | 2 | 3.9 |
| 1XPDF 2% Tween | 3 | 3.6 |
| 1XIPDF 2% Tween (-DDPC) | 4 | 5.8 |
| 1XPDF 1% Tween (-Pre) | 5 | 2.0 |
| 1XPDF 1% Tween (-PreDDPC) | 6 | 4.2 |
| SC Regular PDF | 7 | 85.2 |
| SC Regular Saline | 8 | NA |

Table 23:

| %CV for PK Parameters for Groups Dosed in Preclinical Study 3 | | | | |
|---|---|---|---|---|
| Formulation | Group # | Tmax (min) | Cmax (uIU/mL) | AUClast (min*uIU/mL) |
| 1XPDF 1%Tween | 1 | 56.4 | 84.2 | 84.7 |
| 1XPDF 1%Tween (-DDPC) | 2 | 58.2 | 90.8 | 124.5 |
| 1XPDF 2%Tween | 3 | 75.9 | 81.4 | 105.9 |
| 1XPDF 2%Tween (-DDPC) | 4 | 22.8 | 87.9 | 105.2 |
| 1XPDF 1%Tween (-Pre) | 5 | 97.8 | 54.4 | 95.8 |
| 1XPDF 1%Tween (-PreDDPC) | 6 | 34.4 | 68.2 | 72.7 |
| SC Regular PDF | 7 | 57.1 | 58.3 | 64.2 |
| SC Regular Saline | 8 | 73.8 | 28.7 | 62.5 |

The %CV for the various PK parameters was similar for the various groups. The %F (bioavailability relative to SC control) for PDF with Tween formulations compared to SC regular insulin control was about 2-6%, and $T_{max}$ was in the range of 12-36 minutes. The IN formulation with the highest % bioavailability was 1XPDF/2% Tween without DDPC (5.8%). These PD data suggest that DDPC is not necessary in the PDF formulation to achieve enhanced bioavailability.

EXAMPLE 11

Preclinical Study 4: PK and PD Results Following Oral and Intranasal Administration of Insulin in Rabbits

Table 24 describes the dosage groups in Study 4. The following abbreviations are used: PDF= 45 mg/mL Me-β-CD, 1 mg/mL DDPC, 1 mg/mL EDTA, 10 mM arginine pH 7.0 with NaCl added to achieve about 220 mOsm/kg; 2X PDF = 90 mg/mL Me-β-CD, 2 mg/mL DDPC, 2 mg/mL EDTA (other components remain same as in PDF); TDM = 2.5 mg/mL tetradecylmaltoside. Polysorbate 80 (Tween) was added to various formulations at 1% (10 mg/mL) as indicated. Proplyene glycol (PG) was added to various formulations at 1% or 2.5% (10 or 25 mg/ml). The effect of gelatin at 0.2% was tested on the IN formulations. Three oral groups were dosed, one with regular insulin in absence of enhancers (#8), one with regular insulin in presence of PDF (#9), and one with regular insulin in presence of PDF without DDPC (#7).

Table 24:

| Description of Groups Dosed in Preclinical Study 4 | | | |
|---|---|---|---|
| Group # | Formulation | Route | Dose Level (IU/kg) |
| 1 | IXPDF 1% Tween (-PG) | IN | 6 |
| 2 | 1XPDF 1% Tween (2.5%PG) | IN | 6 |
| 3 | TDMhypotonic | IN | 6 |
| 4 | TDMIsotonic | IN | 6 |
| 5 | 1XPDF 1% Tween (1%PG) | IN | 6 |
| 6 | 1XPDF 1% Tween (0.2% Gelatin) | IN | 6 |
| 7 | 1XPDF Oral(-DDPC+PG) | Oral | 6 |
| 8 | 1XPDF Oral (-DDPC-PG-Tween) | Oral | 6 |
| 9 | 1XPDF Oral (+DDPC+PG) | Oral | 6 |

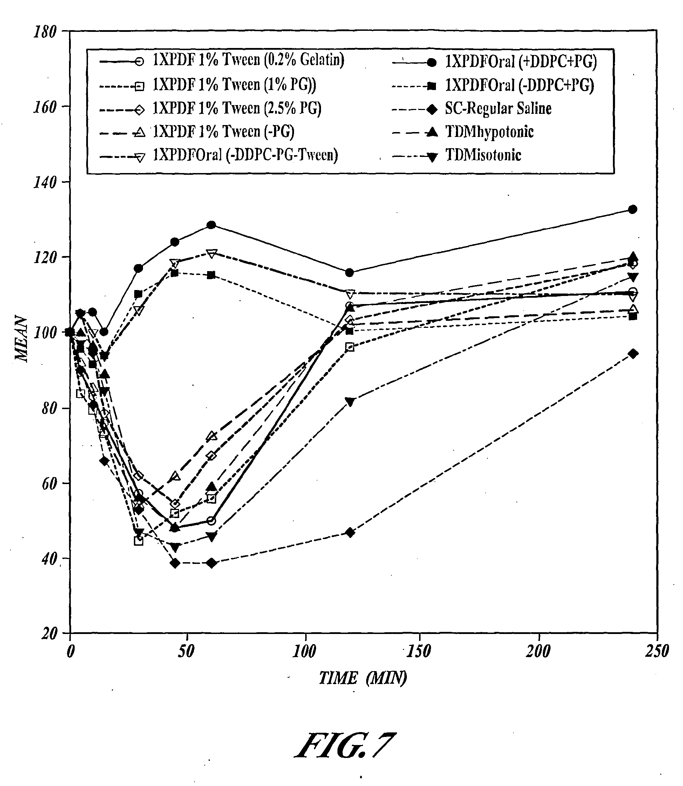

The PD data for the groups dosed in Preclinical Study 4 are shown in Figure 7. PD data was similar between all nasal formulations, but SC dosing had an extended PD effect versus nasal. No PD effect was observed for the oral dose groups. Subcutaneously delivered regular insulin in the absence and presence of PDF had a similar PD effect (and a slower $T_{min}$ and grater $\%C_{min}$ as expected). The data show that the onset (as indicated by $T_{min}$) is faster for regular insulin in the PDF formulations.

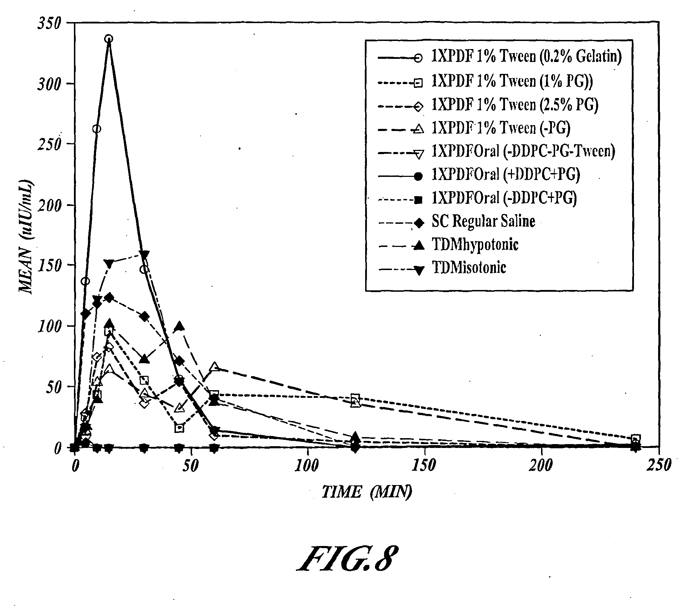

The PK data for the groups dosed in Preclinical Study 4 are presented in Figure 8 and Table 25, Table 26 and Table 27.

Table 25:

| PK Parameters for Groups Dosed in Preclinical Study 4 | | | | |
|---|---|---|---|---|
| Formulation | Tmax (min) | Cmax (uIU/mL) | AUClast (min *uIU/mL) | AUCinf (min*uIU/mL) |
| 1XPDF 1%Tween (-PG) | 59 | 125.06 | 5001.45 | 2565.5917 |
| 1XPDF 1%Tween (2.5%PG) | 18 | 95.2 | 3178 | 5192.0496 |
| TDMhypotonic | 33 | 206.58 | 3971 | 9828.6486 |
| TDMIsotonic | 23 | 179.52 | 5663 | 9788.9524 |
| 1XPDF 1%Tween (1%PG) | 34 | 108 | 6218 | 62759.0604 |
| 1XPDF 1%Tween (0.2% Gelatin) | 13 | 373.6 | 8755.5 | 9067.4665 |
| IXPDFOral (-DDPC+PG) | 5 | 24.56 | 111.9 | N/A |
| 1XPDFOral (-DDPC-PG-Tween) | 5 | 6.6 | 16.5 | N/A |
| 1XPDFOral (+DDPC+PG) | 5 | 3.08 | 64 | 408.0042 |
| SC Regular Insulin | 17 | 144.2 | 5885.5 | 3358.285 |

Table 26:

| PK Data (bioavailability) for Groups Dosed in Preclinical Study 4 | | |
|---|---|---|
| Formulation | AUClast (min*uIU/mL) | %F |
| 1XPDF 1%Tween (-PG) | 5001.45 | 8.5 |
| 1XPDF 1%Tween (2.5%PG) | 3178 | 5.4 |

(continued)

| PK Data (bioavailability) for Groups Dosed in Preclinical Study 4 | | |
|---|---|---|
| Formulation | AUClast (min*uIU/mL) | %F |
| TDMhypotonic | 3971 | 6.7 |
| TDMIsotonic | 5663 | 9.6 |
| 1XPDF 1%Tween(1%PG) | 6218 | 10.6 |
| 1XPDF 1%Tween (0.2% Gelatin) | 8755.5 | 14.9 |
| 1XPDFOral (-DDPC+PG) | 111.9 | 0.2 |
| 1XPDFOral (-DDPC-PG-Tween) | 16.5 | 0.0 |
| 1XPDFOral (+DDPC+PG) | 64 | 0.1 |
| SC Regular Insulin | 5885.5 | |

Table 27:

| %CV for PK Parameters for Groups Dosed in Preclinical Study 4 | | | |
|---|---|---|---|
| Formulation | Tmax (min) | Cmax ($\mu$IU/mL) | AUClast (min *$\mu$IU/mL) |
| 1XPDF 1% Tween(-PG) | 67.4 | 59.9 | 111.1 |
| 1XPDF1%Tween (2.5%PG) | 87.0 | 75.4 | 77.1 |
| TDMhypotonic | 59.3 | 41.3 | 56.6 |
| TDMIsotonic | 42.4 | 73.4 | 91.4 |
| 1XPDF 1%Tween (1%PG) | 142.0 | 51.7 | 95.9 |
| 1XPDF 1%Tween (0.2% Gelatin) | 34.4 | 21.3 | 35.3 |
| 1XPDFOral (-DDPC+PG) | 0.0 | 164.5 | 190.0 |
| 1XPDFOral (-DDPC-PG-Tween) | 0.0 | 199.2 | 199.2 |
| 1XPDFOral (+DDPC+PG) | 0.0 | 116.6 | 178.3 |
| SC Regular Insulin | 73.8 | 28.7 | 62.5 |

For the intranasal groups containing PDF with or without PG, as well as for the groups containing TDM, the PK data were similar, with a %F (bioavailability compared to SC regular insulin) at about 5.4-10.6% and $T_{max}$ in the range of 18-59 minutes. In the case of 1X PDF with 1% Tween in the presence of 0.2% gelatin, there was increased in bioavailability, approximately 14.9%. For the intranasal groups containing PDF with or without PG, as well as for the groups containing TDM, the %CV for $C_{max}$ and AUC were between 50 - 200 %. In contrast, for 1X PDF with 1% Tween in the presence of 0.2% gelatin, there was a decrease in $C_{max}$ and AUC to 21.3% and 35.3%, respectively. It was noted that the %CV for $C_{max}$ and AUC of the 1X PDF with 1% Tween in the presence of 0.2% gelatin formulation were lower than those observed for the SC injection.

These data show that the onset (as indicated by $T_{min}$) is faster for regular insulin in the PDF formulations than SC formulations, as a result the insulin has the profile of rapid-acting insulin. The addition of gelatin enhances the PD and PK (14.9% bioavailability relative to SC control) effect for PDF formulations.

EXAMPLE 12

PK and PD Results for Formulations Containing Viscosity Enhancing Agents

PK and PD were evaluated for rabbits dosed with intranasal insulin formulations containing different viscosity enhancing agents. Viscosity enhancing agents included gelatin, HPMC, MC, and Carbomer. Carbomer is a generic name for a family of polymers known as Carbopol®. Time points were taken at 5, 10, 15,30,45,60, 120, and 240 minutes. Glucose was measured at every time-point with a Glucometer (One-Touch Ultra). Small amounts of 2N HCl or NaOH were added to the formulation when necessary to achieve the desired pH. The insulin used in the study was at a concentration of approximately 28 U/mg. Table 28 shows the formulations used in this study.

Table 28:

| | Insulin Formulations Containing Viscosity Enhancing Agent | | | | | | | | | | |
|---|---|---|---|---|---|---|---|---|---|---|---|
| # | Regular Insulin (U/mL) | Me-β-CD (mg/ mL) | EDTA (mg/mL) | Tween 80 (mg/mL) | Arginine Buffer (mM) | Viscosity Agent | MP (mg/ML) | PP (mg/mL) | PG (mg/ mL) | NaCl (mg/ mL) | PH |
| 1 | 400 | 45 | 1 | 10 | 10 | 0 | 0.33 | 0.17 | 10 | 0 | 7.3 |
| 2 | 400 | 45 | 1 | 10 | 10 | Gelatin (2 mg/mL) | 0.33 | 0.17 | 10 | 0 | 7.3 |
| 3 | 400 | 45 | 1 | 10 | 10 | Gelatin (4 mg/mL) | 0.33 | 0.17 | 10 | 0 | 7.3 |
| 4 | 400 | 45 | 1 | 10 | 10 | (2.5mg/mL) | 0.33 | 0.17 | 10 | 0 | 7.3 |
| 5 | 400 | 45 | 1 | 10 | 10 | MC (2,5 mg/mL) | 0.33 | 0.17 | 10 | 0 | 7.3 |
| 6 | 400 | 45 | 1 | 10 | 10 | Carbomer (Carbopol) 974 P (2.5mg/mL) | 0.33 | 0.17 | 10 | 0 | 7.3 |
| 7 | 400 | 45 | 1 | 10 | 10 | (1mg/mL) | 0.33 | 0.17 | 10 | 0 | 7.3 |
| 8 | 400 | 45 | 1 | 10 | 10 | Gelatin (2 mg/mL) | 0.33 | 0.17 | 10 | 3 | 7.3 |
| **Abbreviations**: Me-β-CD=methyl-beta-cyclodextrin, EDTA=disodium edetate, HPMC = hydroxypropyl methylcellulose (100 cps), MC = methylcellulose (15 cps), CMC = carboxymethylcellulose sodium (low viscosity), <br> MP = methylparaben sodium, PP = propylparaben sodium, PG = propylene glycol, NaCl = sodium chloride | | | | | | | | | | | |

15 mL of each formulation was manufactured and stored in 3cc clear non-silanized glass vials. All the tested insulin formulations were stored at 2-8 °C. All formulations were dosed at 6.0 IU/kg. Table 29 describes the dosage groups used in this study.

Table 29:

| Viscosity Enhancing Agent Dosage Groups | | |
|---|---|---|
| Group # | Formulation | Dose IU/kg |
| 1 | 1XPDF 1%Tween | 6.0 |
| 2 | 1XPDF 1%Tween (0.2% Gelatin) | 6.0 |
| 3 | 1XPDF1%Tween (0.4% Gelatin) | 6.0 |
| 4 | 1XPDF1%Tween (0.25% HPMC) | 6.0 |
| 5 | 1XPDF 1%Tween (0.25%MC) | 6.0 |
| 6 | 1XPDF 1%Tween (0.25% Carbopol) | 6.0 |
| 7 | 1XPDF1%Tween (0.1% CMC) | 6.0 |
| 8 | 1XPDF 1%TW (0.2% Gelatin) | 6.0 |

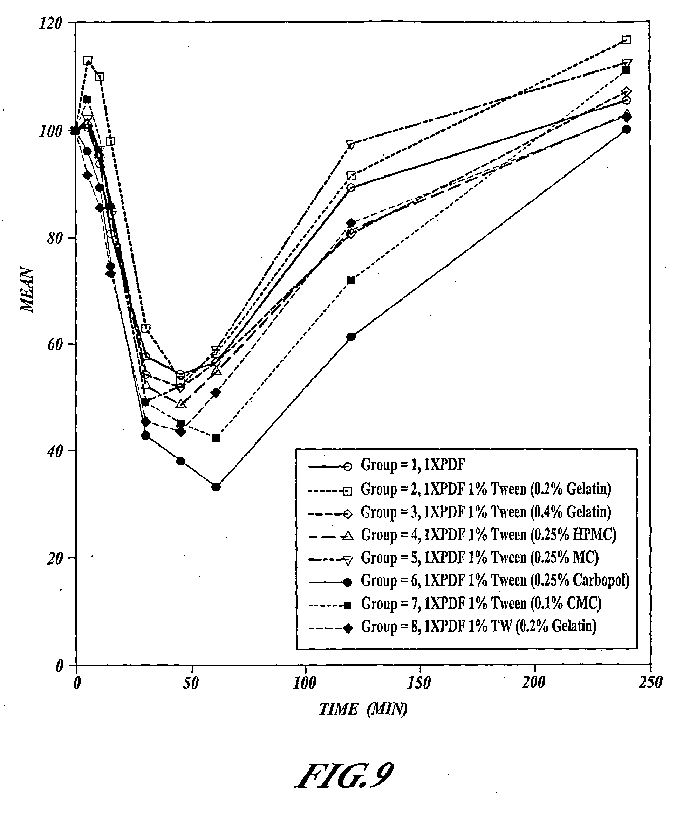

The PD results for % glucose from initial are shown in Figure 9. Figure 9 shows the mean change in % glucose over time for the 8 groups tested. Group 6 (1X PDF/1% Tween/(0.25% Carbopol)) showed the greatest reduction in % glucose from initial compared to all other groups. Glucose troughs for the 8 Groups occurred within 90 minutes as shown in Figure 9. Group 8 (which contained a tonicity agent) had the greatest reduction in % glucose from initial compared to the other gelatin formulations. The formulations containing Carbomer (0.25% Carbopol) and CMC had the greatest reduction in % glucose from initial compared to the other non-gelatin formulations.

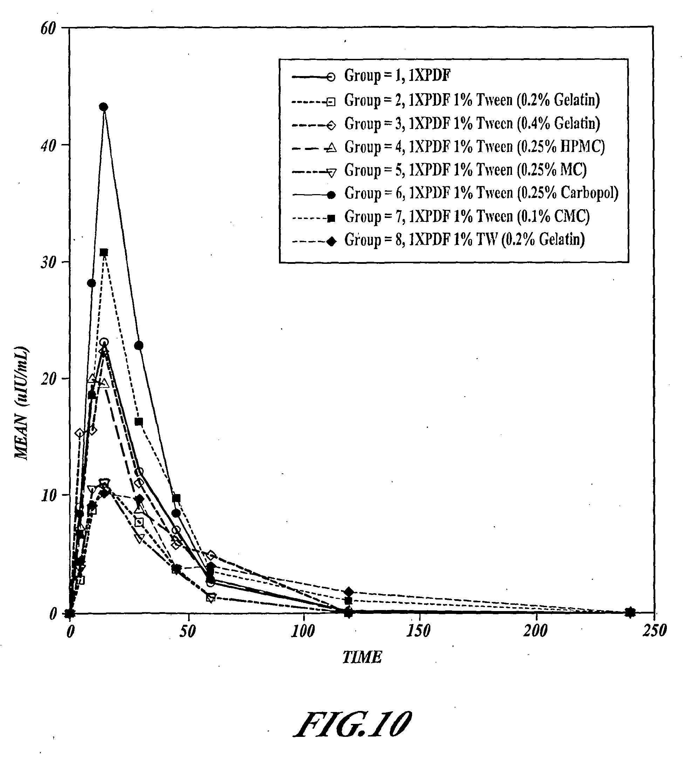

The PK results for mean data per timepoint are shown in Figure 10. In Figure 10, the mean concentration of insulin ($\mu$IU/mL) over time is shown for the 8 groups tested. Figure 10 shows that $C_{max}$ was greatest for Group 6, 1XPDP/1% Tween/(0.25% Carbopol) compared to the other formulations. Peak serum insulin levels for the 8 Groups occurred within 60 minutes as shown in Figure 10. The PK parameters are summarized in Table 30.

Table 30:

| Viscosity Enhancing Agent PK Parameters in Rabbits | | | | | |
|---|---|---|---|---|---|
| Group # | Formulation | Tmax(min) | Cmax($\mu$IU/mL) | AUClast(min*$\mu$IU/mL) | AUCinf(min*$\mu$IU/mL) |
| 1 | 1XPDF1%Tween | 13.00 | 243.68 | 7409.6 | 7546.2311 |
| 2 | 1XPDF1%Tween (0.2%Gelatin) | 18.00 | 119.28 | 3487.6 | 3756.8904 |
| 3 | 1XPDF1%Tween (0.4%Gelatin) | 22.00 | 28064 | 6617.8 | 10094.2851 |
| 4 | 1XPDF1%Tween (0.25%HPMC | 37.00 | 21274 | 6570.05 | 81493682 |
| 5 | 1XPDF1%Tween (0.25%MC) | 14.00 | 114.16 | 3383.2 | 4536.5694 |
| 6 | 1XPDF1%Tween (0.25%Carbopol) | 15.00 | 460.48 | 11583.6 | 12107.2492 |
| 7 | 1XPDF1%Tween (0.1%CMC) | 24.00 | 320.2 | 10482.5 | 11361.0313 |
| 8 | 1XPDF1%TW (0.2%Gelatin) | 29.00 | 231.48 | 6497.95 | 12461.998 |

The % CV results are shown in Table 31.

Table 31:

| Viscosity Enhancing Agent % CV Results in Rabbits | | | | |
|---|---|---|---|---|
| Group # | Formulation | Tmax | Cmax | AUClast |
| 1 | 1XPDF 1%Tween | 21.1 | 68.4 | 73.2 |
| 2 | 1XPDF1%Tween(0.2%Gelatin) | 37.3 | 27.5 | 48.1 |
| 3 | 1XPDF 1%Tween (0.4% Gelatin) | 98.5 | 79.3 | 69.1 |
| 4 | 1XPDF 1%Tween (0.2%HPMC) | 127.3 | 74.7 | 84.0 |
| 5 | 1XPDF 1%Tween (0.25%MC) | 16.0 | 48.2 | 60.7 |
| 6 | 1XPDF 1%Tween (0.25% Carbopol) | 0.0 | 62.0 | 47.6 |
| 7 | 1XPDF 1%Tween (0.1% CMC) | 55.9 | 76.4 | 60.0 |
| 8 | 1XPDF 1%TW (0.2% Gelatin) | 76.5 | 95.0 | 76.1 |

The %F (Bioavailability) results are shown in Table 32.

Table 32:

| Viscosity Enhancing Agent %F Results in Rabbits | | | | |
|---|---|---|---|---|
| Group # | Formulation | Dose IU/kg | AUClast (nin*ulU/mL) | %F |
| 1 | 1XPDF 1%Tween | 6.0 | 7409.6 | 12.6 |
| 2 | 1XPDF1%Tween (0.2%Gelatin) | 6.0 | 3487.6 | 5.9 |
| 3 | 1XPDF 1%Tween (0.4%Gelatin) | 6.0 | 6617.8 | 11.2 |
| 4 | 1XPDF1%Tween(0.25%HPMC) | 6.0 | 6570.05 | 11.2 |
| 5 | 1XPDF 1%Tween (0.25%MC) | 6.0 | 3383.2 | 5.7 |
| 6 | 1XPDF 1%Tween (0.25%Carbopol) | 6.0 | 11583.6 | 19.7 |
| 7 | 1XPDF 1%Tween(0.1%CMC) | 6.0 | 10482.5 | 17.8 |
| 8 | 1XPDF 1%TW(0.2%Gelatin) | 6.0 | 6497.95 | 11.0 |
| | SCRegular Insulin | 0.6 | 5885.5 | |

<u>Summary</u>

The PK and PD results show that the intranasal insulin formulations tested had rapid acting insulin profiles, with peak serum insulin levels within 60 minutes and glucose troughs within 90 minutes. Bioavailability was increased when viscosity enhancers were added to PDF intranasal insulin formulations. Increased tonicity increased bioavailability in formulations containing gelatin. The formulation containing gelatin showed improved performance with isotonic conditions (Group #8; 0.2% Gelatin including NaCl) compared to hypotonic conditions (Group #2; 0.2% Gelatin without NaCl). The formulations containing Carbomer and CMC showed the greatest increase in PK and PD results for intranasal insulin formulations. Bioavailability as shown by %F was 19.7% and 17.8% for Carbomer and CMC, respectively. The PD effect as shown by % glucose from initial was improved with the addition of viscosity enhancing agents, such as Carbomer and CMC, to the intranasal insulin formulations.

PK and PD data in the rabbit confirm *in vitro* results of an increase in insulin permeation across the nasal epithelium in the presence of formulation enhancers. The *in vitro* drug permeation data and *in vivo* PK Rabbit data showed a substantial correlation for intranasal insulin formulations. Using representative intranasal formulations, a XY plot analysis with AUClast (min*$\mu$U/mL) on the X-axis and % permeation on the Y-axis showed a $R^2$ = 0.8994, y = 0.0007x + 0.4191.

EXAMPLE 13

AET Studies 1-8: Antimicrobial Effectiveness Testing (AET)

AET Studyl

AET Study 1 was conducted to determine the Antimicrobial Effectiveness (AET) of an insulin nasal spray placebo with methylparaben sodium, propylparaben sodium, and propylene glycol. Additionally, AET Study 1 examined the AET of increased EDTA alone. The formulations evaluated in AET Study 1 are shown in Table 33. Approximately 120 mL of each formulation was manufactured and tested in duplicate (n=2 analyses per sample).

Table 33

| Formulations evaluated in AET Study 1 | | | | | | | | | | |
|---|---|---|---|---|---|---|---|---|---|---|
| # | Me-β-CD | DDPC | EDTA | Tween 80 | PP | MP | PP | NaCl | Arginine Buffer | pH |
| | (mg/mL) | | | | | | | | (mM) | |
| 1 | 45 | 1 | 1 | 10 | 25 | 0.33 | 0.17 | 4 | 10 | 7 |
| 2 | 45 | 1 | 1 | 10 | 50 | 0.33 | 0.17 | 4 | 10 | 7 |
| 3 | 45 | 1 | 1 | 10 | 100 | 0.33 | 0.17 | 4 | 10 | 7 |
| 4 | 45 | 1 | 10 | 10 | 0 | 0 | 0 | 4 | 10 | 7 |
| 5 | 45 | 1 | 50 | 10 | 0 | 0 | 0 | 4 | 10 | 7 |
| Abbreviations: Me-β-CD = methyl β cyclodextrin, DDPC = L α phosphatidylcholine didecanoyl, EDTA = edetate disodium, MP = methylparaben sodium, PP = propylparaben sodium, PG = propylene glycol, NaCl = sodium chloride | | | | | | | | | | |

The AET methods used were in compliance with the requirements for U.S. Pharmacopeial (USP) and European Pharmacopeial (EP) AET and are described in Tables 34 and 35, respectively. The formulations were also tested for pH (per SOP 403), appearance (visual), and osmolality (per SOP 4000).

Table 34

| USP AET Requirements (USP <51>) | | | | | | | | | | |
|---|---|---|---|---|---|---|---|---|---|---|
| Microorganism | Psudomoas aeruginosa | | Escherichia coli | | Staphylococcus aureus | | Candida albicans | | Aspergillus niger | |
| Days | 14 | 28 | 14 | 28 | 14 | 28 | 14 | 28 | 14 | 28 |
| Log Reduction (Min) | 2.0 | no inc. | 2.0 no | inc. | 2.0 | no inc. | no inc. | no inc. | no inc. | no inc. |

Table 35

| EP AET Requirements (EP <5.1.3>) | | | | | | | | | |
|---|---|---|---|---|---|---|---|---|---|
| Microorganism | Psudomoas aeruginosa | | | Staphylococcus aureus | | | Candida albicans | | Aspergillus niger | |
| Days | 2 | 7 | 28 | 2 | 7 | 28 | 14 | 28 | 14 | 28 |
| Log Reduction (Min) | 2.0 | 3.0 | no inc. | 2.0 | 3.0 | no inc. | 2.0 | no inc. | 2.0 | no inc. |

The combination of 0.33 mg/mL of methylparaben sodium, 0.17 mg/mL of propylparaben sodium, and at least 25 mg/mL propylene glycol was an effective preservative combination and complies with USP standards. These formulations passed all of the USP requirements, but failed EP (for S. aureus and A. niger). Increased EDTA alone did not appear to be effective for either USP or EP requirements.

AET Study 2

AET Study 2 was conducted to determine whether a humectant (propylene glycol) improved antimicrobial effectiveness when methylparaben sodium and propylparaben sodium were used as preservatives. Other preservatives, such as benzakonium chloride (BAK), benzyl alcohol, and sodium benzoate were also evaluated. Two insulin groups at the levels of 500 U/mL or 1000 U/mL were tested. The formulations evaluated in AET Study 2 are listed in Table 36.

AET Study 2

Table 36

| Formulations Evaluated in AET Study 2 | | | | | | | | | | | | |
|---|---|---|---|---|---|---|---|---|---|---|---|---|
| # | Me-β-CD | DDPC | EDTA | Tween 80 | NaCl | MP | PP | Benzyl Alcohol | BAK | Socium Benzoate | Arginine Buffer | pH |
| | (mg/mL) | | | | | | | | | (mM) | | |
| 1 | 45 | 1 | 1 | 10 | 4 | 0.33 | 0.17 | 0 | 0 | 0 | 10 | 7 |
| 2 | 45 | 1 | 1 | 10 | 4 | 0.33 | 0.17 | 5 | 0 | 0 | 10 | 7 |
| 3 | 45 | 1 | 1 | 10 | 4 | 0 | 0 | 5 | 0 | 0 | 10 | 7 |
| 4 | 45 | 1 | 1 | 10 | 4 | 0 | 0 | 0 | 2 | 0 | 10 | 7 |
| 5 | 45 | 1 | 1 | 10 | 4 | 0 | 0 | 0 | 2 | 0 | 10 | 7 |
| Samples 5 also contains 500 units/mL insulin | | | | | | | | | | | | |
| 6 | 45 | 1 | 1 | 10 | 4 | 0 | 0 | 0 | 2 | 0 | 10 | 7 |
| Samples 6 also contains 1000 units/mL insulin | | | | | | | | | | | | |
| 7 | 45 | 1 | 1 | 10 | 4 | 0 | 0 | 0 | 1 | 0 | 10 | 7 |
| 8 | 45 | 1 | 1 | 10 | 4 | 0 | 0 | 0 | 0 | 1 | 10 | 7 |
| 9 | 45 | 1 | 1 | 10 | 4 | 0 | 0 | 0 | 0 | 5 | 10 | 7 |
| 10 | PBS (No Preservative Control) | | | | | | | | | | | |
| 11 | PBS + 5 mg/mL Sodium Benzoate (Preservative Positive Control) | | | | | | | | | | | |
| Abbreviations: Me-β-CD = methyl β cyclodextrin<br>DDPC = L α phosphatidylcholine didecanoyl<br>EDTA = edetate disodium<br>MP = methylparaben sodium<br>PP = propylparaben sodium<br>BAK = benzalkonium chloride<br>NaCl = sodium chloride | | | | | | | | | | | | |

The methods for AET Study 2 were conducted as described for AET Study 1. Additionally, a positive control (PBS with 5 mg/mL sodium benzoate) and a negative control (PBS alone) were included. The results of AET Study 2 showed that methylparaben sodium and propylparaben sodium were not effective preservatives without humectant (such as propylene glycol) included in the formulation. Benzalkonium chloride was an excellent preservative with respect to both USP and EP Antimicrobial Effectiveness Testing, but was found to be incompatible with insulin (i.e., its presence within the formulation caused a precipitation of the insulin). Benzyl alcohol and sodium benzoate were also not effective preservatives at neutral pH and therefore were not appropriate for use within Insulin Nasal Spray formulations.

AET Study 3

The purpose of AET Study 3 was to evaluate other preservatives, such as benzakonium chloride (BAK), benzyl alcohol, and sodium benzoate. Two groups with insulin at the levels of 500 U/mL or 1000 U/mL were tested. The formulations for AET Study 3 are listed in Table 37.

Table 37

| Formulations Evaluated in AET Study 3 | | | | | | | | | | | | |
|---|---|---|---|---|---|---|---|---|---|---|---|---|
| # | Me-β-CD (mg/mL) | DDPC (mg/mL) | EDTA (mg/mL) | Tween 80 (mg/mL) | Arginine Buffer (mM) | MP (mg/mL) | PP (mg/mL) | Benzyl Alcohol (mg/mL) | BAK (mg/mL) | Sodium Benzoate (mg/mL) | NaCl (mg/mL) | pH |
| 1 | 45 | 0 | 1 | 10 | 10 | 0.33 | 0.17 | 5 | 0 | 0 | 4 | 7 |
| 2 | 45 | 0 | 1 | 10 | 10 | 0 | 0 | 5 | 0 | 0 | 4 | 7 |
| 3 | 45 | 0 | 1 | 10 | 10 | 0 | 0 | 0 | 2 | 0 | 4 | 7 |
| 4 | 45 | 0 | 1 | 10 | 10 | 0 | 0 | 0 | 1.5 | 0 | 4 | 7 |
| 5 | 45 | 0 | 1 | 10 | 10 | 0 | 0 | 0 | 1.5 | 0 | 4 | 7 |
| Samples 5 also contains 500 units/mL insulin | | | | | | | | | | | | |
| 6 | 45 | 0 | 1 | 10 | 10 | 0 | 0 | 0 | 1.5 | 0 | 4 | 7 |
| Samples 6 also contains 1000 units/mL insulin | | | | | | | | | | | | |
| 7 | 45 | 0 | 1 | 10 | 10 | 0 | 0 | 0 | 1 | 0 | 4 | 7 |
| Samples 7 also contains 1000 units/mL insulin | | | | | | | | | | | | |
| 8 | PBS (No Preservative Control) | | | | | | | | | | | |
| 9 | PBS + 5 mg/mL Sodium Benzoate (Preservative Positive Control) | | | | | | | | | | | |
| Abbreviations: Me-β-CD = methyl β cyclodextrin<br>DDPC = L α phosphatidylcholine didecanoyl<br>EDTA = edetate disodium<br>MP = methylparaben sodium<br>PP = propylparaben sodium<br>BAK = benzalkonium chloride<br>NaCl = sodium chloride | | | | | | | | | | | | |

The analysis in AET Study 3 was conducted as described for AET Study 1. AET Study 3 results showed benzakonium chloride was incompatible with insulin (caused precipitation), but maintained the best antimicrobial performance. Benzyl alcohol and benzyl alcohol/methylparaben sodium/propylparaben sodium were ineffective as antimicrobial reagents in this study.

AET Study 4

The purpose of AET Study 4 was to determine whether satisfactory USP and EP AET results could be achieved with lower levels of humectant (propylene glycol) when used with methylparaben sodium and propylparaben sodium.

Additionally, alternative preservatives m-cresol and benzyl alcohol were evaluated. AET Study 4 formulations are listed in Table 38.

Table 38

| # | Me-β-CD (mg/ML) | EDTA (mg/ mL) | Tween 80 (mg/mL) | Arginine (mM) | MP/PP (mg/mL) (mg) | PG | Benzyl Alcohol (mg/mL) | m-Cresol (mg/mL) | pH |
|---|---|---|---|---|---|---|---|---|---|
| | | | | | Formulations Evaluated in AET Study 4 | | | | |
| 1 | 45 | 1 | 10 | 10 | 0.33/0.17 | 1 | 0 | 0 | 7.0 |
| 2 | 45 | 1 | 10 | 10 | 0 | 0 | 2.5 | 0 | 7.0 |
| 3 | 45 | 1 | 10 | 30 | 0 | 0 | 5 | 0 | 7.0 |
| 4 | 45 | 1 | 10 | 10 | 0 | 0 | 0 | 1 | 7.0 |
| 5 | 45 | 1 | 10 | 10 | 0 | 0 | 0 | 1 | 7.0 |
| 6 | Negative Control- PBS Alone | | | | | | | | |
| 7 | Positive Control - PBS with 5 mg/mL Sodium Benzoate | | | | | | | | |

Abbreviations: Me-β-CD = methyl β cyclodextrin
EDTA = edetate disodium
MP = methylparaben sodium
PP = propylparaben sodium
PG = propylene glycol

The methods used for AET Study 4 were conducted as described for AET Study 1. The formulations that contain methylparaben sodium and propylparaben sodium with a low concentration of humectant (i.e., propylene glycol) do not achieve antimicrobial effectiveness. The optimal level for methylparaben sodium and propylparaben sodium with a propylene glycol was between 1 and 25 mg/mL propylene glycol. Additionally, benzyl alcohol and m-cresol were not effective preservatives for insulin nasal spray formulations.

AET Study 5

The purpose of AET Study 5 was to conduct antimicrobial effectiveness testing (AET) of insulin spray formulations (placebo and active) to determine whether methylparaben sodium and propylparaben sodium are both required for optimal preservative effectiveness and whether one' is more effective than the other. Additionally, increased levels of methylparaben sodium and propylparaben sodium were evaluated to determine whether increasing their content within the formulation increased antimicrobial effectiveness. The formulations used in AET Study 5 are listed in Table 39.

Table 39

| # | Insulin (U/mL) | Me-β-CD (mg/mL) | DDPC (mg/mL) | EDTA (mg/mL) | Tween 80 (mg/mL) | Arginine (mM) | MP (mg/mL) | PP (mg/mL) | PG (mg/mL) | NaCl (mg/mL) | pH |
|---|---|---|---|---|---|---|---|---|---|---|---|
| | | | | | Formulations evaluated in AET Study 5 | | | | | | |
| 1 | 0 | 45 | 1 | 1 | 10 | 10 | 0.33 | 0 | 0 | 2 | 7.0 |
| 2 | 0 | 45 | 1 | 1 | 10 | 10 | 0 | 0.17 | 0 | 2 | 7.0 |
| 3 | 0 | 45 | 1 | 1 | 10 | 10 | 0.33 | 0 | 1 | 2 | 7.0 |
| 4 | 0 | 45 | 1 | 1 | 10 | 10 | 0 | 0.17 | 1 | 2 | 7.0 |
| 5 | 0 | 45 | 1 | 1 | 10 | 10 | 0 | 0 | 1 | 2 | 7.0 |
| 6 | 0 | 45 | 1 | 1 | 10 | 10 | 0.33 | 0.17 | 0 | 2 | 7.0 |
| 7 | 500 | 45 | 1 | 1 | 10 | 10 | 0.33 | 0.17 | 1 | 2 | 7.0 |

(continued)

| # | Insulin (U/mL) | Me-β-CD (mg/mL) | DDPC (mg/mL) | EDTA (mg/mL) | Tween 80 (mg/mL) | Arginine (mM) | MP (mg/mL) | PP (mg/mL) | PG (mg/mL) | NaCl (mg/mL) | pH |
|---|---|---|---|---|---|---|---|---|---|---|---|
| | | | | | Formulations evaluated in AET Study 5 | | | | | | |
| 8 | 500 | 45 | 1 | 1 | 10 | 10 | 3.33 | 1.7 | 1 | 2 | 7.0 |
| 9 | 500 | 45 | 1 | 1 | 10 | 10 | 7 | 3 | 1 | 2 | 7.0 |
| 10 | Negative Control-PBS Alone | | | | | | | | | | |
| 11 | Positive Control - PBS with 5 mg/mL Benzalkonium Chloride | | | | | | | | | | |

Abbreviations: Me-β-CD = methyl β cyclodextrin
DDPC = L α phosphatidylcholine didecanoyl
EDTA = edetate disodium
MP = methylparaben sodium
PP = propylparaben sodium
PG = propylene glycol
NaCl = sodium chloride

The methods used in AET Study 5 were conducted as described for AET Study 1. The results of AET Study 5 showed that increasing methylparaben sodium and propylparaben sodium levels to at least ten-fold of 0.33 mg/mL methylparaben sodium and 0.17 mg/mL propylparapben sodium increases antimicrobial effectiveness. Additionally, it was evident that 0.33 mg/mL methylparaben sodium alone had the same antimicrobial effectiveness as 0.17 mg/mL propylparaben sodium alone, which also had the same antimicrobial effectiveness of the combination.

AET Study 6

The purpose of AET Study 6 was to conduct antimicrobial effectiveness testing (AET) of insulin spray formulations (placebo) to determine the optimal level of propylene glycol needed for use with methylparaben sodium and propylparaben sodium. Additionally, increased levels of methylparaben sodium and propylparaben sodium were evaluated with a static level of propylene glycol to determine whether increasing their content within the formulation increased antimicrobial effectiveness. Finally, ethanol was also be evaluated as a potential preservative. The formulations evaluated in AET Study 6 are listed in Table 40.

Table 40

| | | | | | | | Formulations Evaluated in AET Study 6 | | | | | |
|---|---|---|---|---|---|---|---|---|---|---|---|---|
| # | Insulin (U/ mL) | Me-β-CD (mg/ | DDPC (mg/mL) | EDTA (mg/mL) | Tween 80 (mg/mL) | Arginine (mM) | EtOH (%) | MP (mg/mL) | PP (mg/ mL) | PG (mg/ mL) | NaCl (mg/ mL) | pH |
| 1 | 0 | 45 | 1 | 1 | 10 | 10 | 0 | 0.33 | 0.17 | 25 | 0 | 7.0 |
| 2 | 0 | 45 | 1 | 1 | 10 | 10 | 0 | 0 | 0 | 25 | 0 | 7.0 |
| 3 | 0 | 45 | 1 | 1 | 10 | 10 | 0 | 0.33 | 0.17 | 15 | 0 | 7.0 |
| 4 | 0 | 45 | 1 | 1 | 10 | 10 | 0 | 0.33 | 0.17 | 10 | 0 | 7.0 |
| 5 | 0 | 45 | 1 | 1 | 10 | 10 | 0 | 0.33 | 0.17 | 5 | 2 | 7.0 |
| 6 | 0 | 45 | 1 | 1 | 10 | 10 | 0 | 0.33 | 0.17 | 2.5 | 3 | 7.0 |
| 7 | 0 | 45 | 1 | 1 | 10 | 10 | 0 | 0.33 | 0.17 | 1 | 4 | 7.0 |
| 8 | 0 | 45 | 1 | 1 | 10 | 10 | 0 | 0.495 | 0.255 | 5 | 2 | 7.0 |
| 9 | 0 | 45 | 1 | 1 | 10 | 10 | 0 | 0.66 | 0.34 | 5 | 2 | 7.0 |
| 10 | 0 | 45 | 1 | 1 | 10 | 10 | 0 | 1.65 | 0.85 | 5 | 2 | 7.0 |
| 11 | 0 | 45 | 1 | 1 | 10 | 10 | 1 | 0 | 0 | 0 | 0 | 7.0 |
| 12 | 0 | 45 | 1 | 1 | 10 | 10 | 2 | 0 | 0 | 0 | 0 | 7.0 |
| 13 | Negative Control - PBS Alone | | | | | | | | | | | |
| 14 | Positive Control - PBS with 5 mg/mL Benzalkonium Chloride | | | | | | | | | | | |

Abbreviations: Me-β-CD = methyl β cyclodextrin
DDPC = L α phosphatidylcholine didecanoyl
EDTA = edetate disodium
EtOH = ethanol
MP = methylparaben sodium
PP = propylparaben sodium
PG = propylene glycol
NaCl = sodium chloride

The methods for AET Study 6 were conducted as described for AET Study 1. The results of AET Study 6 show that the optimal level of propylene glycol was 10 mg/mL. The AET results of Insulin Nasal Spray Formulations with 10, 15, 20, and 25 mg/mL propylene glycol were very similar; but the AET results were less successful when the propylene glycol level was less than 10 mg/mL. All of the formulations passed the USP AET requirements except for the P. auerignosa requirement. With respect to this category, the formulations were bacteriostatic (i.e., there was no indication of microbial growth). All formulations failed the EP requirements for the earliest time points for each required organism. Ethanol alone (at 1% or 2%) appeared to have antimicribal activity similar to that of methylparaben sodium/propylparaben sodium/propylene glycol.

AET Study 7

The purpose of AET Study 7 was to conduct antimicrobial effectiveness testing (AET) of insulin spray formulations (placebo) that contain 20 mg/mL Tween 80. One in vivo pharmacokinetic study demonstrated that increasing Tween 80 content to 20 mg/mL may help increase bioavailability, but Tween 80 micelles are also known to interact with preservatives (specifically with the parabens). In addition, AET Study 7 was conducted to determine the optimal level of propylene glycol needed for use with methylparaben sodium and propylparaben sodium. The increased levels of methylparaben sodium and propylparaben sodium were evaluated with a static level of propylene glycol to determine whether increasing their content within the formulation increases antimicrobial effectiveness. Finally, ethanol was also evaluated as a potential preservative. The formulations evaluated in AET Study 7 are listed in Table 41.

Table 41

| | | | | | | | | | | | | |
|---|---|---|---|---|---|---|---|---|---|---|---|---|
| | Formulations Evaluated in AET Study 7 |
| # | Insulin (U/mL) | Me-β-CD (mg/mL) | DDPC (mg/mL) | EDTA (mg/mL) | Tween 80 (mg/mL) | Arginine (mM) | EtOH (%) | MP (mg/mL) | PP (mg/mL) | PG (mg/mL) | NaCl (mg/mL) | pH |
| 1 | 0 | 45 | 1 | 1 | 20 | 10 | 0 | 0.33 | 0.17 | 25 | 0 | 7.0 |
| 2 | 0 | 45 | 1 | 1 | 20 | 10 | 0 | 0 | 0 | 25 | 0 | 7.0 |
| 3 | 0 | 45 | 1 | 1 | 20 | 10 | 0 | 0.33 | 0.17 | 15 | 0 | 7.0 |
| 4 | 0 | 45 | 1 | 1 | 20 | 10 | 0 | 0.33 | 0.17 | 10 | 0 | 7.0 |
| 5 | 0 | 45 | 1 | 1 | 20 | 10 | 0 | 0.33 | 0.17 | 5 | 2 | 7.0 |
| 6 | 0 | 45 | 1 | 1 | 20 | 10 | 0 | 0.33 | 0.17 | 2.5 | 3 | 7.0 |
| 7 | 0 | 45 | 1 | 1 | 20 | 10 | 0 | 0.33 | 0.17 | 1 | 4 | 7.0 |
| 8 | 0 | 45 | 1 | 1 | 20 | 10 | 0 | 0.495 | 0.255 | 5 | 2 | 7.0 |
| 9 | 0 | 45 | 1 | 1 | 20 | 10 | 0 | 0.66 | 0.34 | 5 | 2 | 7.0 |
| 10 | 0 | 45 | 1 | 1 | 20 | 10 | 0 | 1.65 | 0.85 | 5 | 2 | 7.0 |
| 11 | 0 | 45 | 1 | 1 | 20 | 10 | 1 | 0 | 0 | 0 | 0 | 7.0 |
| 12 | 0 | 45 | 1 | 1 | 20 | 10 | 2 | 0 | 0 | 0 | 0 | 7.0 |
| 13 | colspan="12" | Negative Control-PBS Alone |
| 14 | colspan="12" | Positive Control - PBS with 5 mg/mL Benzalkonium Chloride |

Abbreviations: Me-β-CD = methyl β cyclodextrin
DDPC = L α phosphatidylcholine didecanoyl
EDTA = edetate disodium
EtOH = ethanol
MP = methylparaben sodium
PP = propylparaben sodium
PG = propylene glycol
NaCl = sodium chloride

The methods for AET Study 7 were conducted as described for AET Study 1. The results from AET Study 7 showed that increasing the Tween 80 content from 10 mg/mL to 20 mg/mL reduced antimicrobial activity, even when the highest level of propylene glycol (i.e., 25 mg/mL) was added. Ethanol was not an effective preservative (at 1 %) when used in combination with 20 mg/mL Tween 80.

AET Study 8

AET Study 8 was conducted to test the antimicrobial effectiveness testing (AET) of insulin spray formulations (active) with formulations containing propylene glycol levels at 1, 5, 10, and 25 mg/mL. Additionally, the three concentrations of insulin nasal spray were tested: 250, 500, and 1000 U/mL. The formulations evaluated in AET Study 8 are listed in Table 42.

Table 42

| | | | | | | | | | | | |
|---|---|---|---|---|---|---|---|---|---|---|---|
| | Formulations Evaluated in AET Study 8 |
| # | Insulin (U/mL) | Me-β-CD (mg/ mL) | DDPC (mg/mL) | EDTA (mg/mL) | Tween 80 (mg/mL) | Arginine (mM) | MP (mg/mL) | PP (mg/mL) | PG (mg/mL) | NaCl (mg/mL) | pH |
| 1 | 250 | 45 | 1 | 1 | 10 | 10 | 0.33 | 0.17 | 25 | 0 | 7.0 |
| 2 | 500 | 45 | 1 | 1 | 10 | 10 | 0.33 | 0.17 | 25 | 0 | 7.0 |
| 3 | 1000 | 45 | 1 | 1 | 10 | 10 | 0.33 | 0.17 | 25 | 0 | 7.0 |
| 4 | 250 | 45 | 1 | 1 | 10 | 10 | 0.33 | 0.17 | 1 | 4 | 7.0 |
| 5 | 500 | 45 | 1 | 1 | 10 | 10 | 0.33 | 0.17 | 1 | 4 | 7.0 |
| 6 | 1000 | 45 | 1 | 1 | 10 | 10 | 0.33 | 0.17 | 1 | 3.5 | 7.0 |
| 7 | 500 | 45 | 1 | 1 | 10 | 10 | 0.33 | 0.17 | 10 | 0 | 7.0 |
| 8 | 500 | 45 | 1 | 1 | 10 | 10 | 3.33 | 0.17 | 5 | 2 | 7.0 |
| 9 | colspan="11" | Negative Control - PBS Alone |
| 10 | colspan="11" | Positive Control - PBS with 5 mg/mL Benzalkonium Chloride |

Abbreviations: Me-β-CD = methyl β cyclodextrin
DDPC = L α phosphatidylcholine didecanoyl
EDTA = edetate disodium
MP = methylparaben sodium
PP =propylparaben sodium
PG = propylene glycol
NaCl = sodium chloride

The methods used for analysis are described for AET Study 1. The results of AET Study 8 showed that the addition of insulin to the formulations improved AET performance. Additionally, when the propylene glycol level was high (i.e., 25 mg/mL or 10 mg/mL), the insulin-containing formulations with methylparaben sodium and propylparaben sodium passed USP AET requirements. EP requirements, however, were not met.

AET Studies 1 through 8 Summary

The data from Studies 1-8 showed that with respect to AET the combination of the methylparaben sodium; propylparaben sodium, and the humectant propylene glycol resulted in increased preservative effectiveness compared to methylparaben sodium and propylparaben sodium alone. Additionally, several other preservatives were evaluated over the course of these studies, such as benzalkonium chloride, sodium benzoate, benzyl alcohol, ethanol, increased EDTA, benzethonium chloride and meta-cresol; however, the best results were achieved with methylparaben sodium/ propylparaben sodium/propylene glycol. Each of other preservatives was either incompatible with insulin (as in the case of benzalknonium chloride, which caused insulin precipitation) or were likely rendered ineffective due to interactions with methyl-β-cyclodextrin and/or polysorbate 80. While methylparaben sodium and propylparaben sodium also interacted with methyl-β-cyclodextrin and/or polysorbate 80, the addition of the humectant, propylene glycol, interferes with this interaction, thereby rendering the parabens more available for antimicrobial activity. The results showed that 10 mg/mL of humectant added to the 0.17 mg/mL propylparaben sodium, 0.33 mg/mL methylparaben sodium formulations produced good antimicrobial activity.

One aspect of the invention herein consists of a preservative combination for use within insulin nasal spray formulations that provides bacteriostatic effect when treated for the U.S. Pharmacopeial and European Pharmacopieal Antimicrobial Effectiveness Tests (AET).

The best AET performing formulation contained water, solubilizer(s), surface active agent(s), buffer, chelator, tonicifier, and preservative. The preferred solubilizer was Me-β-CD. The preferred surface active agents were a combination of DDPC and a polysorbate (such as Tween 80), or polysorbate alone. The preferred chelator was EDTA. The preferred tonicifier was sodium chloride. The preferred preservatives were methylparaben sodium and propylparaben sodium. The formulation also contained a humectant such as propylene glycol, which provided for optimal AET performance.

EXAMPLE 14

Insulin-Formulation Stability

Stability was tested for intranasal insulin formulations at 5°C/Ambient Humidity (routine storage), 25°C/60% RH (accelerated storage), accelerated storage with agitation, and routine or accelerated storage in combination with thrice daily (TID) aerosolization (to mimic patient use). After three months (84 days) of storage, HPLC results showed no significant change in insulin content at 5°C/Ambient Humidity (99.2% insulin recovery) and a minor loss of insulin content was observed at 25°C/60% RH (96.3% insulin recovery). There was no significant insulin loss upon TID aerosolization for short incubation times (11 days) with formulations containing 250 U/mL, 500 U/mL or 1000 U/mL. No significant decrease in stability was observed after 100 rpm agitation for 24 hours at accelerated temperature, in contrast, a marketed insulin product showed a reduction of at least 20% insulin content under the same conditions.

EXAMPLE 15

Human PD Clinical Study

A human study was completed to measure Pharmacodynamic (PD) data following nasal administration of insulin formulations with enhancers compared to administration of currently on the market glucose regulating pharmaceuticals, NovoLog and Exubera. A glucometer was used to measure glucose levels. A summary of the percent reduction in glucose for each treatment group is shown in Table 43. The incidence of 30%, 20%, and 10% reduction in glucose percent for each treatment group is shown in Table 44.

Table 43

| Glucose Percent Reduction by Treatment Group | | | | | |
|---|---|---|---|---|---|
| Treatment Group | # of Subjects | Mean (STD) | Median | Range | CV (%) |
| Nasal Placebo | 12 | 10 (6.3) | 9 | 0-25 | 63.0 |

(continued)

| Glucose Percent Reduction by Treatment Group | | | | | |
|---|---|---|---|---|---|
| Treatment Group | # of Subjects | Mean (STD) | Median | Range | CV (%) |
| NovoLog (SC) | 12 | 44.3 (12.36) | 44 | 25-62 | 27.9 |
| Nasal 25IU | 11 | 17.7 (9.53) | 20 | 0-30 | 54.0 |
| Nasal 50 IU | 11 | 22 (12.31) | 24 | 0-42 | 56.0 |
| Nasal 100 IU | 11 | 28.5 (19.67) | 19 | 11-69 | 69.0 |
| Exubera 3 mg | 6 | 23.8 (11.9) | 21 | 13-44 | 50.0 |

Table 44

| Incidence of Human Subjects with 30%, 20%, and 10% Glucose Reduction | | | | |
|---|---|---|---|---|
| | | Subjects with Glucose % Reduction | | |
| Treatment Group | # of Subjects | GE 30% N (%) | GE 20% N (%) | GE 10% N (%) |
| Nasal Placebo | 12 | 0 (0%) | 1 (8.3%) | 4 (33%) |
| NovoLog (SC) | 12 | 10 (83.3%) | 12 (100%) | 12 (100%) |
| Nasal 25IU | 11 | 0 (0%) | 5(45.5%) | 8 (72.7%) |
| Nasal 50 IU | 11 | 4 (36.4%) | 6 (54.5%) | 9(81.8%) |
| Nasal 100 IU | 11 | 3 (27.3%) | 4 (36.4%) | 11 (100%) |
| Exubera 3 mg | 6 | 2 (33.3%) | 4 (66.7%) | 6 (100%) |

The results of this intial PD study show that intranasal administration of insulin is effective in reducing the percent glucose in a patient. Nasal administration of 50 IU and 100 IU resulted in similar glucose reduction as Exubera, a currently marketed glucose regulating pharmaceutical.

**Claims**

1. A pharmaceutical formulation for intranasal delivery of insulin to a patient, comprising an aqueous mixture of a monomeric insulin, a methyl-β-cyclodextrin, and a polysorbate.

2. The formulation of claim 1, wherein the insulin is a human insulin.

3. The formulation of claim 1, wherein the insulin is a rapid acting human insulin.

4. The formulation of claim 1, wherein the insulin is selected from the group consisting of natural human insulin, human insulin (LysB3, GluB29), human insulin (LysB3, IleB28), human insulin (GlyA21, HisB31, HisB32), human insulin (AspB28), human insulin (AspB10), human insulin (LysB28, ProB29), and mixtures thereof.

5. The formulation of claim 4, wherein the insulin is human insulin (AspB28).

6. The formulation of claim 1, wherein the polysorbate is selected from the group consisting of polysorbate 80, and polysorbate 20, and a mixture thereof

7. The formulation of claim 6, wherein the surface-active agent is polysorbate 80.

8. The formulation of claim 1, further comprising a chelating agent selected from the group consisting of ethylene diamine tetraacetic acid, ethylene glycol tetraacetic acid, and mixtures thereof.

9. The formulation of claim 1, further comprising one or more polyols, preferably wherein the polyol is selected from the group consisting of sucrose, mannitol, sorbitol, lactose, L-arabinose, D-erythrose, D-ribose, D-xylose, D-mannose, trehalose, D-galactose, lactulose, cellobiose, gentibiose, glycerin, polyethylene glycol, and mixtures thereof; more preferably wherein the polyols are lactose and sorbitol.

10. The formulation of claim 1, further comprising a preservative; preferably wherein the preservative is selected from the group consisting of chlorobutanol, methyl paraben, propyl paraben, butyl paraben, benzalkonium chloride, benzethonium chloride, sodium benzoate, sorbic acid, phenol, ortho-cresol, meta-cresol, para-cresol, and mixtures thereof; more preferably wherein the preservative is methylparaben and propylparaben.

11. The formulation of claim 1, further comprising an aerosol of droplets having diameters from 1 to 700 microns in size.

12. The formulation of claim 1, further comprising a humectant, preferably wherein the humectant is selected from the group consisting of propylene glycol, glycerine, glyceryl triacetate, a polyol, a polymeric polyol, lactic acid, urea, and mixtures thereof;
more preferably wherein the humectant is propylene glycol.

13. The formulation of claim 1, further comprising a buffer, preferably wherein the buffer is selected from the group consisting of glutamate, acetate, glycine, histidine, argimne, lysine, methionine, lactate, formate, glycolate, and mixtures thereof;
more preferably wherein the buffer is arginine.

14. The formulation of claim 13, wherein the buffer has a $pK_a$ from 5 to 9, preferably wherein the buffer has a $pK_a$ from 6 to 8.

15. The formulation of claim 1, further comprising a viscosity enhancing agent, preferably wherein the viscosity enhancing agent is selected from the group consisting of gelatin, hydroxypropyl methylcellulose, methylcellulose, carbomer, carboxymethylcellulose, and mixtures thereof;
more preferably wherein the viscosity enhancing agent is carbomer, carboxymethylcellulose, or gelatin.

16. The formulation of claim 1, having a pH of 7.0 $\pm$ 0.5.

17. The formulation of claim 1, further comprising a tonicifier.

18. The formulation of claim 1, having an osmolarity of from 50 to 350 mOsm/L.

19. The formulation of claim 1, **characterized by** a bioavailability greater than about 15%.

20. A use of the formulation of any of the preceding claims for the manufacture of a medicament for treating the signs and symptoms of a disease or condition in a human including diabetes mellitus, hyperglycemia, dyslipidemia, inducing satiety in an individual, promoting weight loss in an individual, obesity, cancer, colon cancer, and prostate cancer.

21. A pharmaceutical formulation according to any of Claims 1-19, for use in treating the signs and symptoms of a disease or condition in a human including diabetes mellitus, hyperglycemia, dyslipidemia, inducing satiety in an individual, promoting weight loss in an individual, obesity, cancer, colon cancer, and prostate cancer.

22. The use of claim 20 or claim 21, wherein the medicament elevates the blood level of insulin in a human for at least about 6 hours post administration.

23. The use of claim 20 or claim 21, wherein the medicament reduces the percent glucose in a human by greater than about 10%.

24. The use of claim 20 or claim 21, wherein the medicament is administered as an aerosol of droplets having diameters from 1 to 700 microns in size.

**Patentansprüche**

1. Pharmazeutische Formulierung zur intranasalen Verabreichung von Insulin an einen Patienten, umfassend ein wässriges Gemisch eines monomeren Insulins, eines Methyl-β-cyclodextrins und eines Polysorbats.

**2.** Formulierung nach Anspruch 1, wobei das Insulin ein humanes Insulin ist.

**3.** Formulierung nach Anspruch 1, wobei das Insulin ein schnell wirkendes humanes Insulin ist.

**4.** Formulierung nach Anspruch 1, wobei das Insulin aus der Gruppe ausgewählt ist, welche aus natürlichem humanem Insulin, humanem Insulin (LysB3, GluB29), humanem Insulin (LysB3, IleB28), humanem Insulin (GlyA21, HisB31, HisB32), humanem Insulin (AspB28), humanem Insulin (AspB10), humanem Insulin (LysB28, ProB29) und Gemischen davon besteht.

**5.** Formulierung nach Anspruch 4, wobei das Insulin humanes Insulin (AspB28) ist.

**6.** Formulierung nach Anspruch 1, wobei das Polysorbat aus der Gruppe ausgewählt ist, welche aus Polysorbat 80 und Polysorbat 20 und einem Gemisch davon besteht.

**7.** Formulierung nach Anspruch 6, wobei das oberflächenaktive Mittel Polysorbat 80 ist.

**8.** Formulierung nach Anspruch 1, ferner umfassend einen Chelatbildner, welcher aus der Gruppe ausgewählt ist, die aus Ethylendiamintetraessigsäure, Ethylenglycoltetraessigsäure und Gemischen davon besteht.

**9.** Formulierung nach Anspruch 1, ferner umfassend ein oder mehrere Polyole, wobei das Polyol bevorzugt aus der Gruppe ausgewählt ist, welche aus Saccharose, Mannitol, Sorbitol, Laktose, L-Arabinose, D-Erythrose, D-Ribose, D-Xylose, D-Mannose, Trehalose, D-Galactose, Laktulose, Cellobiose, Gentibiose, Glycerin, Polyethylenglycol und Gemischen davon besteht; wobei die Polyole stärker bevorzugt Laktose und Sorbitol sind.

**10.** Formulierung nach Anspruch 1, ferner umfassend einen Konservierungsstoff; wobei der Konservierungsstoff bevorzugt aus der Gruppe ausgewählt ist, welche aus Chlorbutanol, Methylparaben, Propylparaben, Butylparaben, Benzalkoniumchlorid, Benzethoniumchlorid, Natriumbenzoat, Sorbinsäure, Phenol, ortho-Cresol, meta-Cresol, para-Cresol und Gemischen davon besteht; wobei der Konservierungsstoff stärker bevorzugt Methylparaben und Propylparaben ist.

**11.** Formulierung nach Anspruch 1, ferner umfassend ein Aerosol von Tropfen mit Durchmessern von 1 bis 700 Mikron in der Größe.

**12.** Formulierung nach Anspruch 1, ferner umfassend ein Netzmittel, wobei das Netzmittel bevorzugt aus der Gruppe ausgewählt ist, welche aus Propylenglycol, Glycerin, Glyceryltriacetat, einem Polyol, einem polymeren Polyol, Milchsäure, Harnstoff und Gemischen davon besteht; wobei das Netzmittel stärker bevorzugt Propylenglycol ist.

**13.** Formulierung nach Anspruch 1, ferner umfassend einen Puffer, wobei der Puffer bevorzugt aus der Gruppe ausgewählt ist, welche aus Glutamat, Acetat, Glycin, Histidin, Arginin, Lysin, Methionin, Laktat, Formiat, Glycolat und Gemischen davon besteht; wobei der Puffer stärker bevorzugt Arginin ist.

**14.** Formulierung nach Anspruch 13, wobei der Puffer einen $pK_a$-Wert von 5 bis 9 aufweist, wobei der Puffer bevorzugt einen $pK_a$-Wert von 6 bis 8 aufweist.

**15.** Formulierung nach Anspruch 1, ferner umfassend ein viskositätssteigerndes Mittel, wobei das viskositätssteigernde Mittel bevorzugt aus der Gruppe ausgewählt ist, welche aus Gelatine, Hydroxypropylmethylcellulose, Methylcellulose, Carbomer, Carboxymethylcellulose und Gemischen davon besteht; wobei das viskositätssteigernde Mittel stärker bevorzugt Carbomer, Carboxymethylcellulose oder Gelatine ist.

**16.** Formulierung nach Anspruch 1, welche einen pH-Wert von 7,0 $\pm$ 0,5 aufweist.

**17.** Formulierung nach Anspruch 1, ferner umfassend ein Mittel für Tonizität.

**18.** Formulierung nach Anspruch 1, welche eine Osmolarität von 50 bis 350 mOsm/L aufweist.

**19.** Formulierung nach Anspruch 1, welche **gekennzeichnet ist durch** eine Bioverfügbarkeit, die höher als etwa 15 % ist.

**20.** Verwendung der Formulierung nach einem der vorhergehenden Ansprüche zur Herstellung eines Medikaments zur Behandlung der Anzeichen und Symptome einer Erkrankung oder eines Zustandes bei einem Menschen, einschließlich Diabetes mellitus, Hyperglykämie, Dyslipidämie, Induzieren von Sattheit bei einem Individuum, Fördern von Gewichtsverlust bei einem Individuum, Fettleibigkeit, Krebs, Kolonkrebs und Prostatakrebs.

**21.** Pharmazeutische Formulierung gemäß einem der Ansprüche 1 bis 19 zur Verwendung in der Behandlung der Anzeichen und Symptome einer Erkrankung oder eines Zustandes bei einem Menschen, einschließlich Diabetes mellitus, Hyperglykämie, Dyslipidämie, Induzieren von Sattheit bei einem Individuum, Fördern von Gewichtsverlust bei einem Individuum, Fettleibigkeit, Krebs, Kolonkrebs und Prostatakrebs.

**22.** Verwendung nach Anspruch 20 oder Anspruch 21, wobei das Medikament den Blutspiegel von Insulin in einem Menschen für mindestens etwa 6 Stunden nach der Verabreichung erhöht.

**23.** Verwendung nach Anspruch 20 oder Anspruch 21, wobei das Medikament den Prozentsatz von Glucose in einem Menschen um mehr als etwa 10 % erniedrigt.

**24.** Verwendung nach Anspruch 20 oder Anspruch 21, wobei das Medikament als ein Aerosol von Tropfen mit Durchmessern von 1 bis 700 Mikron in der Größe verabreicht wird.

**Revendications**

**1.** Formulation pharmaceutique pour une délivrance intranasale d'insuline à un patient, comprenant un mélange aqueux d'insuline monomérique, de méthyl-β-cyclodextrine, et de polysorbate.

**2.** Formulation selon la revendication 1, dans laquelle l'insuline est une insuline humaine.

**3.** Formulation selon la revendication 1, dans laquelle l'insuline est une insuline humaine à action rapide.

**4.** Formulation selon la revendication 1, dans laquelle l'insuline est sélectionnée dans le groupe consistant en l'insuline humaine naturelle, l'insuline humaine (LysB3, GluB29), l'insuline humaine (LysB3, IleB28), l'insuline humaine (GlyA21, HisB31, HisB32), l'insuline humaine (AspB28), l'insuline humaine (AspB10), l'insuline humaine (LysB28, ProB29), et des mélanges de celles-ci.

**5.** Formulation selon la revendication 4, dans laquelle l'insuline est l'insuline humaine (AspB28).

**6.** Formulation selon la revendication 1, dans laquelle le polysorbate est sélectionné dans le groupe consistant en le polysorbate 80 et le polysorbate 20, et un mélange de ceux-ci.

**7.** Formulation selon la revendication 6, dans laquelle l'agent de surface actif est le polysorbate 80.

**8.** Formulation selon la revendication 1, comprenant en outre un agent chélatant sélectionné dans le groupe consistant en l'acide éthylène diamine tétraacétique, l'acide éthylène glycol tétraacétique, et des mélanges de ceux-ci.

**9.** Formulation selon la revendication 1, comprenant en outre un ou plusieurs polyols, où de préférence le polyol est sélectionné dans le groupe consistant en le saccharose, le mannitol, le sorbitol, le lactose, le L-arabinose, le D-érythrose, le D-ribose, le D-xylose, le D-mannose, le tréhalose, le D-galactose, le lactulose, le cellobiose, le gentiobiose, la glycérine, le polyéthylène glycol, et des mélanges de ceux-ci ;
où, de manière davantage préférée, les polyols sont le lactose et le sorbitol.

**10.** Formulation selon la revendication 1, comprenant en outre un conservateur ; où de préférence le conservateur est sélectionné dans le groupe consistant en le chlorobutanol, le méthylparabène, le propylparabène, le butylparabène, le chlorure de benzalkonium, le chlorure de benzéthonium, le benzoate de sodium, l'acide sorbique, le phénol, l'orthocrésol, le métacrésol, le paracrésol, et des mélanges de ceux-ci ;
où, de manière davantage préférée, le conservateur est le méthylparabène et le propylparabène.

**11.** Formulation selon la revendication 1, comprenant en outre un aérosol de gouttelettes ayant des diamètres d'une taille comprise entre 1 et 700 microns.

**12.** Formulation selon la revendication 1, comprenant en outre un humectant, où de préférence l'humectant est sélectionné dans le groupe consistant en le propylène glycol, la glycérine, le triacétate de glycéryle, un polyol, un polyol polymérique, l'acide lactique, l'urée, et des mélanges de ceux-ci ;
où, de manière davantage préférée, l'humectant est le propylène glycol.

**13.** Formulation selon la revendication 1, comprenant en outre un tampon, où de préférence le tampon est sélectionné dans le groupe consistant en le glutamate, l'acétate, la glycine, l'histidine, l'arginine, la lysine, la méthionine, le lactate, le formate, le glycolate, et des mélanges de ceux-ci ;
où, de manière davantage préférée, le tampon est l'arginine.

**14.** Formulation selon la revendication 13, dans laquelle le tampon possède un $pK_a$ compris entre 5 et 9, où de préférence le tampon possède un $pK_a$ compris entre 6 et 8.

**15.** Formulation selon la revendication 1, comprenant en outre un agent améliorant la viscosité, où de préférence l'agent améliorant la viscosité est sélectionné dans le groupe consistant en la gélatine, l'hydroxypropylméthylcellulose, la méthylcellulose, un carbomère, la carboxyméthylcellulose, et des mélanges de ceux-ci ;
où, de manière davantage préférée, l'agent améliorant la viscosité est un carbomère, la carboxyméthylcellulose ou la gélatine.

**16.** Formulation selon la revendication 1, ayant un pH de 7,0 $\pm$ 0,5.

**17.** Formulation selon la revendication 1, comprenant en outre un agent tonifiant.

**18.** Formulation selon la revendication 1, ayant une osmolarité comprise entre 50 et 350 mOsm/l.

**19.** Formulation selon la revendication 1, **caractérisée par** une biodisponibilité supérieure à environ 15 %.

**20.** Utilisation de la formulation selon l'une quelconque des revendications précédentes pour la fabrication d'un médicament destiné à traiter les signes et les symptômes d'une maladie ou d'un état pathologique chez un humain comprenant le diabète sucré, l'hyperglycémie, la dyslipidémie, induire la satiété chez un individu, favoriser une perte de poids chez un individu, l'obésité, le cancer, le cancer du côlon, et le cancer de la prostate.

**21.** Formulation pharmaceutique selon l'une quelconque des revendications 1 à 19, destinée à être utilisée pour traiter les signes et les symptômes d'une maladie ou d'un état pathologique chez un humain, comprenant le diabète sucré, l'hyperglycémie, la dyslipidémie, induire la satiété chez un individu, favoriser une perte de poids chez un individu, l'obésité, le cancer, le cancer du côlon, et le cancer de la prostate.

**22.** Utilisation selon la revendication 20 ou la revendication 21, dans laquelle le médicament élève le taux sanguin d'insuline chez un humain pendant au moins 6 heures après l'administration.

**23.** Utilisation selon la revendication 20 ou la revendication 21, dans laquelle le médicament réduit le pourcentage de glucose chez un humain de plus d'environ 10 %.

**24.** Utilisation selon la revendication 20 ou la revendication 21, dans laquelle le médicament est administré en tant qu'aérosol de gouttelettes ayant des diamètres d'une taille comprise entre 1 et 700 microns.

*FIG.1*

**FIG.2**

*FIG.3*

*FIG.4*

**FIG.5**

EP 1 951 198 B1

FIG.6

FIG.7

*FIG.8*

**FIG.9**

*FIG.10*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6737045 B
- US 6506724 B
- US 20030036504 A1
- EP 1083924 B1
- WO 9830231 A1
- WO 0073331 A2
- EP 1031347 A
- US 2003211972 A
- WO 9422461 A
- US 4839343 A
- US 6190894 B
- US 5908825 A
- US 6004583 A
- US 5681811 A
- US 3972995 A
- US 4259314 A
- US 4680323 A
- US 4740365 A
- US 4573996 A
- US 4292299 A
- US 4715369 A
- US 4876092 A
- US 4855142 A
- US 4250163 A
- US 4226848 A
- US 4948580 A
- US 33093 A
- US 4235871 A
- US 4501728 A
- US 4837028 A
- US 4179337 A
- US 4511069 A
- US 4652441 A
- US 4917893 A
- US 4677191 A
- US 4728721 A
- US 4675189 A
- US 233239 A

**Non-patent literature cited in the description**

- **ZHANG Y et al.** *ZHONGUA YAOLI XUEBAO - ACTA PHARMACOLOGICA SINICA,* November 2001, vol. 22 (11), 1051-1056
- *US. Pharmacopeia National Formulary,* 1990, 1857-1859
- **Laursen et al.** *Eur. J Endocrinology,* 1996, vol. 135, 309-315
- **Lagowski.** Macmillan Encyclopedia of Chemistry. Simon & Schuster, 1997, vol. 1, 273-4
- **Dawson.** Data for Biochemical Research. Oxford Science Publications, 1986, 419
- **Lide.** CRC Handbook of Chemistry and Physics. Taylor & Francis Group, 2005, 2-41
- **Ugwoke et al.** *Adv. Drug Deliv. Rev.,* 1998, vol. 29, 1656-57
- **Fasano et al.** *Proc. Nat. Acad. Sci., U.S.A.,* 1991, vol. 8, 5242-5246